# EUROPEAN PATENT SPECIFICATION

(11) **EP 3 194 419 B1**
(45) Date of publication and mention of the grant of the patent: **05.09.2018**
(21) Application number: 15771043.5
(22) Date of filing: 07.09.2015
(51) Int. Cl.: C07J 43/00, A61K 31/58, A61P 5/24, C07J 31/00, C07J 41/00

(54) **3-NITROGEN OR SULPHUR SUBSTITUTED OESTRA-1,3,5(10),16-TETRAENE AKR1C3 INHIBITORS**
3-STICKSTOFF- ODER SCHWEFELSUBSTITUIERTE OESTRA-1,3,5 (10),16-TETRAEN-AKR1C3-INHIBITOREN
INHIBITEURS D'AKR1C3 DE TYPE ESTRA-1,3,5(10),16-TÉTRAÈNE SUBSTITUÉS PAR UN 3-AZOTE OU SOUFRE

(30) Priority: 11.09.2014 EP 14184403
(43) Date of publication of application: 26.07.2017
(73) Proprietor: Bayer Pharma Aktiengesellschaft, 13353 Berlin (DE)
(72) Inventor: BOTHE, Ulrich, 13187 Berlin (DE); CANCHO GRANDE, Yolanda, 51373 Leverkusen (DE); IRLBACHER, Horst, 10405 Berlin (DE); RAY, Nicholas Charles, Harlow Essex CM19 5TR (GB)
(74) Representative: BIP Patents
(86) International application number: PCT/EP2015/070327
(87) International publication number: WO 2016/037956

(56) References cited:
- WO-A1-2012/129673
- WO-A1-2013/045407
- WO-A1-2014/128108
- US-A1- 2008 255 075
- ÉDITH BELLAVANCE ET AL: "Potent and Selective Steroidal Inhibitors of 17[beta]-Hydroxysteroid Dehydrogenase Type 7, an Enzyme That Catalyzes the Reduction of the Key Hormones Estrone and Dihydrotestosterone", JOURNAL OF MEDICINAL CHEMISTRY, vol. 52, no. 23, 10 December 2009 (2009-12-10), pages 7488-7502, XP55134310, ISSN: 0022-2623, DOI: 10.1021/jm900921c -& , 22 September 2009 (2009-09-22), XP55230713, Retrieved from the Internet: URL:http://pubs.acs.org/doi/suppl/10.1021/ jm900921c/suppl_file/jm900921c_si_001.pdf [retrieved on 2015-11-24]
- RENÉ MALTAIS ET AL: "Discovery of a Non-Estrogenic Irreversible Inhibitor of 17[beta]-Hydroxysteroid Dehydrogenase Type 1 from 3-Substituted-16[beta]-( m -carbamoylbenzyl)-estradiol Derivatives", JOURNAL OF MEDICINAL CHEMISTRY, vol. 57, no. 1, 9 January 2014 (2014-01-09), pages 204-222, XP55134354, ISSN: 0022-2623, DOI: 10.1021/jm401639v
- MICHEL PONS ET AL: "Affinity Labelling of the Estradiol-17beta Dehydrogenase from Human Placenta with Substrate Analogs", EUROPEAN JOURNAL OF BIOCHEMISTRY, vol. 68, no. 2, 1 September 1976 (1976-09-01), pages 385-394, XP55134903, ISSN: 0014-2956, DOI: 10.1111/j.1432-1033.1976.tb10825.x

## Description

The invention relates to AKR1C3 inhibitors and to processes for preparation thereof, to the use thereof for treatment and/or prophylaxis of diseases and to the use thereof for production of medicaments for treatment and/or prophylaxis of diseases, especially of bleeding disorders and endometriosis.

The aldo-keto reductase 1C3 (AKR1C3; synonyms: type 5 17β-hydroxysteroid dehydrogenase or prostaglandin F synthase) is a multifunctional enzyme and catalyses, among other processes, the reduction of 4-androstene-3,17-dione (a weak androgen) to testosterone (a potent androgen) and of oestrone (a weak oestrogen) to 17β-oestradiol (a strong oestrogen). In addition, the reduction of prostaglandin (PG) H2 to PGF2α and PGD2 to 9α,11β-PGF2 is inhibited (Penning, T., M. et. al. (2006). Aldo-keto reductase (AKR) 1C3: Role in prostate disease and the development of specific inhibitors, Molecular and Cellular Endocrinology, 248(1-2), 182 -191).

The local formation of oestradiol (E2) plays a central role for the initiation and the progression of breast cancers and endometriosis. The reduction of tissue levels of oestrogens and especially of oestradiol is achieved by the therapeutic administration of aromatase inhibitors (in order to inhibit the formation of oestrogens from androgens) and of sulphatase inhibitors (in order to block the formation of oestrone from oestrone sulphate). However, both therapeutic approaches have the disadvantage that systemic oestrogen levels are radically reduced (Oster, A. et. al. (2010). J. Med. Chem, 53, 8176 - 8186). Recently, it has been demonstrated experimentally that endometriotic lesions are capable of local synthesis of oestradiol (Delvoux, B. et al. (2009). J Clin Endocrinol Metab, 94, 876 - 883). For the subtype of ovarian endometriosis, overexpression of AKR1C3 mRNA has been described (Smuc, T. et al. (2009). Mol Cell Endocrinol, 301(1 - 2): 59-64).

There is a great need for the identification of novel inhibitors of the enzyme AKR1C3, since inhibitors have potential for treatment of hormone-dependent disorders, for example endometriosis, but also for treatment of hormone-independent disorders (Byrns, M., C., Jin, Y., Penning, T., M. (2010). Journal of Steroid Biochemistry and Molecular Biology, 118, 177 - 87, Lovering, A., L. et. al. (2004). Cancer Res, 64(5), 1802 - 1810). As well as endometriosis, these also include prostate cancer (Fung, K., M. et al. (2008). Endocr Relat Cancer, 13(1), 169 - 180), prostate hyperplasia (Roberts, R., O. et al. (2006). Prostate, 66(4), 392 - 404), endometrial carcinoma (Rizner, T., L. et al. (2006). Mol Cell Endocrinol, 248(1-2), 126 - 135), polycystic ovary syndrome (Qin, K. et al. (2006). J Endocrinol Metab, 91(1), 270 - 276), pulmonary carcinoma (Lan, Q. et al. (2004). Carcinogenesis, 25(11), 2177 - 2181), non-Hodgkins lymphoma (Lan, Q. et al. (2007). Hum Genet, 121(2), 161 - 168), hair loss (Colombe, L. et al. (2007). Exp Dermatol, 16(9), 762 - 769), obesity (Svensson, P., A. et al. (2008). Cell MolBiol Lett, 13(4), 599 - 613), bladder carcinoma (Figueroa, J., D. (2008). Carcinogenesis, 29(10), 1955 - 1962), chronic myeloid leukaemia (Birtwistle, J. (2009). Mutat Res, 662(1-2), 67 - 74), renal cell carcinoma (Azzarello, J., T. (2009). Int J Clin Exp Pathol, 3(2), 147 - 155), breast cancer (Byrns, M., C. (2010). J Steroid Biochem Mol Biol, 118(3), 177 - 187), premature sexual maturity (He, C. (2010). Hum Genet, 128(5), 515 - 527) and chronic obstructive pulmonary disease (Pierrou, S. (2007). Am J Respir Crit Care, 175(6), 577 - 586).

Some non-steroidal inhibitors of AKR1C3 are known (review articles: Day, J., M., Tutill, H., J., Purohit, A. and Reed, M., J. (2008). Endocrine-Related Cancer 15, 665 - 692; Adenijii, A., O., Chem, M., Penning, T., M. (2013). J of Steroid Biochemistry and Molecular Biology, 137, 136 - 149). For additional inhibitors see patent applications US20100190826, WO2007100066, WO2014039820, WO2013142390, WO2013059245. For recent publications see Brožič, P. et al. (2012). J. Med. Chem., 55, 7417 - 7424,. Adenijii, A. O. et al. (2012). J Med Chem, 55, 2311 - 2323 and Jamieson, S. M. F. et al. (2012). J. Med. Chem., 55, 7746 - 7758, Liedtke, A., L. et al. (2013). J. Med. Chem., 56, 2429 - 2446, Watanabe, K. et al. (2013). Bioorg. & Med. Chem., 21, 5261 - 5270, Flanagan, J., A. et. al. 2014). Bioorg. & Med. Chem., 22, 967 - 977, Gazvoda, M. et al. (2013). Eur. J. of Med. Chem., 62, 89 - 97, Heinrich, D., M. et al. (2013) Eur. J. of Med. Chem., 62, 738. Bifunctional AKR1C3 inhibitors/androgen receptor modulators have been described in WO2012142208.

An example of a steroidal substance which has been described is EM-1404, based on the oestratriene skeleton with a spirolactone unit at position 17 (F. Labrie et al. US Patent 6,541,463; 2003).

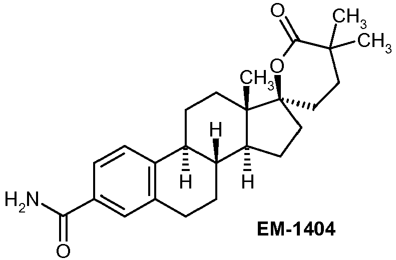

Further steroidal AKR1C3 inhibitors with a lactone unit were described in Bydal, P., Luu-The, Van, Labrie, Poirier, D. F. (2009). European Journal of Medicinal Chemistry, 44, 632-644. Fluorinated oestratriene derivatives were described in Deluca, D., Moller, G., Rosinus, A., Elger, W., Hillisch, A., Adamski, J. (2006). Mol Cell Endocrinol, 248, 218 - 224.

Steroidal ARK1C3 inhibitors based on an oestra-1,3,5(10),16-tetraene core bearing a heterocyclic ring at the 17-position have been described in WO 20140009274, WO2014128108 and WO 2013045407. These inhibitors comprise substituents at the 3-position of the oestra-1,3,5(10),16-tetraene which are linked with a carbon or an oxygen atom like an alkyl, carboxamid or ether group. However, no compounds have been described based on nitrogen or sulphur linked substituents.

US Patent US 5,604,213 (S. E. Barrie et al.) described 17-(3-pyridyl)oestra-1,3,5(10),16-tetraen-3-ol, a structure substituted on carbon atom 3 by a free hydroxyl group, as a 17α-hydroxylase/C17-20 lyase (Cyp17A1) inhibitor, but not as an AKR1C3 inhibitor. 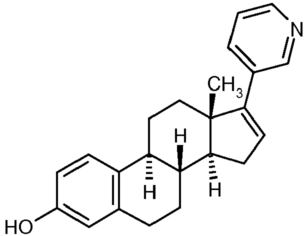 17-(3-Pyridyl)oestra-1,3,5(10),16-tetraen-3-ol

17-(3-Pyridyl)oestra-1,3,5(10),16-tetraene derivatives substituted at position 3 by a carboxamide group are not described in US Patent US 5,604,213.

The application US2005/0203075 describes oestra-1,3,5(10),16-tetraene derivatives substituted by a - CONH₂ group at the 3 position as having antiproliferative and antiangiogenetic action, without reference to a specific molecular target. However, these derivatives are not substituted by a heterocycle at position 17 of the oestra-1,3,5(10),16-tetraene skeleton.

A review of 17-pyridyl- and 17-pyrimidinylandrostane derivatives which are described as Cyp17A1 inhibitors can be found in Moreira, V., M. et al. (2008) Current Medicinal Chemistry, 15 (9), 868-899.

Even though numerous AKR1C3 inhibitors have been described, there is still a need for substances having improved properties.

Therefore it is an object of the present invention to provide new substances active as AKR1C3 inhibitors. The present invention provides compounds of the formula (I): 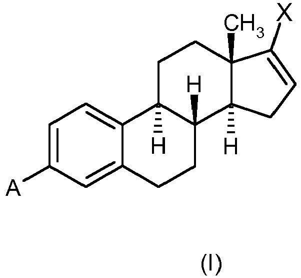 where:
- A: represents a group selected from: 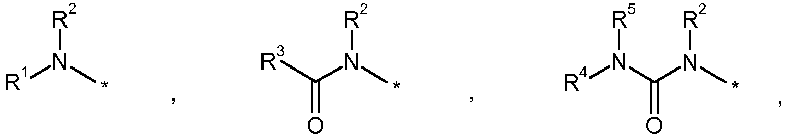 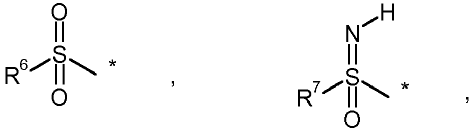 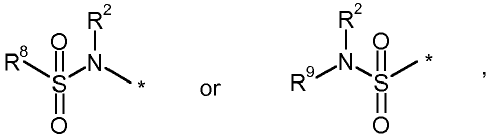 wherein * indicates the point of attachment of said group with the rest of the molecule;
- X: is a group selected from: 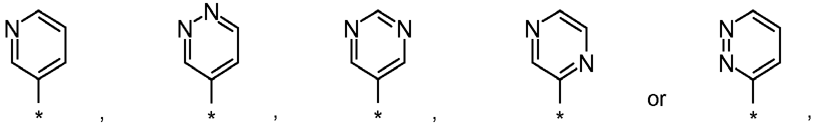 wherein * indicates the point of attachment of the group with the rest of the molecule and where the group is optionally substituted, one or more times, independently from each other, with a substituent selected from halogen, CN, OH, RR²N-, C₁-C₆-alkyl, C₁-C₆-haloalkyl, C₁-C₆-alkoxy, C₁-C₆-haloalkoxy, wherein C₁-C₆-alkyl and C₁-C₆-haloalkyl groups are optionally substituted with OH;
- R¹: is C₁-C₆-alkyl or C₁-C₆-haloalkyl, and where R¹ is optionally substituted with one or two substituents, independently from each other, selected from OH, CN, RO(CO)-, RR²N(CO)-, RR²NSO₂-, C₁-C₆-alkyl-(CO)(NH)SO₂- or 5-tetrazolyl;
- R², R⁵: are, independently from each other, hydrogen or C₁-C₆-alkyl, where C₁-C₆-alkyl groups are optionally substituted, one or more times, independently from each other, with halogen,
- R³, R⁴: are, independently from each other, C₁-C₆-alkyl or C₁-C₆-haloalkyl, and whereby
R³ and R⁴ are optionally substituted with one or two substituents, independently from each other, selected from OH, CN, RO(CO)-, RR²N(CO)-, RR²NSO₂-, C₁-C₆-alkyl-(CO)(NH)SO₂- or 5-tetrazolyl;
- R⁶, R⁷, R⁸, R⁹: are, independently from each other, C₁-C₆-alkyl or C₁-C₆-haloalkyl, and whereby
R⁶, R⁷, R⁸ and R⁹ are optionally substituted with one or two substituents, independently from each other, selected from OH, CN, RO(CO)-, RR²N(CO)-, RR²NSO₂-, C₁-C₆-alkyl-(CO)(NH)SO₂- or 5-tetrazolyl;
- R: is hydrogen or a C₁-C₆-alkyl group;
or a tautomer, an N-oxide, a hydrate, a solvate, or a salt thereof, or a mixture of same.

The terms as mentioned in the present text have preferably the following meanings:
The term "halogen atom", "halogen", "halo-" or "Hal-" is to be understood as meaning a fluorine, chlorine, bromine or iodine atom, preferably a fluorine or chlorine atom.

The term "C₁-C₆-alkyl" is to be understood as preferably meaning a linear or branched, saturated, monovalent hydrocarbon group having 1, 2, 3, 4, 5, or 6 carbon atoms, e.g. a methyl, ethyl, propyl, butyl, pentyl, hexyl, iso-propyl, iso-butyl, sec-butyl, tert-butyl, iso-pentyl, 2-methylbutyl, 1-methylbutyl, 1-ethylpropyl, 1,2-dimethylpropyl, neo-pentyl, 1,1-dimethylpropyl, 4-methylpentyl, 3-methylpentyl, 2-methylpentyl, 1-methylpentyl, 2-ethylbutyl, 1-ethylbutyl, 3,3-dimethylbutyl, 2,2-dimethylbutyl, 1,1-dimethylbutyl, 2,3-dimethylbutyl, 1,3-dimethylbutyl, or 1,2-dimethylbutyl group, or an isomer thereof. Particularly, said group has 1, 2, 3 or 4 carbon atoms ("C₁-C₄-alkyl"), *e.g.* a methyl, ethyl, propyl, butyl, iso-propyl, iso-butyl, sec-butyl, tert-butyl group, more particularly 1, 2 or 3 carbon atoms ("C₁-C₃-alkyl"), *e.g.* a methyl, ethyl, n-propyl or iso-propyl group.

The term "C₁-C₆-haloalkyl" is to be understood as preferably meaning a linear or branched, saturated, monovalent hydrocarbon group in which the term "C₁-C₆-alkyl" is defined *supra,* and in which one or more hydrogen atoms is replaced by a halogen atom, in identically or differently, *i.e.* one halogen atom being independent from another. Particularly, said halogen atom is F. Said C₁-C₆-haloalkyl group is, for example, -CF₃, -CHF₂, -CH₂F, -CF₂CF₃, or -CH₂CF₃.

The term "C₁-C₆-alkoxy" is to be understood as preferably meaning a linear or branched, saturated, monovalent, hydrocarbon group of formula -O-alkyl, in which the term "alkyl" is defined *supra, e.g.* a methoxy, ethoxy, n-propoxy, iso-propoxy, n-butoxy, iso-butoxy, tert-butoxy, sec-butoxy, pentoxy, iso-pentoxy, or n-hexoxy group, or an isomer thereof. Particularly, said "C₁-C₆-alkoxy" can contain 1, 2, 3, 4 or 5 carbon atoms, (a "C₁-C₅-alkoxy").

The term "C₁-C₆-haloalkoxy" is to be understood as preferably meaning a linear or branched, saturated, monovalent C₁-C₆-alkoxy group, as defined *supra,* in which one or more of the hydrogen atoms is replaced, in identically or differently, by a halogen atom. Particularly, said halogen atom is F. Said C₁-C₆-haloalkoxy group is, for example, -OCF₃, -OCHF₂, -OCH₂F, -OCF₂CF₃, or -OCH₂CF₃.

The term "C₁-C₆", as used throughout this text, e.g. in the context of the definition of "C₁-C₆-alkyl", "C₁-C₆-haloalkyl", "C₁-C₆-alkoxy", or "C₁-C₆-haloalkoxy" is to be understood as meaning an alkyl group having a finite number of carbon atoms of 1 to 6, *i.e.* 1, 2, 3, 4, 5, or 6 carbon atoms. It is to be understood further that said term "C₁-C₆" is to be interpreted as any sub-range comprised therein, e.g. C₁-C₆, C₂-C₅, C₃-C₄, C₁-C₂, C₁-C₃, C₁-C₄, C₁-C_{5;} particularly C₁-C₂, C₁-C₃, C₁-C₄, C₁-C₅, C₁-C_{6;} more particularly C₁-C₄; in the case of "C₁-C₆-haloalkyl" or "C₁-C₆-haloalkoxy" even more particularly C₁-C₂.

In accordance with another embodiment, the present invention covers compounds of general formula *(I), supra,* in which:
- A: represents a group selected from: 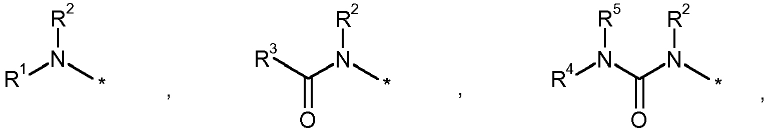 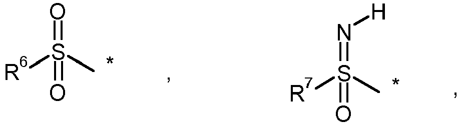 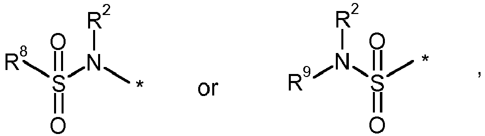 wherein * indicates the point of attachment of said group with the rest of the molecule;
- X: is a group selected from: 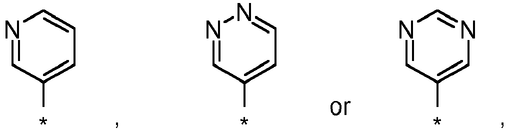 wherein * indicates the point of attachment of the group with the rest of the molecule and where the group is optionally substituted, one or more times, independently from each other, with a substituent selected from halogen, C₁-C₄-alkyl, C₁-C₄-haloalkyl, C₁-C₄-alkoxy, wherein C₁-C₄-alkyl and C₁-C₄-haloalkyl groups are optionally substituted with OH;
- R¹: is C₁-C₄-alkyl, which is optionally substituted with one or two substituents, independently from each other, selected from
OH, RO(CO)-, RR²N(CO)-, RR²NSO₂- or 5-tetrazolyl;
- R², R⁵: are, independently from each other, hydrogen or C₁-C₄-alkyl, where C₁-C₄-alkyl groups are optionally substituted, one or more times, independently from each other, with halogen;
- R³, R⁴: are, independently from each other, C₁-C₄-alkyl, and whereby
R³ and R⁴ are optionally substituted with one or two substituents, independently from each other, selected from OH, RO(CO)-, RR²N(CO)-, RR²NSO₂-, C₁-C₄-alkyl-(CO)(NH)SO₂- or 5-tetrazolyl;
- R⁶, R⁷, R⁸, R⁹: are, independently from each other, C₁-C₄-alkyl, and whereby
R⁶, R⁷, R⁸ and R⁹ are optionally substituted with one or two substituents, independently from each other, selected from OH, RO(CO)-, RR²N(CO)-, RR²NSO₂- or 5-tetrazolyl;
- R: is hydrogen or a C₁-C₄-alkyl group;
or a tautomer, an N-oxide, a hydrate, a solvate, or a salt thereof, or a mixture of same.

In accordance with another embodiment, the present invention covers compounds of general formula *(I), supra,* in which:
- A: represents a group selected from: 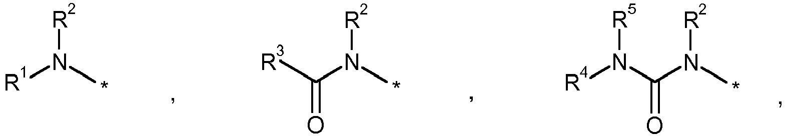 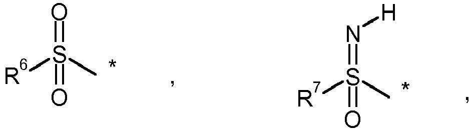 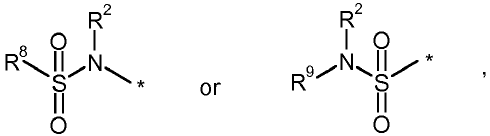 wherein * indicates the point of attachment of said group with the rest of the molecule;
- X: is a group selected from: 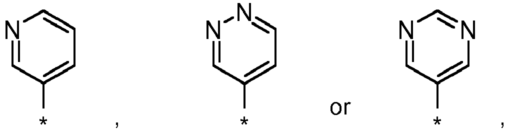 wherein * indicates the point of attachment of the group with the rest of the molecule and where the group is optionally substituted, one or two times, independently from each other, with a substituent selected from fluoro, chloro, methyl, trifluoromethyl or methoxy;
- R¹: is propyl, which is optionally substituted with RO(CO)-;
- R², R⁵: are, independently from each other, hydrogen or methyl;
- R³, R⁴: are, independently from each other, C₁-C₃-alkyl, and whereby
R³ and R⁴ are optionally substituted with one or two substituents, independently from each other, selected from OH, RO(CO)-, RR²N(CO)-, RR²NSO₂-, C₁-C₂-alkyl-(CO)(NH)SO₂- or 5-tetrazolyl;
- R⁶, R⁷, R⁸, R⁹: are, independently from each other, C₁-C₄-alkyl, and whereby
R⁶, R⁷, R⁸ and R⁹ are optionally substituted with one or two substituents, independently from each other, selected from OH, RO(CO)- or RR²N(CO)-;
- R: is hydrogen or a C₁-C₄-alkyl group;
or a tautomer, an N-oxide, a hydrate, a solvate, or a salt thereof, or a mixture of same.

In accordance with another embodiment, the present invention covers compounds of general formula *(I), supra,* in which:
- A: represents a group selected from: 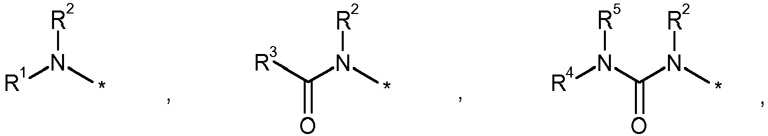 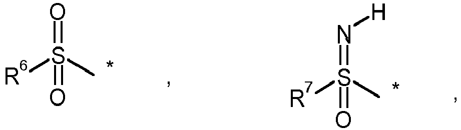 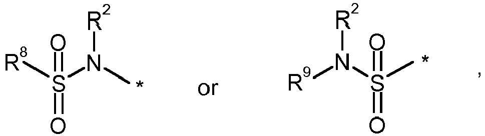 wherein * indicates the point of attachment of said group with the rest of the molecule;
- X: is a group selected from: 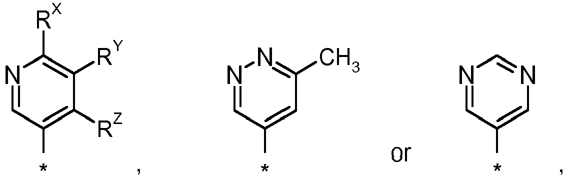 wherein * indicates the point of attachment of the group with the rest of the molecule and,
- R^{x}: is hydrogen or methyl,
- R^{Y}: is hydrogen, fluoro, chloro, methyl, trifluoromethyl or methoxy,
- R^{Z}: is hydrogen or methyl,
wherein only one of R^{X}, R^{Y} and R^{Z} is different from hydrogen and;
- R¹: is -CH₂-CH₂-CH₂-COOH;
- R²: is hydrogen or methyl;
- R³: is C₁-C₃-alkyl, which is optionally substituted with one substituent, selected from OH, HO(CO)-, H₂N(CO)-, CH₃-(CO)(NH)SO₂- or 5-tetrazolyl;
- R⁴: is -CH₂-CH₂-COOH;
- R⁵: is hydrogen;
- R⁶: is C₁-C₄-alkyl, which is optionally substituted with one substituent, selected from OH, RO(CO)- or H₂N(CO)-;
- R⁷: is methyl;
- R⁸: is -CH₂-CH₂-COOH;
- R⁹: is C₂-C₃-alkyl, which is substituted with one substituent, selected from OH or HO(CO)-;
- R: is hydrogen or methyl;
or a tautomer, an N-oxide, a hydrate, a solvate, or a salt thereof, or a mixture of same.

In accordance with another embodiment, the present invention covers compounds of general formula *(I), supra,* selected from a group comprising the following compounds:
- tert-butyl N-{[17-(5-fluoropyridin-3-yl)estra-1(10),2,4,16-tetraen-3-yl]sulfonyl}-N-methyl-beta-alaninate
- N-{[17-(5-fluoropyridin-3-yl)estra-1(10),2,4,16-tetraen-3-yl]sulfonyl}-N-methyl-beta-alanine
- 17-(5-fluoropyridin-3-yl)-N-(3-hydroxypropyl)-N-methylestra-1(10),2,4,16-tetraene-3-sulfonamide
- tert-butyl N-methyl-N-{[17-(pyrimidin-5-yl)estra-1(10),2,4,16-tetraen-3-yl]sulfonyl}-beta-alaninate
- N-methyl-N-{[17-(pyrimidin-5-yl)estra-1(10),2,4,16-tetraen-3-yl]sulfonyl}-beta-alanine
- tert-butyl N-methyl-N-{[17-(6-methylpyridazin-4-yl)estra-1(10),2,4,16-tetraen-3-yl]sulfonyl}-beta-alaninate
- N-methyl-N-{[17-(6-methylpyridazin-4-yl)estra-1(10),2,4,16-tetraen-3-yl]sulfonyl}-beta-alanine
- 17-(5-fluoropyridin-3-yl)-3-(methylsulfonyl)estra-1(10),2,4,16-tetraene
- methyl 4-{[17-(5-fluoropyridin-3-yl)estra-1(10),2,4,16-tetraen-3-yl]sulfonyl}butanoate
- 4-{[17-(5-fluoropyridin-3-yl)estra-1(10),2,4,16-tetraen-3-yl]sulfonyl}butanoic acid
- methyl 4-{[17-(pyridin-3-yl)estra-1(10),2,4,16-tetraen-3-yl]sulfonyl}butanoate
- 4-{[17-(pyridin-3-yl)estra-1(10),2,4,16-tetraen-3-yl]sulfonyl}butanoic acid
- methyl 4-({17-[5-(trifluoromethyl)pyridin-3-yl]estra-1(10),2,4,16-tetraen-3-yl}sulfonyl) butanoate
- 4-({17-[5-(trifluoromethyl)pyridin-3-yl]estra-1(10),2,4,16-tetraen-3-yl}sulfonyl)butanoic acid
- 4-{[17-(6-methylpyridin-3-yl)estra-1,3,5(10),16-tetraen-3-yl]sulfonyl}butanoic acid
- 4-{[17-(5-methoxypyridin-3-yl)estra-1,3,5(10),16-tetraen-3-yl]sulfonyl}butanoic acid
- 4-{[17-(5-methylpyridin-3-yl)estra-1,3,5(10),16-tetraen-3-yl]sulfonyl}butanoic acid
- 4-{[17-(4-methylpyridin-3-yl)estra-1,3,5(10),16-tetraen-3-yl]sulfonyl}butanoic acid
- 4-{[17-(5-chloropyridin-3-yl)estra-1,3,5(10),16-tetraen-3-yl]sulfonyl}butanoic acid
- 4-{[17-(5-fluoropyridin-3-yl)estra-1(10),2,4,16-tetraen-3-yl]sulfonyl}butanamide
- 4-{[17-(5-fluoropyridin-3-yl)estra-1(10),2,4,16-tetraen-3-yl]sulfonyl}butan-1-ol
- tert-butyl 4-{[17-(5-fluoropyridin-3-yl)estra-1(10),2,4,16-tetraen-3-yl]amino}-4-oxobutanoate
- 4-{[17-(5-fluoropyridin-3-yl)estra-1(10),2,4,16-tetraen-3-yl]amino}-4-oxobutanoic acid
- tert-butyl 4-oxo-4-{[17-(pyrimidin-5-yl)estra-1(10),2,4,16-tetraen-3-yl]amino}butanoate
- 4-oxo-4-{[17-(pyrimidin-5-yl)estra-1(10),2,4,16-tetraen-3-yl]amino}butanoic acid
- N-[17-(5-fluoropyridin-3-yl)estra-1(10),2,4,16-tetraen-3-yl]butanediamide
- N-[17-(5-fluoropyridin-3-yl)estra-1(10),2,4,16-tetraen-3-yl]-4-hydroxybutanamide
- N-[17-(5-fluoropyridin-3-yl)estra-1(10),2,4,16-tetraen-3-yl]-3-sulfamoylpropanamide
- 3-(acetylsulfamoyl)-N-[17-(5-fluoropyridin-3-yl)estra-1(10),2,4,16-tetraen-3-yl]propanamide
- N-[17-(5-fluoropyridin-3-yl)estra-1(10),2,4,16-tetraen-3-yl]-N-methyl-3-sulfamoylpropanamide
- 3-(acetylsulfamoyl)-N-[17-(5-fluoropyridin-3-yl)estra-1(10),2,4,16-tetraen-3-yl]-N-methyl propanamide
- N-[17-(5-fluoropyridin-3-yl)estra-1(10),2,4,16-tetraen-3-yl]-3-(2H-tetrazol-5-yl)propanamide
- N-[17-(5-fluoropyridin-3-yl)estra-1(10),2,4,16-tetraen-3-yl]-N-methyl-3-(2H-tetrazol-5-yl) propanamide
- tert-butyl 4-{[17-(5-fluoropyridin-3-yl)estra-1(10),2,4,16-tetraen-3-yl](methyl)amino}-4-oxobutanoate
- 4-{[17-(5-fluoropyridin-3-yl)estra-1(10),2,4,16-tetraen-3-yl](methyl)amino}-4-oxobutanoic acid
- methyl 3-{[17-(5-fluoropyridin-3-yl)estra-1(10),2,4,16-tetraen-3-yl](methyl)sulfamoyl} propanoate
- methyl 3-{[17-(5-fluoropyridin-3-yl)estra-1(10),2,4,16-tetraen-3-yl]sulfamoyl}propanoate
- 3-{[17-(5-fluoropyridin-3-yl)estra-1(10),2,4,16-tetraen-3-yl](methyl)sulfamoyl}propanoic acid
- 3-{[17-(5-fluoropyridin-3-yl)estra-1(10),2,4,16-tetraen-3-yl]sulfamoyl}propanoic acid
- N-[17-(5-fluoropyridin-3-yl)estra-1(10),2,4,16-tetraen-3-yl]acetamide
- ethyl N-{[17-(5-fluoropyridm-3-yl)estra-1(10),2,4,16-tetraen-3-yl]carbamoyl}-beta-alaninate
- N-{[17-(5-fluoropyridin-3-yl)estra-1(10),2,4,16-tetraen-3-yl]carbamoyl}-beta-alanine
- methyl 4-{[17-(5-fluoropyridin-3-yl)estra-1(10),2,4,16-tetraen-3-yl](methyl)amino}butanoate
- 4-{[17-(5-fluoropyridin-3-yl)estra-1(10),2,4,16-tetraen-3-yl](methyl)amino}butanoic acid
- 4-{[17-(6-methylpyridazin-4-yl)estra-1(10),2,4,16-tetraen-3-yl]amino}-4-oxobutanoic acid
- 17-(5-fluoropyridin-3-yl)-3-(S-methylsulfonimidoyl)estra-1(10),2,4,16-tetraene and the tautomers, N-oxides, hydrates, solvates or salts thereof, or a mixture consisting of the above.

In a further embodiment of the above-mentioned aspect, the invention relates to compounds of formula (I), wherein:
- A: represents a group selected from: 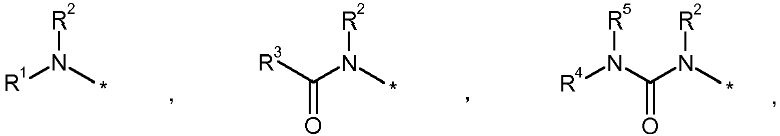 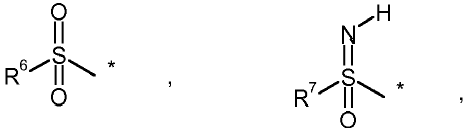 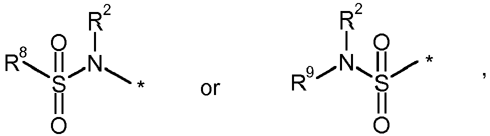 wherein * indicates the point of attachment of said group with the rest of the molecule.

In a further embodiment of the above-mentioned aspect, the invention relates to compounds of formula (I), wherein:
- A: represents a group selected from: 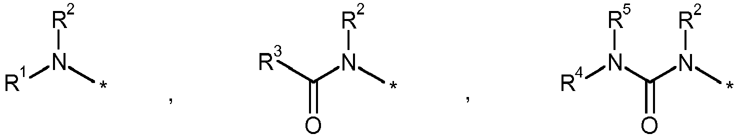 or 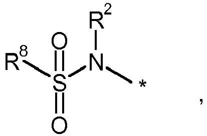 wherein * indicates the point of attachment of said group with the rest of the molecule.

In a further embodiment of the above-mentioned aspect, the invention relates to compounds of formula (I), wherein:
- A: represents a group selected from: 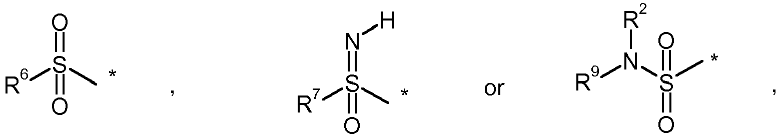 wherein * indicates the point of attachment of said group with the rest of the molecule.

In a further embodiment of the above-mentioned aspect, the invention relates to compounds of formula (I), wherein:
- A: represents a group: 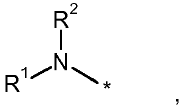 wherein * indicates the point of attachment of said group with the rest of the molecule.

In a further embodiment of the above-mentioned aspect, the invention relates to compounds of formula (I), wherein:
- A: represents a group: 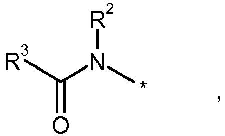 wherein * indicates the point of attachment of said group with the rest of the molecule.

In a further embodiment of the above-mentioned aspect, the invention relates to compounds of formula (I), wherein:
- A: represents a group: 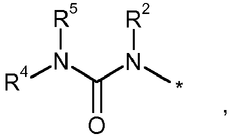 wherein * indicates the point of attachment of said group with the rest of the molecule.

In a further embodiment of the above-mentioned aspect, the invention relates to compounds of formula (I), wherein:
- A: represents a group: 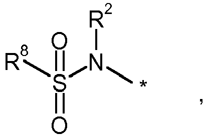 wherein * indicates the point of attachment of said group with the rest of the molecule.

In a further embodiment of the above-mentioned aspect, the invention relates to compounds of formula (I), wherein:
- A: represents a group: 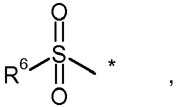 wherein * indicates the point of attachment of said group with the rest of the molecule.

In a further embodiment of the above-mentioned aspect, the invention relates to compounds of formula (I), wherein:
- A: represents a group: 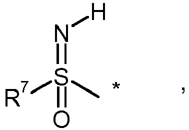 wherein * indicates the point of attachment of said group with the rest of the molecule.

In a further embodiment of the above-mentioned aspect, the invention relates to compounds of formula (I), wherein:
- A: represents a group: 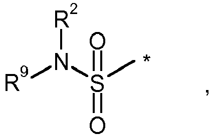 wherein * indicates the point of attachment of said group with the rest of the molecule.

In a further embodiment of the above-mentioned aspect, the invention relates to compounds of formula (I), wherein:
- X: is a group selected from: 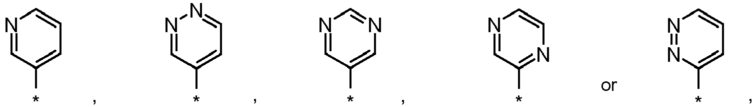 wherein * indicates the point of attachment of the group with the rest of the molecule and where the group is optionally substituted, one or more times, independently from each other, with a substituent selected from halogen, CN, OH, RR²N-, C₁-C₆-alkyl, C₁-C₆-haloalkyl, C₁-C₆-alkoxy, C₁-C₆-haloalkoxy, wherein C₁-C₆-alkyl and C₁-C₆-haloalkyl groups are optionally substituted with OH;

In a further embodiment of the above-mentioned aspect, the invention relates to compounds of formula (I), wherein:
- X: is a group selected from: 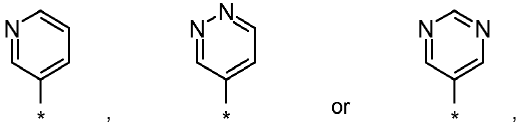 wherein * indicates the point of attachment of the group with the rest of the molecule and where the group is optionally substituted, one or more times, independently from each other, with a substituent selected from halogen, CN, OH, RR²N-, C₁-C₆-alkyl, C₁-C₆-haloalkyl, C₁-C₆-alkoxy, C₁-C₆-haloalkoxy, wherein C₁-C₆-alkyl and C₁-C₆-haloalkyl groups are optionally substituted with OH.

In a further embodiment of the above-mentioned aspect, the invention relates to compounds of formula (I), wherein:
- X: is a group selected from: 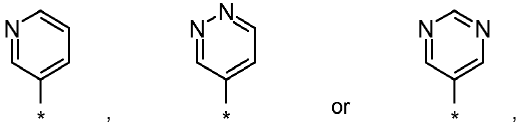 wherein * indicates the point of attachment of the group with the rest of the molecule and where the group is optionally substituted, one or more times, independently from each other, with a substituent selected from halogen, C₁-C₄-alkyl, C₁-C₄-haloalkyl, C₁-C₄-alkoxy, wherein C₁-C₄-alkyl and C₁-C₄-haloalkyl groups are optionally substituted with OH.

In a further embodiment of the above-mentioned aspect, the invention relates to compounds of formula (I), wherein:
- X: is a group selected from: 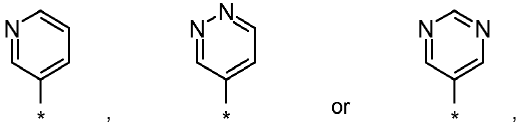 wherein * indicates the point of attachment of the group with the rest of the molecule and where the group is optionally substituted, one or more times, independently from each other, with a substituent selected from fluoro, chloro, methyl, trifluoromethyl or methoxy.

In a further embodiment of the above-mentioned aspect, the invention relates to compounds of formula (I), wherein:
- X: is a group selected from: 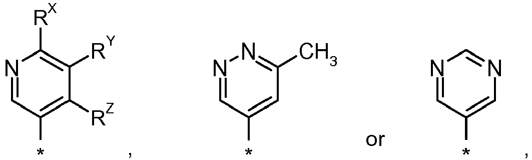 wherein * indicates the point of attachment of the group with the rest of the molecule and,
- R^{X}: is hydrogen or methyl,
- R^{Y}: is hydrogen, fluoro, chloro, methyl, trifluoromethyl or methoxy,
- R^{Z}: is hydrogen or methyl, wherein only one of R^{X}, R^{Y} and R^{Z} is different from hydrogen.

In a further embodiment of the above-mentioned aspect, the invention relates to compounds of formula (I), wherein:
- X: is 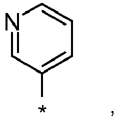 wherein * indicates the point of attachment of the group with the rest of the molecule and where the group is optionally substituted, one or more times, independently from each other, with a substituent selected from fluoro, chloro, methyl, trifluoromethyl or methoxy.

In a further embodiment of the above-mentioned aspect, the invention relates to compounds of formula (I), wherein:
- X: is 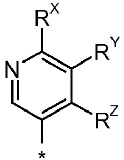 wherein * indicates the point of attachment of the group with the rest of the molecule and,
- R^{X}: is hydrogen or methyl,
- R^{Y}: is hydrogen, fluoro, chloro, methyl, trifluoromethyl or methoxy,
- R^{Z}: is hydrogen or methyl,
wherein only one of R^{X}, R^{Y} and R^{Z} is different from hydrogen.

In a further embodiment of the above-mentioned aspect, the invention relates to compounds of formula (I), wherein:
- X: is 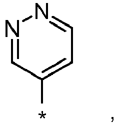 wherein * indicates the point of attachment of the group with the rest of the molecule and where the group is optionally substituted, one or more times, independently from each other, with a substituent selected from fluoro, chloro, methyl, trifluoromethyl or methoxy.

In a further embodiment of the above-mentioned aspect, the invention relates to compounds of formula (I), wherein:
- X: is 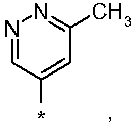 wherein * indicates the point of attachment of the group with the rest of the molecule.

In a further embodiment of the above-mentioned aspect, the invention relates to compounds of formula (I), wherein:
- X: is 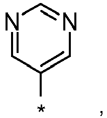 wherein * indicates the point of attachment of the group with the rest of the molecule and where the group is optionally substituted, one or more times, independently from each other, with a substituent selected from fluoro, chloro, methyl, trifluoromethyl or methoxy.

In a further embodiment of the above-mentioned aspect, the invention relates to compounds of formula (I), wherein:
- X: is 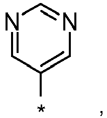 wherein * indicates the point of attachment of the group with the rest of the molecule.

In a further embodiment of the above-mentioned aspect, the invention relates to compounds of formula (I), wherein:
- R¹: is C₁-C₆-alkyl or C₁-C₆-haloalkyl, and where R¹ is optionally substituted with one or two substituents, independently from each other, selected from OH, CN, RO(CO)-, RR²N(CO)-, RR²NSO₂-, C₁-C₆-alkyl-(CO)(NH)SO₂- or 5-tetrazolyl.

In a further embodiment of the above-mentioned aspect, the invention relates to compounds of formula (I), wherein:
- R², R⁵: are, independently from each other, hydrogen or C₁-C₆-alkyl, where C₁-C₆-alkyl groups are optionally substituted, one or more times, independently from each other, with halogen.

In a further embodiment of the above-mentioned aspect, the invention relates to compounds of formula (I), wherein:
- R³, R⁴: are, independently from each other, C₁-C₆-alkyl or C₁-C₆-haloalkyl, and whereby R³ and R⁴ are optionally substituted with one or two substituents, independently from each other, selected from OH, CN, RO(CO)-, RR²N(CO)-, RR²NSO₂-, C₁-C₆-alkyl-(CO)(NH)SO₂- or 5-tetrazolyl;

In a further embodiment of the above-mentioned aspect, the invention relates to compounds of formula (I), wherein:
- R⁶, R⁷, R⁸, R⁹: are, independently from each other, C₁-C₆-alkyl or C₁-C₆-haloalkyl, and whereby R⁶, R⁷, R⁸ and R⁹ are optionally substituted with one or two substituents, independently from each other, selected from OH, CN, RO(CO)-, RR²N(CO)-, RR²NSO₂-, C₁-C₆-alkyl-(CO)(NH)SO₂- or 5-tetrazolyl.

In a further embodiment of the above-mentioned aspect, the invention relates to compounds of formula (I), wherein:
- R: is hydrogen or a C₁-C₆-alkyl group.

In a further embodiment of the above-mentioned aspect, the invention relates to compounds of formula (I), wherein:
- R¹: is C₁-C₄-alkyl, which is optionally substituted with one or two substituents, independently from each other, selected from OH, RO(CO)-, RR²N(CO)-, RR²NSO₂- or 5-tetrazolyl.

In a further embodiment of the above-mentioned aspect, the invention relates to compounds of formula (I), wherein:
- R², R⁵: are, independently from each other, hydrogen or C₁-C₄-alkyl, where C₁-C₄-alkyl groups are optionally substituted, one or more times, independently from each other, with halogen.

In a further embodiment of the above-mentioned aspect, the invention relates to compounds of formula (I), wherein:
- R³, R⁴: are, independently from each other, C₁-C₄-alkyl, and whereby R³ and R⁴ are optionally substituted with one or two substituents, independently from each other, selected from OH, RO(CO)-, RR²N(CO)-, RR²NSO₂-, C₁-C₄-alkyl-(CO)(NH)SO₂- or 5-tetrazolyl.

In a further embodiment of the above-mentioned aspect, the invention relates to compounds of formula (I), wherein:
- R⁶, R⁷, R⁸, R⁹: are, independently from each other, C₁-C₄-alkyl, and whereby R⁶, R⁷, R⁸ and R⁹ are optionally substituted with one or two substituents, independently from each other, selected from OH, RO(CO)-, RR²N(CO)-, RR²NSO₂- or 5-tetrazolyl.

In a further embodiment of the above-mentioned aspect, the invention relates to compounds of formula (I), wherein:
- R: is hydrogen or a C₁-C₄-alkyl group.

In a further embodiment of the above-mentioned aspect, the invention relates to compounds of formula (I), wherein:
- R¹: is propyl, which is optionally substituted with RO(CO)-.

In a further embodiment of the above-mentioned aspect, the invention relates to compounds of formula (I), wherein:
- R², R⁵: are, independently from each other, hydrogen or methyl.

In a further embodiment of the above-mentioned aspect, the invention relates to compounds of formula (I), wherein:
- R³, R⁴: are, independently from each other, C₁-C₃-alkyl, and whereby R³ and R⁴ are optionally substituted with one or two substituents, independently from each other, selected from OH, RO(CO)-, RR²N(CO)-, RR²NSO₂-, C₁-C₂-alkyl-(CO)(NH)SO₂- or 5-tetrazolyl.

In a further embodiment of the above-mentioned aspect, the invention relates to compounds of formula (I), wherein :
- R⁶, R⁷, R⁸, R⁹: are, independently from each other, C₁-C₄-alkyl, and whereby R⁶, R⁷, R⁸ and R⁹ are optionally substituted with one or two substituents, independently from each other, selected from OH, RO(CO)- or RR²N(CO)-.

In a further embodiment of the above-mentioned aspect, the invention relates to compounds of formula (I), wherein:
- R¹: is -CH₂-CH₂-CH₂-COOH.

In a further embodiment of the above-mentioned aspect, the invention relates to compounds of formula (I), wherein:
- R²: is hydrogen or methyl.

In a further embodiment of the above-mentioned aspect, the invention relates to compounds of formula (I), wherein:
- R²: is hydrogen.

In a further embodiment of the above-mentioned aspect, the invention relates to compounds of formula (I), wherein:
- R³: is C₁-C₃-alkyl, which is optionally substituted with one substituent, selected from OH, HO(CO)-, H₂N(CO)-, CH₃-(CO)(NH)SO₂- or 5-tetrazolyl.

In a further embodiment of the above-mentioned aspect, the invention relates to compounds of formula (I), wherein:
- R⁴: is -CH₂-CH₂-COOH;

In a further embodiment of the above-mentioned aspect, the invention relates to compounds of formula (I), wherein:
- R⁵: is hydrogen.

In a further embodiment of the above-mentioned aspect, the invention relates to compounds of formula (I), wherein:
- R⁶: is C₁-C₄-alkyl, which is optionally substituted with one substituent, selected from OH, RO(CO)- or H₂N(CO)-.

In a further embodiment of the above-mentioned aspect, the invention relates to compounds of formula (I), wherein:
- R⁷: is methyl.

In a further embodiment of the above-mentioned aspect, the invention relates to compounds of formula (I), wherein:
- R⁸: is -CH₂-CH₂-COOH.

In a further embodiment of the above-mentioned aspect, the invention relates to compounds of formula (I), wherein:
- R⁹: is C₂-C₃-alkyl, which is substituted with one substituent, selected from OH or HO(CO)-.

In a further embodiment of the above-mentioned aspect, the invention relates to compounds of formula (I), wherein:
- R: is hydrogen or methyl.

In a further embodiment of the above-mentioned aspect, the invention relates to compounds of formula (I), wherein :
- R: is hydrogen.

The term "substituted" means that one or more hydrogens on the designated atom is replaced with a selection from the indicated group, provided that the designated atom's normal valency under the existing circumstances is not exceeded, and that the substitution results in a stable compound. Combinations of substituents and/or variables are permissible only if such combinations result in stable compounds.

The term "optionally substituted" means optional substitution with the specified groups, radicals or moieties.

Ring system substituent means a substituent attached to an aromatic or nonaromatic ring system which, for example, replaces an available hydrogen on the ring system.

As used herein, the term "one or more", e.g. in the definition of the substituents of the compounds of the general formulae of the present invention, is understood as meaning "one, two, three, four or five, particularly one, two, three or four, more particularly one, two or three, even more particularly one or two".

The invention also includes all suitable isotopic variations of a compound of the invention. An isotopic variation of a compound of the invention is defined as one in which at least one atom is replaced by an atom having the same atomic number but an atomic mass different from the atomic mass usually or predominantly found in nature. Examples of isotopes that can be incorporated into a compound of the invention include isotopes of hydrogen, carbon, nitrogen, oxygen, phosphorus, sulphur, fluorine, chlorine, bromine and iodine, such as ²H(deuterium), ³H (tritium), ¹¹C, ¹³C, ¹⁴C, ¹⁵N, ¹⁷O, ¹⁸O, ³²P, ³³P, ³³S, ³⁴S, ³⁵S, ³⁶S, ¹⁸F, ³⁶Cl, ⁸²Br, ¹²³I, ¹²⁴I, ¹²⁹I and ¹³¹I, respectively. Certain isotopic variations of a compound of the invention, for example, those in which one or more radioactive isotopes such as ³H or ¹⁴C are incorporated, are useful in drug and/or substrate tissue distribution studies. Tritiated and carbon-14, i.e., ¹⁴C, isotopes are particularly preferred for their ease of preparation and detectability. Further, substitution with isotopes such as deuterium may afford certain therapeutic advantages resulting from greater metabolic stability, for example, increased in vivo half-life or reduced dosage requirements and hence may be preferred in some circumstances. Isotopic variations of a compound of the invention can generally be prepared by conventional procedures known by a person skilled in the art such as by the illustrative methods or by the preparations described in the examples hereafter using appropriate isotopic variations of suitable reagents.

Where the plural form of the word compounds, salts, polymorphs, hydrates, solvates and the like, is used herein, this is taken to mean also a single compound, salt, polymorph, isomer, hydrate, solvate or the like.

By "stable compound' or "stable structure" is meant a compound that is sufficiently robust to survive isolation to a useful degree of purity from a reaction mixture, and formulation into an efficacious therapeutic agent.

The compounds of this invention may contain one or more asymmetric centre, depending upon the location and nature of the various substituents desired. Asymmetric carbon atoms may be present in the (R) or (S) configuration, resulting in racemic mixtures in the case of a single asymmetric centre, and diastereomeric mixtures in the case of multiple asymmetric centres. In certain instances, asymmetry may also be present due to restricted rotation about a given bond, for example, the central bond adjoining two substituted aromatic rings of the specified compounds.

The compounds of the present invention may contain sulphur atoms which are asymmetric, such as an asymmetric sulphoxide or sulphoximine group, of structure: 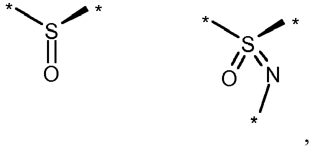 for example,
in which * indicates atoms to which the rest of the molecule can be bound.

Substituents on a ring may also be present in either cis or trans form. It is intended that all such configurations (including enantiomers and diastereomers), are included within the scope of the present invention.

Preferred compounds are those which produce the more desirable biological activity. Separated, pure or partially purified isomers and stereoisomers or racemic or diastereomeric mixtures of the compounds of this invention are also included within the scope of the present invention. The purification and the separation of such materials can be accomplished by standard techniques known in the art.

The optical isomers can be obtained by resolution of the racemic mixtures according to conventional processes, for example, by the formation of diastereoisomeric salts using an optically active acid or base or formation of covalent diastereomers. Examples of appropriate acids are tartaric, diacetyltartaric, ditoluoyltartaric and camphorsulfonic acid. Mixtures of diastereoisomers can be separated into their individual diastereomers on the basis of their physical and/or chemical differences by methods known in the art, for example, by chromatography or fractional crystallisation. The optically active bases or acids are then liberated from the separated diastereomeric salts. A different process for separation of optical isomers involves the use of chiral chromatography (e.g., chiral HPLC columns), with or without conventional derivatisation, optimally chosen to maximise the separation of the enantiomers. Suitable chiral HPLC columns are manufactured by Daicel, e.g., Chiracel, O., D. and Chiracel, O.,J. among many others, all routinely selectable. Enzymatic separations, with or without derivatisation, are also useful. The optically active compounds of this invention can likewise be obtained by chiral syntheses utilizing optically active starting materials.

In order to limit different types of isomers from each other, reference is made to IUPAC Rules Section E (Pure Appl Chem 45, 11-30, 1976).

Isolation of a single stereoisomer, e.g. a single enantiomer or a single diastereomer, of a compound of the present invention may be achieved by any suitable state of the art method, such as chromatography, especially chiral chromatography, for example.

Further, the compounds of the present invention may exist as tautomers. For example, any compound of the present invention which contains a pyrazole moiety as a heteroaryl group for example can exist as a 1H tautomer, or a 2H tautomer, or even a mixture in any amount of the two tautomers, or a triazole moiety for example can exist as a 1H tautomer, a 2H tautomer, or a 4H tautomer, or even a mixture in any amount of said 1H, 2H and 4H tautomers, namely:

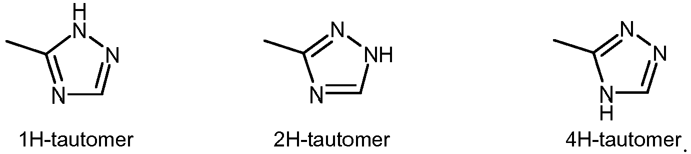

The present invention includes all possible tautomers of the compounds of the present invention as single tautomers, or as any mixture of said tautomers, in any ratio.

Further, the compounds of the present invention can exist as N-oxides, which are defined in that at least one nitrogen of the compounds of the present invention is oxidised. The present invention includes all such possible N-oxides.

The present invention also relates to useful forms of the compounds as disclosed herein, such as metabolites, hydrates, solvates, prodrugs, salts, in particular pharmaceutically acceptable salts, and co-precipitates.

The compounds of the present invention can exist as a hydrate, or as a solvate, wherein the compounds of the present invention contain polar solvents, in particular water, methanol or ethanol for example as structural element of the crystal lattice of the compounds. The amount of polar solvents, in particular water, may exist in a stoichiometric or non-stoichiometric ratio. In the case of stoichiometric solvates, *e.g.* a hydrate, hemi-, (semi-), mono-, sesqui-, di-, tri-, tetra-, penta- *etc.* solvates or hydrates, respectively, are possible. The present invention includes all such hydrates or solvates.

Further, the compounds of the present invention can exist in free form, *e.g.* as a free base, or as a free acid, or as a zwitterion, or can exist in the form of a salt. Said salt may be any salt, either an organic or inorganic addition salt, particularly any pharmaceutically acceptable organic or inorganic addition salt, customarily used in pharmacy.

The term "pharmaceutically acceptable salt" refers to a relatively non-toxic, inorganic or organic acid addition salt of a compound of the present invention, for example, see Berge, S. M. et al. (1977) Pharmaceutical Salts. J. Pharm. Sci., 66, 1-19.

A suitable pharmaceutically acceptable salt of the compounds of the present invention may be, for example, an acid-addition salt of a compound of the present invention bearing a nitrogen atom, in a chain or in a ring, for example, which is sufficiently basic, such as an acid-addition salt with an inorganic acid, such as hydrochloric, hydrobromic, hydroiodic, sulfuric, bisulfuric, phosphoric, or nitric acid, for example, or with an organic acid, such as formic, acetic, acetoacetic, pyruvic, trifluoroacetic, propionic, butyric, hexanoic, heptanoic, undecanoic, lauric, benzoic, salicylic, 2-(4-hydroxybenzoyl)-benzoic, camphoric, cinnamic, cyclopentanepropionic, digluconic, 3-hydroxy-2-naphthoic, nicotinic, pamoic, pectinic, persulfuric, 3-phenylpropionic, picric, pivalic, 2-hydroxyethanesulfonate, itaconic, sulfamic, trifluoromethanesulfonic, dodecylsulfuric, ethansulfonic, benzenesulfonic, para-toluenesulfonic, methansulfonic, 2-naphthalenesulfonic, naphthalinedisulfonic, camphorsulfonic acid, citric, tartaric, stearic, lactic, oxalic, malonic, succinic, malic, adipic, alginic, maleic, fumaric, D-gluconic, mandelic, ascorbic, glucoheptanoic, glycerophosphoric, aspartic, sulfosalicylic, hemisulfuric, or thiocyanic acid, for example.

Further, another suitably pharmaceutically acceptable salt of a compound of the present invention which is sufficiently acidic, is an alkali metal salt, for example a sodium or potassium salt, an alkaline earth metal salt, for example a calcium or magnesium salt, an ammonium salt or a salt with an organic base which affords a physiologically acceptable cation, for example a salt with N-methyl-glucamine, dimethyl-glucamine, ethyl-glucamine, lysine, dicyclohexylamine, 1,6-hexadiamine, ethanolamine, glucosamine, sarcosine, serinol, tris-hydroxy-methyl-aminomethane, aminopropandiol, sovak-base, 1-amino-2,3,4-butantriol. Additionally, basic nitrogen containing groups may be quaternised with such agents as lower alkyl halides such as methyl, ethyl, propyl, and butyl chlorides, bromides and iodides ; dialkyl sulfates like dimethyl, diethyl, and dibutyl sulfate; and diamyl sulfates, long chain halides such as decyl, lauryl, myristyl and strearyl chlorides, bromides and iodides, aralkyl halides like benzyl and phenethyl bromides and others.

Those skilled in the art will further recognise that acid addition salts of the claimed compounds may be prepared by reaction of the compounds with the appropriate inorganic or organic acid via any of a number of known methods. Alternatively, alkali and alkaline earth metal salts of acidic compounds of the invention are prepared by reacting the compounds of the invention with the appropriate base via a variety of known methods.

The present invention includes all possible salts of the compounds of the present invention as single salts, or as any mixture of said salts, in any ratio.

Furthermore, the present invention includes all possible crystalline forms, or polymorphs, of the compounds of the present invention, either as single polymorphs, or as a mixture of more than one polymorphs, in any ratio.

When radicals in the compounds of the present invention are substituted, the radicals may be mono- or polysubstituted, unless specified otherwise. In the context of the present invention, all radicals which occur more than once are defined independently of one another. Substitution by one, two or three identical or different substituents is preferred.

In the context of the present invention, the term "treatment" or "treating" includes inhibition, retardation, checking, alleviating, attenuating, restricting, reducing, suppressing, repelling or healing of a disease, a condition, a disorder, an injury or a health problem, or the development, the course or the progression of such states and/or the symptoms of such states. The term "therapy" is understood here to be synonymous with the term "treatment".

The terms "prevention", "prophylaxis" or "preclusion" are used synonymously in the context of the present invention and refer to the avoidance or reduction of the risk of contracting, experiencing, suffering from or having a disease, a condition, a disorder, an injury or a health problem, or a development or advancement of such states and/or the symptoms of such states.

The treatment or prevention of a disease, a condition, a disorder, an injury or a health problem may be partial or complete.

The compounds of the present invention have an unforeseeable, valuable spectrum of pharmacological and pharmacokinetic activity. They are therefore suitable for use as medicaments for treatment and/or prophylaxis of disorders in humans and animals. The term "treatment" in the context of the present invention includes prophylaxis. The pharmaceutical efficacy of the compounds of the present invention can be explained by the action thereof as an AKR1C3 inhibitor. As shown in Tables 1 (Example 47, inhibition of AKR1C3 in a biochemical assay) and 2 (Example 48, inhibition of AKR1C3 in a cell-based system), the compounds of the present invention are potent inhibitors of the AKR1C3 enzyme.

In addition, the compounds of the present invention are suitable for treatment and/or prophylaxis of uterine leiomyoma, of uterine bleeding disorders, of dysmenorrhoea, of prostate carcinoma, of prostate hyperplasia, of acne, of seborrhoea, of hair loss, of premature sexual maturity, of polycystic ovary syndrome, of breast cancer, of lung cancer, of endometrial carcinoma, of renal cell carcinoma, of bladder carcinoma, of non-Hodgkins lymphoma, of chronic obstructive pulmonary disease (COPD), of obesity, or of inflammation-related pain.

The present invention further provides for the use of the compounds of the present invention for production of a medicament for treatment and/or prophylaxis of disorders, especially of the aforementioned disorders.

The present invention further provides a method for treatment and/or prophylaxis of disorders, especially the aforementioned disorders, using an effective amount of the compounds of the present invention.

The present invention further provides for the use of the compounds of the present invention for treatment and/or prophylaxis of disorders, especially of the aforementioned disorders.

The present invention further provides the compounds of the present invention for use in a method for treatment and/or prophylaxis of the aforementioned disorders.

The present invention further provides medicaments comprising at least one inventive compound and at least one or more than one further active ingredient, especially for treatment and/or prophylaxis of the aforementioned disorders. Preferred examples of suitable combination active ingredients include: selective oestrogen receptor modulators (SERMs), oestrogen receptor (ER) antagonists, aromatase inhibitors, 17β-HSD1 inhibitors, steroid sulphatase (STS) inhibitors, GnRH agonists and antagonists, kisspeptin receptor (KISSR) antagonists, selective androgen receptor modulators (SARMs), androgens, 5α-reductase inhibitors, C(17,20)-lyase inhibitors, selective progesterone receptor modulators (SPRMs), gestagens, antigestagens, oral contraceptives, inhibitors of mitogen-activated protein (MAP) kinases and inhibitors of the MAP kinases (Mkk3/6, Mek1/2, Erk1/2), inhibitors of the protein kinases B (PKBα/β/γ; Akt1/2/3), inhibitors of the phosphoinositide 3-kinases (PI3K), inhibitors of cyclin-dependent kinase (CDK1/2), inhibitors of the hypoxia-induced signalling pathway (HIF1alpha inhibitors, activators of prolylhydroxylases), histone deacetylase (HDAC) inhibitors, prostaglandin F receptor (FP) (PTGFR) antagonists, and non-steroidal inflammation inhibitors (NSAIDs).

For example, the compounds of the present invention can be combined with known antihyperproliferative, cytostatic or cytotoxic substances for treatment of cancers. In addition, the compounds of the present invention can also be used in combination with radiotherapy and/or surgical intervention.

Examples of suitable combination active ingredients include:
131I-chTNT, abarelix, abiraterone, aclarubicin, aldesleukin, alemtuzumab, alitretinoin, altretamine, aminoglutethimide, amrubicin, amsacrine, anastrozole, arglabin, arsentrioxidas, asparaginase, azacitidine, basiliximab, RDEA 119, belotecan, bendamustine, bevacizumab, bexarotene, bicalutamide, bisantrene, bleomycin, bortezomib, buserelin, busulfan, cabazitaxel, calcium folinate, calcium levofolinate, capecitabine, carboplatin, carmofur, carmustine, catumaxomab, celecoxib, celmoleukin, cetuximab, chlorambucil, chlormadinone, chlormethine, cisplatin, cladribine, clodronic acid, clofarabine, crisantaspase, cyclophosphamide, cyproterone, cytarabine, dacarbazine, dactinomycin, darbepoetin alfa, dasatinib, daunorubicin, decitabine, degarelix, denileukin diftitox, denosumab, deslorelin, dibrospidium chloride, docetaxel, doxifluridine, doxorubicin, doxorubicin + oestrone, eculizumab, edrecolomab, elliptinium acetate, eltrombopag, endostatin, enocitabine, epirubicin, epitiostanol, epoetin alfa, epoetin beta, eptaplatin, eribulin, erlotinib, oestradiol, oestramustine, etoposide, everolimus, exemestane, fadrozole, filgrastim, fludarabine, fluorouracil, flutamide, formestane, fotemustine, fulvoestrant, gallium nitrate, ganirelix, gefitinib, gemcitabine, gemtuzumab, glutoxim, goserelin, histamine dihydrochloride, histrelin, hydroxycarbamide, 1-125 pellets, ibandronic acid, ibritumomab tiuxetan, idarubicin, ifosfamide, imatinib, imiquimod, improsulfan, interferon alfa, interferon beta, interferon gamma, ipilimumab, irinotecan, ixabepilone, lanreotide, lapatinib, lenalidomide, lenograstim, lentinan, letrozole, leuprorelin, levamisole, lisuride, lobaplatin, lomustine, lonidamine, masoprocol, medroxyprogesterone, megestrol, melphalan, mepitiostan, mercaptopurine, methotrexate, methoxsalen, methyl aminolevulinate, methyltestosterone, mifamurtide, miltefosine, miriplatin, mitobronitol, mitoguazone, mitolactol, mitomycin, mitotane, mitoxantrone, nedaplatin, nelarabine, nilotinib, nilutamide, nimotuzumab, nimustine, nitracrine, ofatumumab, omeprazole, oprelvekin, oxaliplatin, p53 gene therapy, paclitaxel, palifermin, palladium-103 pellets, pamidronic acid, panitumumab, pazopanib, pegaspargase, pEG-epoetin beta (methoxy PEG-epoetin beta), pegfilgrastim, peginterferon alfa-2b, pemetrexed, pentazocine, pentostatin, peplomycin, perfosfamid, picibanil, pirarubicin, plerixafor, plicamycin, poliglusam, polyoestradiol phosphate, polysaccharide-K, porfimer sodium, pralatrexate, prednimustine, procarbazine, quinagolide, radium-223 chloride, raloxifen, raltitrexed, ranimustine, razoxane, regorafenib, risedronic acid, rituximab, romidepsin, romiplostim, sargramostim, sipuleucel-T, sizofiran, sobuzoxan, sodium glycididazole, sorafenib, streptozocin, sunitinib, talaporfin, tamibarotene, tamoxifen, tasonermin, teceleukin, tegafur, tegafur + gimeracil + oteracil, temoporfin, temozolomide, temsirolimus, teniposide, testosterone, tetrofosmin, thalidomide, thiotepa, thymalfasin, tioguanine, tocilizumab, topotecan, toremifene, tositumomab, trabectedin, trastuzumab, treosulfan, tretinoin, trilostane, triptorelin, trofosfamide, tryptophan, ubenimex, valrubicin, vandetanib, vapreotide, vemurafenib, vinblastine, vincristine, vindesine, vinflunine, vinorelbine, vorinostat, vorozole, yttrium-90 glass microspheres, zinostatin, zinostatin stimalamer, zoledronic acid, zorubicin.

The present invention preferably relates to medicaments comprising at least one inventive compound and one or more of the following active ingredients, especially for treatment and/or prophylaxis of androgen receptor-dependent proliferative disorders:
LHRH (luteinizing hormone-releasing hormone) agonists,
LHRH (luteinizing hormone-releasing hormone) antagonists,
C(17,20)-lyase inhibitors,
type I 5-α-reductase inhibitors,
type II 5-α-reductase inhibitors,
mixed type I/II 5-α-reductase inhibitors,
α-radiation-emitting radiopharmaceuticals for treatment of bone metastases, for example radium-223 chloride,
cytostatics,
VEGF (Vascular Endothelial Growth Factor) kinase inhibitors,
antigestagens,
antioestrogens,
EGF antibodies,
oestrogens or
other androgen receptor antagonists,
poly(ADP-ribose) polymerase I inhibitors, or
bi-specific T-cell engagers (BiTE) coupled to a cell surface protein, for example prostate-specific membrane antigen (PSMA).

The invention also relates to pharmaceutical formulations comprising at least one compound of the general formula I (or physiologically acceptable addition salts with organic and inorganic acids) and to the use of these compounds for production of medicaments, especially for the aforementioned indications.

The compounds can be used for the aforementioned indications after either oral or parenteral administration.

The compounds of the present invention can act systemically and/or locally. For this purpose, they can be administered in a suitable manner, for example by the oral, parenteral, pulmonal, nasal, sublingual, lingual, buccal, rectal, dermal, transdermal, conjunctival, otic route, or as an implant or stent.

The compounds of the present invention can be administered in administration forms suitable for these administration routes.

Suitable administration forms for oral administration are those which release the compounds of the present invention in a rapid and/or modified manner, work according to the prior art and contain the compounds of the present invention in crystalline and/or amorphous and/or dissolved form, for example tablets (uncoated or coated tablets, for example with enteric or retarded-dissolution or insoluble coatings which control the release of the inventive compound), tablets or films/wafers which disintegrate rapidly in the oral cavity, films/lyophilizates, capsules (for example hard or soft gelatin capsules), sugar-coated tablets, granules, pellets, powders, emulsions, suspensions, aerosols or solutions.

Parenteral administration can be accomplished with avoidance of an absorption step (for example by an intravenous, intraarterial, intracardial, intraspinal or intralumbar route) or with inclusion of an absorption (for example by an intramuscular, subcutaneous, intracutaneous, percutaneous or intraperitoneal route). Suitable administration forms for parenteral administration include injection and infusion formulations in the form of solutions, suspensions, emulsions, lyophilizates or sterile powders.

For the other administration routes, suitable examples are inhalation medicaments (including powder inhalers, nebulizers), nasal drops, solutions or sprays; tablets for lingual, sublingual or buccal administration, films/wafers or capsules, suppositories, ear or eye preparations, vaginal capsules, aqueous suspensions (lotions, shaking mixtures), lipophilic suspensions, ointments, creams, transdermal therapeutic systems (for example patches), milk, pastes, foams, dusting powders, implants, intrauterine coils, vaginal rings or stents.

The compounds of the present invention can be converted to the administration forms mentioned. This can be done in a manner known per se, by mixing with inert, nontoxic, pharmaceutically suitable excipients. These excipients include carriers (for example microcrystalline cellulose, lactose, mannitol), solvents (e.g. liquid polyethylene glycols), emulsifiers and dispersing or wetting agents (for example sodium dodecylsulphate, polyoxysorbitan oleate), binders (for example polyvinylpyrrolidone), synthetic and natural polymers (for example albumin), stabilizers (e.g. antioxidants, for example ascorbic acid), dyes (e.g. inorganic pigments, for example iron oxides) and flavour and/or odour correctants.

The present invention further provides medicaments which comprise at least one inventive compound, typically together with one or more inert, nontoxic, pharmaceutically suitable excipients, and the use thereof for the aforementioned purposes.

In the case of oral administration, the amount is about 0.01 to 100 mg/kg of body weight per day. The amount of a compound of the formula I to be administered varies within a wide range and can cover any effective amount. Depending on the condition to be treated and the mode of administration, the amount of the compound administered may be 0.01 - 100 mg/kg of body weight per day.

In spite of this, it may be necessary to deviate from the amounts specified, specifically depending on body weight, administration route, and individual behaviour towards the active ingredient, type of formulation, and time or interval of administration. For instance, less than the aforementioned minimum amount may be sufficient in some cases, while the upper limit mentioned has to be exceeded in other cases. In the case of administration of greater amounts, it may be advisable to divide them into several individual doses over the day.

The percentages in the tests and examples which follow are, unless indicated otherwise, percentages by weight; parts are parts by weight. Solvent ratios, dilution ratios and concentration data for liquid/liquid solutions are based in each case on volume.

The present invention further provides medicaments for treatment and prophylaxis of endometriosis, of uterine leiomyoma, of uterine bleeding disorders, of dysmenorrhoea, of prostate carcinoma, of prostate hyperplasia, of acne, of seborrhoea, of hair loss, of premature sexual maturity, of polycystic ovary syndrome, of breast cancer, of lung cancer, of endometrial carcinoma, of renal cell carcinoma, of bladder carcinoma, of non-Hodgkins lymphoma, of chronic obstructive pulmonary disease (COPD), of obesity, or of inflammation-related pain.

The present invention further provides for the use of the compounds of general formula (I), in the form of a pharmaceutical formulation, for enteral, parenteral, vaginal, intrauterine and oral administration.

**Abbreviations and acronyms:**

| | |
|---|---|
| DMF | *N*,*N*-dimethylformamide |
| DMSO | dimethyl sulphoxide |
| NMP | 1-methylpyrrolidin-2-one |
| THF | tetrahydrofuran |
| h | hour(s) |
| HPLC | high-pressure, high-performance liquid chromatography |
| LC-MS | liquid chromatography-coupled mass spectroscopy |
| ES-MS | electrospray mass spectrometry |
| min | minute(s) |
| MS | mass spectrometry |
| NMR | nuclear magnetic resonance spectroscopy |
| PDA | photodiode array detector |
| Rt | retention time |
| TFA | trifluoroacetic acid |
| UPLC- MS | ultra pressure liquid chromatography-coupled mass spectrometry |
| room temp. / RT | room temperature |
| i.d. | internal diameter |
| PDA | photodiode array detector |

The compounds of the present invention can be prepared as described in the following section. The Synthesis Schemes and the procedures described below illustrate general synthetic routes to the compounds of general formula (I) of the invention and are not intended to be limiting. The order of transformations as exemplified in the Synthesis Schemes can be modified in various ways. The order of transformations exemplified in the Synthesis Schemes is therefore not intended to be limiting. In addition, interconversion of any of the substituents can be achieved before and/or after the exemplified transformations. These modifications can be such as the introduction of protecting groups, cleavage of protecting groups, exchange, reduction or oxidation of functional groups, halogenation, metallation, substitution or other reactions known to the person skilled in the art. These transformations include those which introduce a functionality which allows for further interconversion of substituents. Appropriate protecting groups and their introduction and cleavage are well-known from the literature (see for example Wuts, P., G., M. and Greene, T., W. (2006). Protective Groups in Organic Synthesis, 4th edition, Wiley). Specific examples are described in the subsequent paragraphs. Further, it is possible that two or more successive steps may be performed without work-up between said steps, e.g. a "one-pot" reaction.

### Syntheses of Compounds (Overview):

Depending of the definition of the group A in the general formula (I), various synthetic routes have to be used. To demonstrate these different routes, subsets of compounds of the general formula (I) are depicted in the following Synthesis Schemes as IC1, IC2, IC3, IC4, IC5, IC6, IC7, IC8 and IC9.

As mentioned in the section before, some of the inventive compounds may show substitutents which can be transformed to other subsitutents by reaction methods known to the person skilled in the art. For example, if the compounds of the present invention display a carboxylic ester group such as a methyl or ethyl ester, hydrolysis of the ester as described in the literature can be performed to lead to compounds bearing a carboxylic acid group. For this purpose, a compound of the present invention with an ester group is mixed with a solvent such as tetrahydrofuran (THF), methanol or dimethyl sulphoxide (DMSO) or with a mixture of methanol and THF. Then aqueous sodium hydroxide solution or aqueous lithium hydroxide solution is added and the mixture is stirred at room temperature. The mixture is optionally heated. If the ester is a tert-butyl carboxylate, it can be converted to the carboxylic acid by methods known from the literature, for example with trifluoroacetic acid in dichloromethane or chloroform or with hydrogen chloride in 1,4-dioxane. The reaction with trifluoroacetic acid in dichloromethane is preferred.

The compounds of the present invention which have a carboxylic acid or a carboxylic ester group can be reduced to compounds with a hydroxyl group by methods known in the art. For example a compound with a carboxylic acid moiety can be treated in THF with isobutyl chloroformate in the presence of triethylamine followed after one hour by treatment with sodium borohydride or the compound can be treated with diisobutylaluminum hydride to form the compounds with a hydroxyl group.

### Synthesis of subsets of the inventive compounds

Some of the compounds of the present invention can be prepared proceeding from 17-oxoestra-1,3,5(10)-triene-3-sulfonyl chloride (CAS 148259-10-3, Steroids (1993), 58(3), 106-11) according to Synthesis Scheme 1.

Reaction of CAS 148259-10-3 with primary or secondary amines in the presence of a tertiary amine such as triethylamine in solvents such as DMF leads to the formation of intermediates 1.

Intermediates 2 can be prepared by methods related to those described in WO 20140009274 and WO 2013045407. In addition, the reaction of intermediates 1 with lithium bis(trimethylsilyl)amide in suitable solvents such as toluene and THF or mixtures of them and subsequent addition of N,N-bis(trifluoromethanesulfonyl)aniline leads to intermediates 2.

The subset IC1 of the compounds of the present invention, in which A represents the group -SO₂NR²R⁹, is prepared from intermediates 2 with a Suzuki reaction in a similar manner as described in WO 20140009274 and WO 2013045407.

As mentioned above, functional group transformations starting from the subset IC1 of the compounds can be performed to afford additional inventive compounds.

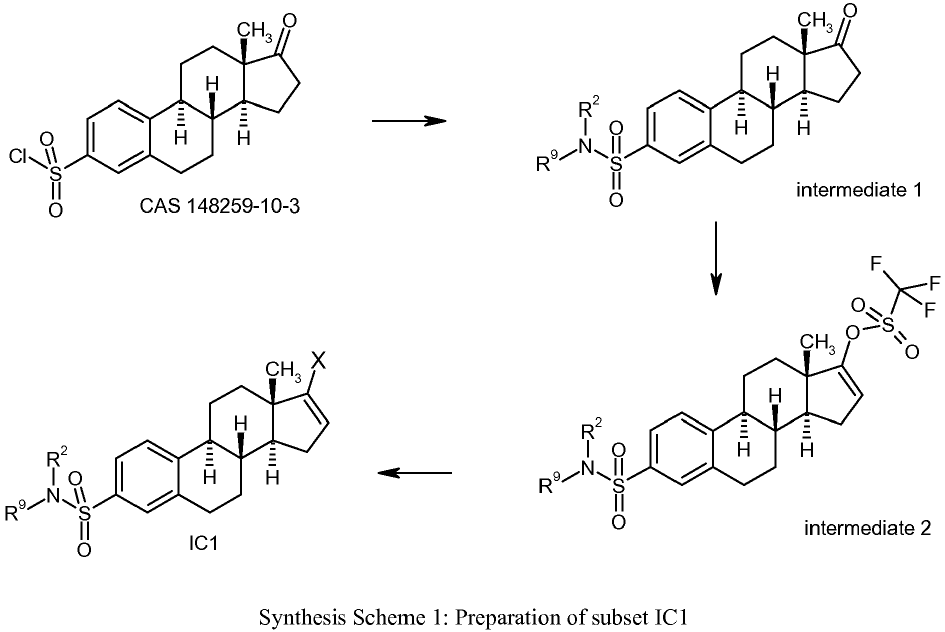

The subset IC2 of the compounds of the present invention, in which A represents the group -SO₂R⁶, can be prepared starting from 3-sulfanylestra-1,3,5(10)-trien-17-one (CAS 1670-31-1; Li, Pui Kai et al, Steroids, 58(3), 106-11; 1993) according to Synthesis Scheme 2.

Reaction with optionally substituted alkyl chloride, alkyl bromide or alkyl iodide in the presence of a base leads to intermediates 3. Bases such as cesium carbonate, potassium carbonate or sodium hydride in a solvent such as DMF, NMP or DMSO can be applied. The reaction with an optionally substituted alkylbromide or alkyliodide in DMSO in the presence of potassium carbonate is preferred.

Oxidation of intermediates 3 using *meta*-chloroperoxybenzoic acid (*m*CPBA) in dichloromethane leads to intermediates 4 displaying a sulfone moiety.

Intermediates 5 can be prepared by methods described for the preparation of intermediates 2 in Synthesis Scheme 1. The use of trifluoromethansulfonic anhydride and triethylamine in dichloromethane is preferred.

The subset IC2 of the compounds of the present invention can be prepared by a Suzuki reaction using similar conditions to those described in WO 20140009274 and WO 2013045407. The use of dichlorobis(triphenylphosphine)palladium(II) in a mixture of 2M aqueous sodium carbonate solution and dioxane is preferred.

Analogously to Synthesis Scheme 1, compounds of the present invention with carboxylic acid groups can be prepared from the subset IC2 of compounds of Synthesis Scheme 2 with carboxylic ester groups.

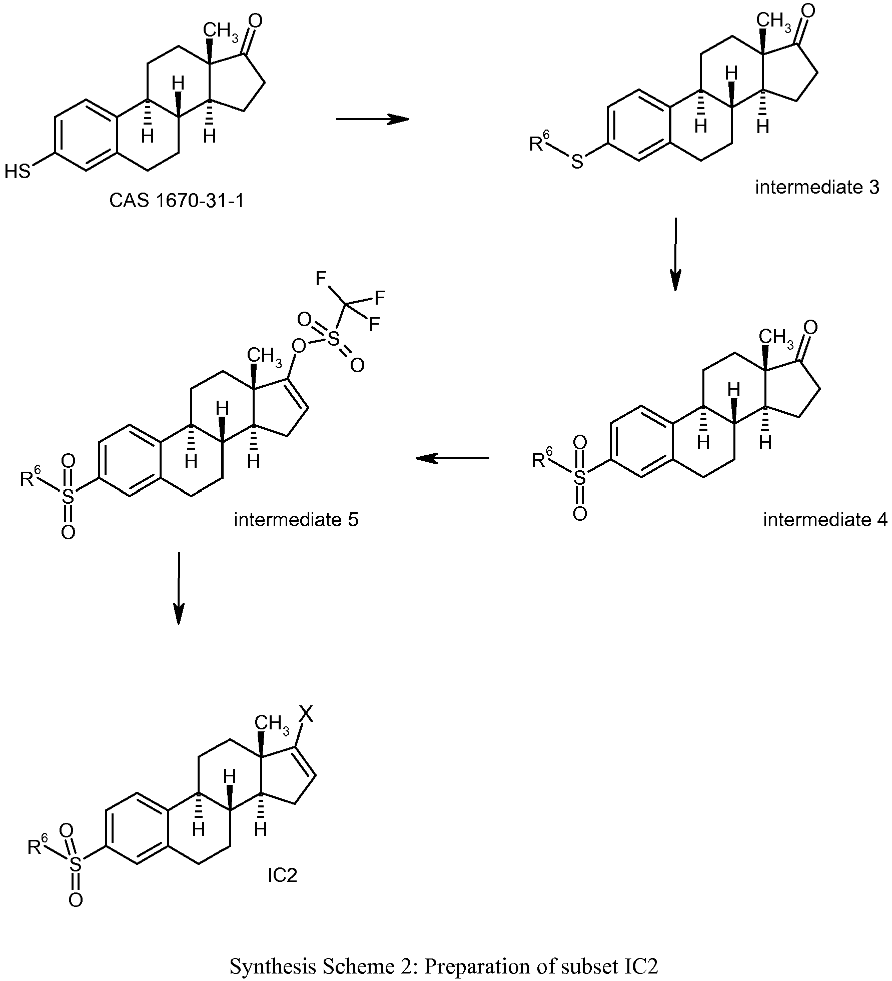

Another subset IC3 of the compounds of the present invention, in which A represents the group -NH-COR³, can be prepared proceeding from 3-aminoestra-1,3,5(10)-trien-17-one (CAS18119-98-7; Schoen, U. et al. (2005). Tetrahedron Letters, 46(42), 7111 - 7115;) according to Synthesis Scheme 3.

An amide coupling reaction with a carboxylic acid leads to the formation of intermediates 6. Coupling reagents can be applied. The use of HATU in the presence of 4-methylmorpholine is preferred. Intermediates 7 and 8 can be prepared using methods known in the literature (WO 2013045407, Synthesis scheme 3). A subset IC3 of the compounds of the present invention can be prepared by a Suzuki reaction using similar conditions described in WO 20140009274 and WO 2013045407. Starting from the subset IC3 another subset IC4 of compounds of the present invention, in which A represents the group -NR¹⁰-COR³ (whereby R¹⁰ has the meaning of C₁-C₆-alkyl), can be prepared by alkylation reactions. Treating IC3 with a suitable base and an alkyl halide in a solvent such as DMF leads to the formation of IC4. Sodium hydride is preferred as base. Polar solvents such as DMF or NMP can be applied. DMF is preferred. In the case that the substituent R³ in IC3 or IC4 contains functional groups, these groups can be transformed to other functional groups by methods known from the literature to form additional inventive compounds. For example, if R³ displays a carboxylic ester group, this group can be transformed to a carboxylic acid group or a hydroxyl group as described in Synthesis Scheme 1. The carboxylic acids can be reacted with amines to form carboxamides using coupling methodologies (for example methologies based on HATU) affording additional compounds of the present invention.

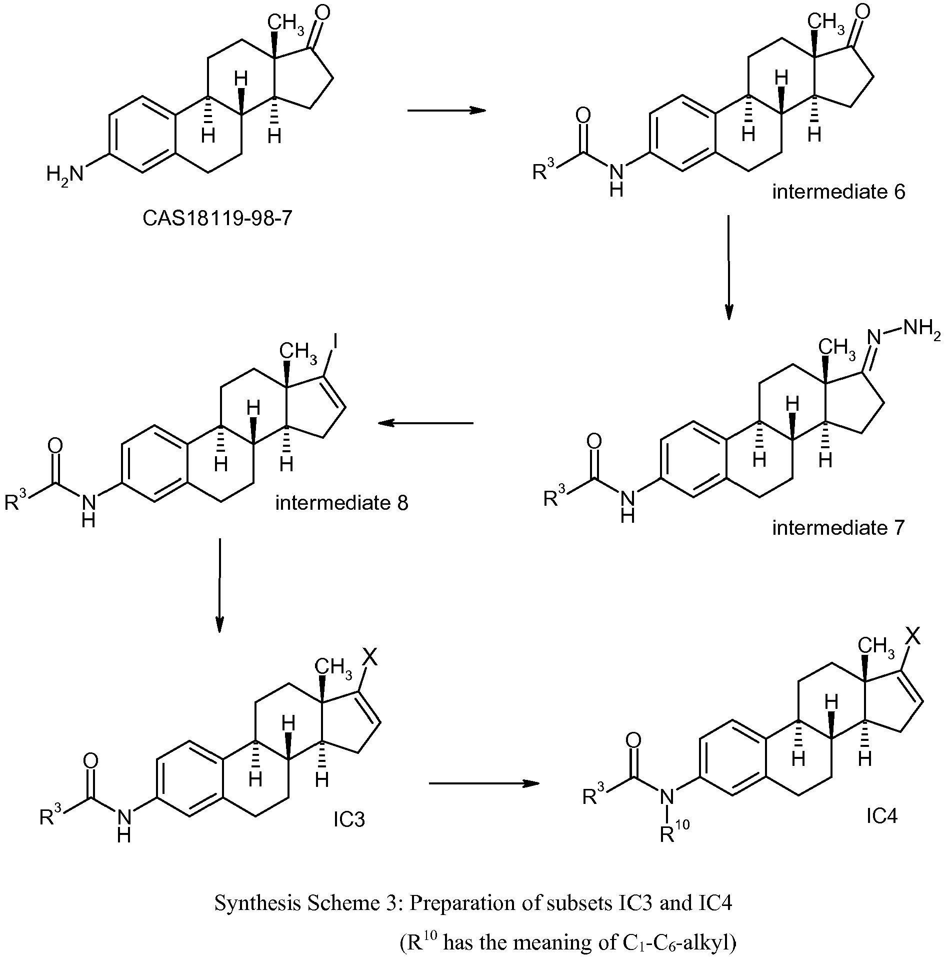

Starting from oestra-1,3,5(10),16-tetraene derivatives (described in WO 2013045407) intermediates 9 can be prepared by a *Curtius* rearrangement using diphenylphosphoryl azide and triethylamine in *tert-*butanol (Shioiri, T. et al. (1972). Journal of the American Chemical Society, 94, 17, 6203-6205). Intermediates 9 can be transformed to intermediates 10 using trifluoroacetic acid in dichloromethane. Intermediates 11 can be prepared from intermediates 10 by methods known from the literature. For example, a reductive amination can be applied.

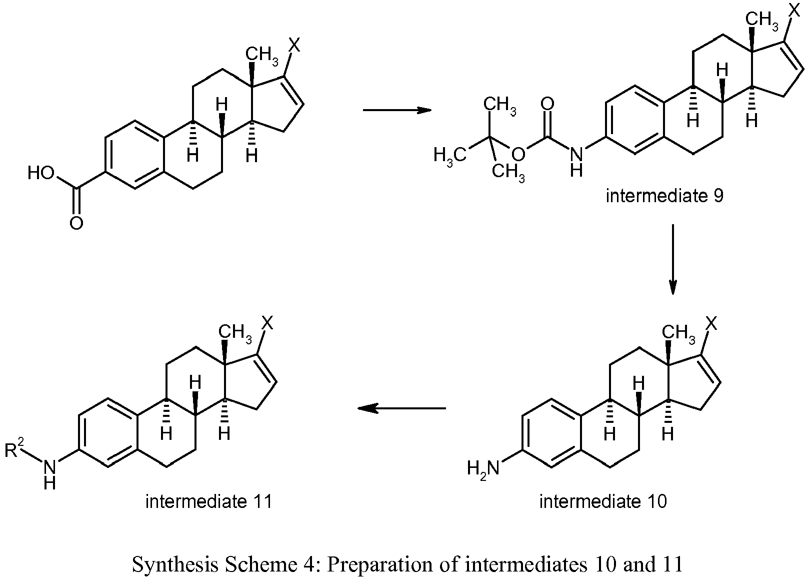

Intermediates 10 and 11 can be transformed to the subsets IC5, IC6, IC7 and IC8.

For the preparation of the subset IC5, in which A represents the group -NR²-SO₂R⁸, a reaction of an intermediate 10 or 11 with a sulfonylchloride in the presence of a tertiary amine such as triethylamine can be applied in a suitable solvent such as DMF.

For the preparation of the subset IC6, in which A represents the group -NR²-COR³, a reaction of intermediates 10 or 11 with carboxylic acids can be applied. The use of HATU in the presence of 4-methylmorpholine is preferred. In addition, compounds of the subset IC6 can also be obtained from intermediates 10 or 11 using carboxylic acid chlorides and a base such as triethylamine. For example acetylchloride and triethylamine can be applied for the synthesis of a subset of compounds in which R³ represents methyl.

For the preparation of the subset IC7, in which A represents the group -NR²-CO-NR⁴R⁵, a reaction of intermediates 10 or 11 with isocyanates can be used.

For the synthesis of the subset IC8, in which A represents the group -NR¹R², the intermediates 10 or 11 can be reacted with optionally substituted alkyl halogenides in the presence of a base such as potassium carbonate in a suitable solvent or a solvent mixture such as a mixture of DMF and acetonitrile.

In the case that R¹, R³, R⁴ or R⁸ in IC6, IC7 or IC8 stand for a functional group, this group can be transformed to another functional group. For example, if R¹, R³, R⁴ or R⁸ in IC6, IC7 or IC8 stand for a carboxylic ester, it can be transformed to a carboxylic acid via saponification or to an alcohol by reduction. For example, in the case that R³, R⁴ or R⁸ in IC5, IC6 or IC7 stand for a sulfamoyl group (-SO₂NH₂), this group can be transformed to an acetylsulfamoyl group using acetic acid, 1-(3-dimethylaminopropyl)-3-ethylcarbodiimide hydrochloride (EDC) and 4-dimethylaminopyridine (DMAP) in dichloromethane.

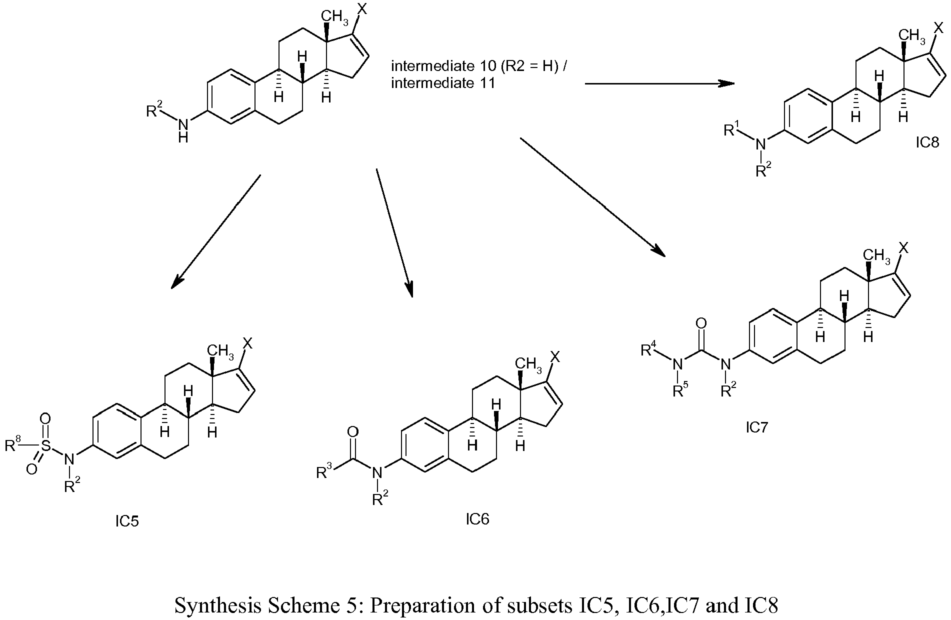

Another subset IC9 of compounds of the present invention, in which A represents the group -NH-COR³, can be prepared according to Synthesis Scheme 6. Intermediates 6 can be treated with trifluoromethanesulfonic anhydride in the presence of triethylamine in dichloromethane to afford intermediates 12. For the transformation of intermediates 12 to compounds of the subset IC9 Suzuki-reactions using conditions described in WO 20140009274 and WO 2013045407 can be applied.

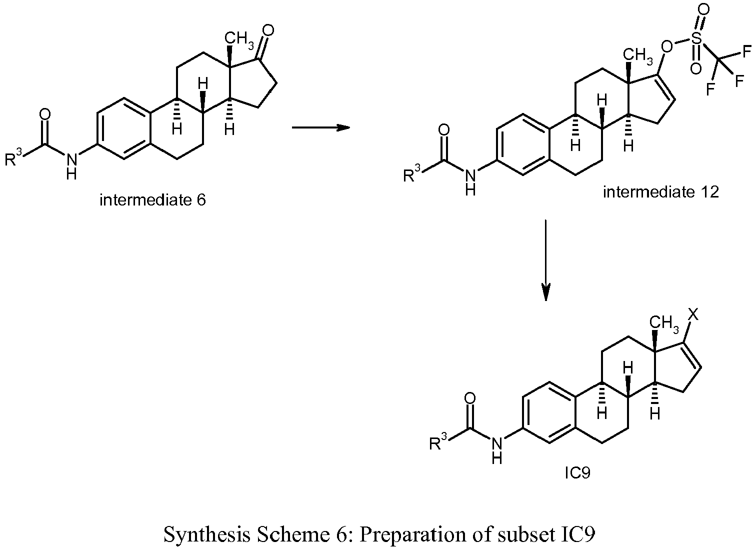

A subset IC10 of compounds of the present invention, in which A represents the group -SO(NH)-R⁷, can be obtained according to Synthesis Scheme 7. Reaction of intermediates 3-A with *meta-*chloroperoxybenzoic acid (mCPBA) in dichloromethane leads to the formation of intermediates 13. Intermediates 14 can be obtained using iodosobenzene diacetate, 2,2,2-trifluoroacetamide, rhodium(II)acetate dimer and magnesium oxide in dichloromethane. Intermediates 15 can be prepared using trifluoromethanesulfonic anhydride in the presence of a tertiary amine such as triethylamine. The compounds of subset IC10 can be prepared from intermediates 15 by a Suzuki-reaction using an aqueous base. The use of 2M sodium bicarbonate solution and dioxane is preferred. Dichlorobis(triphenylphosphine)palladium(II) is preferred as a catalyst.

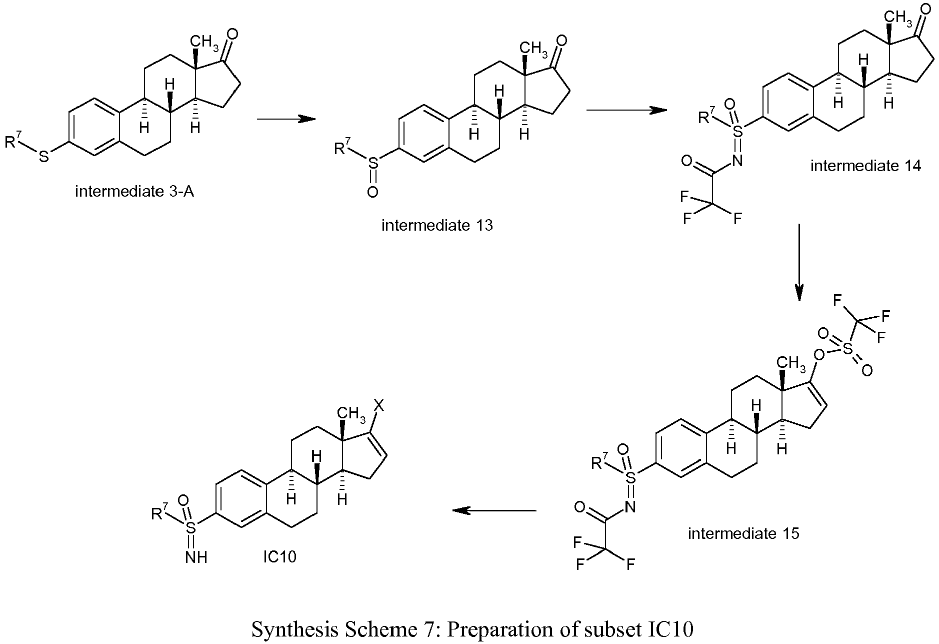

### LC-MS Methods:

### Method A:

MS instrument type: Waters ZMD; HPLC instrument type: Waters 1525; column: Phenomenex Luna 3µ C18(2) 30 mm x 4.6 mm; mobile phase A: water 0.1% formic acid, mobile phase B: acetonitrile 0.1% formic acid; gradient: 0.0 min 95% A -> 0.5 min 95% A -> 4.5 min 5% A -> 5.5 min 5% A; flow rate: 2 ml/min; UV detection: DAD.

### Method B:

MS instrument type: Waters Micromass ZQ2000; HPLC instrument type: Waters Acquity UPLC system; column: Acquity UPLC BEH C18 1.7micron 100 mm x 2.1 mm; mobile phase A: water 0.1% formic acid, mobile phase B: acetonitrile 0.1% formic acid; gradient: 0.0 min 95% A -> 0.4 min 95% A -> 6.0 min 5% A -> 6.8 min 5% A; flow rate: 0.4 ml/min; UV detection: PDA.

### Method C:

MS instrument type: Waters ZQ; HPLC instrument type: HP1100 series; column: Phenomenex Luna 3µm C18(2) 30 mm x 4.6 mm; mobile phase A: water 0.1% formic acid, mobile phase B: acetonitrile 0.1% formic acid; gradient: 0.0 min 95% A -> 0.5 min 95% A -> 4.5 min 5% A -> 5.5 min 5% A; flow rate: 2 ml/min; UV detection: PDA.

### Method D:

MS instrument type: Waters ACQUITY SQD; HPLC instrument type: Waters Acquity UPLC HSS T3 1.8 µ 50 x 1 mm; mobile phase A: 1 l of water + 0.25 ml of 99% strength formic acid, mobile phase B: acetonitrile 0.1% formic acid; gradient: 0.0 min 90% A → 1.2 min 5% A → 2.0 min 5% A oven: 50°C; flow rate: 0.40 ml/min; UV detection: PDA.

### Method E:

MS instrument type: Agilent 1260 infinity purifications system. Agilent 6100 series single Quadrupole LC/MS; column: XSELECT CSH Prep C18 5µm OBD, 30X150mm; mobile phase A: 0.1% aqueous formic acid, mobile phase B: 0.1% formic acid in acetonitrile; gradient: 10%-95%, 22min, centred around a specific focused gradient; flow rate: 60ml/min. Sample: Injection of a 20-60mg/ml solution in DMSO (+ optional formic acid and water)

### Method F:

Instrument: Waters Acquity UPLC-MS SQD; column Acquity UPLC BEH C18 1.7 50x2.1mm; eluent A: water + 0.1% Vol. formic acid (99%), eluent B: acetonitril; gradient: 0-1.6 min 1-99% B, 1.6-2.0 min 99% B; flow 0.8 ml/min; temperature: 60 °C; injection: 2 µl; DAD scan: 210-400 nm

### Method G:

Instrument: Waters Autopurificationsystem SQD; column: Waters XBrigde C18 5µ 100x30mm; eluent A: water + 0.1% Vol. formic acid (99%), eluent B: acetonitrile; narrow gradients within: 0-8.0 min 1-100% B, then 8.0-10.0 min 100% B; flow 50.0 ml/min; temperature: Rt; injection: 2500 µl; DAD scan: 210-400 nm

### Intermediate 1-A

### tert-butyl N-methyl-N-{[17-oxoestra-1(10),2,4-trien-3-yl]sulfonyl}-beta-alaninate

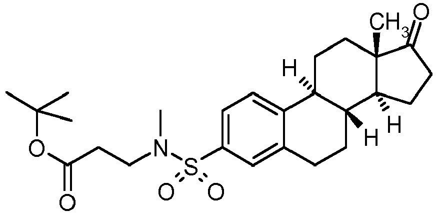

To a solution of 1.88 g (5.32 mmol) of 17-oxoestra-1(10),2,4-triene-3-sulfonyl chloride (Steroids, 1993, 58, 3, 106-111) in 17 ml of N,N-dimethylformamide under argon at 0°C 1.63 ml (11.7 mmol) of triethylamine and 0.933 g (5.86 mmol) of *tert*-butyl N-methyl-beta-alaninate (CAS No.: 143707-72-6) were added. The mixture was then allowed to warm to RT and stirred for 45 minutes. Water was added to the reaction mixture and partitioned into ethyl acetate. The organic phase was further washed with brine, dried over sodium sulfate, filtered and concentrated *in vacuo.* The residue was purified by chromatography using a prepacked silica gel cartridge (mobile phase: cyclohexane/ethyl acetate, gradient 0% to 40%). The yield was 1.9 g (75% of theory) of the title compound.
LC-MS (method D): Rₜ = 4.28 min; m/z = 476.1 (M+H)⁺
¹H-NMR (300 MHz, CDCl₃): δ [ppm] = 0.93 (s, 3H), 1.42-1.47 (m, 9H), 1.53 (s, 6H), 2.02 - 2.57 (m, 9H), 2.77 (s, 3H), 2.94 - 3.02 (m, 2H), 3.29 (dd, 2H), 7.42 (d, 1H), 7.50 - 7.57 (m, 2H).

### Intermediate 2-A

### tert-butyl N-methyl-N-{[17-{[(trifluoromethyl)sulfonyl]oxy}estra-1(10),2,4,16-tetraen-3-yl]sulfonyl}-beta-alaninate

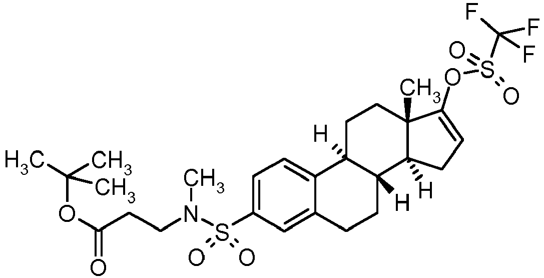

To a solution of 1.69 g (3.55 mmol) of tert-butyl N-methyl-N-{[17-oxoestra-1(10),2,4-trien-3-yl]sulfonyl}-beta-alaninate (Intermediate 1-A) in 10 ml of tetrahydrofuran under argon at -78°C 4.26 ml (4.26 mmol) of lithium bis(trimethylsilyl)amide 1M in toluene was added. The reaction mixture was allowed to warm to -30°C and maintained at -30°C for 25 minutes and then cooled to -78°C followed by the addition of a solution of 1.46 g (4.08 mmol) of 1,1,1-trifluoro-N-phenyl-N-[(trifluoromethyl)sulfonyl]methanesulfonamide (CAS No.: 37595-74-7) in 3 ml tetrahydrofuran. The reaction mixture was allowed to warm to -10°C over 25 minutes and maintained at -10°C for 45 minutes. The reaction mixture was diluted with ethyl acetate and quenched by addition of a saturated sodium hydrogen carbonate solution, stirred for 5 minutes. The phases were separated, the organic phase was further washed with brine, dried over sodium sulfate, filtered and concentrated *in vacuo.* The residue was purified by chromatography using a prepacked silica gel cartridge (mobile phase: cyclohexane/ethyl acetate, gradient 0% to 15%). The yield was 1.19 g (57% of theory) of the title compound.
LC-MS (method A): Rₜ = 5.01 min; m/z = 552.3 (M- *t*Bu +H)⁺
¹H-NMR (300 MHz, CDCl₃): δ [ppm] = 1.02 (s, 3H), 1.38 -1.56 (m, 10H), 1.57 - 1.89 (m, 4H), 1.90 - 2.04 (m, 2H), 2.11 (ddd, 1H), 2.31 - 2.46 (m, 3H), 2.54 (dd, 2H), 2.76 (s, 3H), 2.93 - 3.02 (m, 2H), 3.28 (dd, 2H), 5.63 (dd, 1H), 7.39 (d, 1H), 7.49 - 7.57 (m, 2H).

### Intermediate 3-A

### methyl 4-{[17-oxoestra-1(10),2,4-trien-3-yl]sulfanyl}butanoate

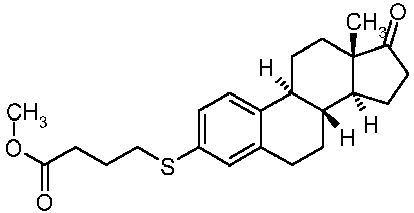

To a solution of 1.6 g (5.58 mmol) of 3-sulfanylestra-1(10),2,4-trien-17-one (CAS No.: 13965-03-2; Steroids, 1993, 58, 3, 106-111) in 6.0 ml of dimethyl sulfoxide under argon 810 mg (5.86 mmol) of potassium carbonate were added and 0.84 ml (6.70 mmol) of methyl 4-bromobutanoate (CAS No.: 4897-84-1). The mixture was stirred at 65°C for 2 hours. The reaction mixture was allowed to cool to RT, water was added followed by extraction into ethyl acetate. The organic phase was further washed with brine, dried over sodium sulfate, filtered and concentrated *in vacuo.* The residue was purified by chromatography using a prepacked silica gel cartridge (mobile phase: cyclohexane/ethyl acetate, gradient 0% to 40%). The yield was 1.67 g (77% of theory) of the title compound.
LC-MS (method D): Rₜ = 4.32 min; m/z = 387.1 (M+H)⁺
¹H NMR (300 MHz, CDCl₃): δ [ppm] = 0.91 (s, 3H), 1.35 - 1.72 (m, 7H), 1.91 - 2.19 (m, 6H), 2.43 - 2.51 (m, 4H), 2.85 - 2.97 (m, 4H), 3.67 (s, 3H), 7.08 - 7.24 (m, 3H).

### Intermediate 3-B

### 3-(methylsulfanyl)estra-1(10),2,4-trien-17-one

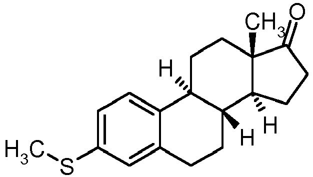

To a solution of 180 mg (0.62 mmol) of 3-sulfanylestra-1(10),2,4-trien-17-one (CAS No.: 13965-03-2; Steroids, 1993, 58, 3, 106-111) in 700 µl of N,N-dimethylformamide under argon 87 mg (0.628 mmol) of potassium carbonate and 43 µl (10.2 mmol) of iodomethane (CAS No.: 74-88-4) were added. The reaction mixture was stirred for 18 hours at RT and then partitioned between water and ethyl acetate. The organic phase was further washed with brine, dried over sodium sulfate, filtered and concentrated *in vacuo.* The residue was purified by chromatography using a prepacked silica gel cartridge (mobile phase: cyclohexane/ethyl acetate, gradient 0% to 40%). The yield was 161 mg (83% of theory) of the title compound.
¹H NMR (300 MHz, CDCl₃): δ [ppm] = 0.91 (s, 3H), 1.34 - 1.74 (m, 5H), 1.92 - 2.33 (m, 6H), 2.35 - 2.44 (m, 1H), 2.46 (s, 3H), 2.89 (dd, 2H), 7.01 - 7.10 (m, 2H), 7.20 (s, 1H), 7.26 (s, 1H).

### Intermediate 4-A

### methyl 4-{[17-oxoestra-1(10),2,4-trien-3-yl]sulfonyl}butanoate

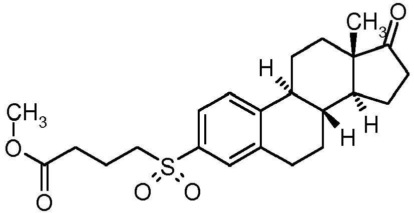

To a solution of 1.67 g (4.32 mmol) of methyl 4-{[17-oxoestra-1(10),2,4-trien-3-yl]sulfanyl}butanoate (Intermediate 3-A) in 20 ml of dichloromethane 2.32 g (10.3 mmol) of *meta*-chloroperoxybenzoic acid, 77% in four portions over 15 minutes was added. The mixture was stirred at RT for 3 hours. It was diluted with 100 ml of dichloromethane, 10% sodium sulfite aqueous solution was added. The mixture was stirred for 10 minutes and extracted. The organic phase was washed with a saturated sodium hydrogen carbonate solution and brine, dried over sodium sulfate, filtered and concentrated *in vacuo.* The residue was purified by chromatography using a prepacked silica gel cartridge (mobile phase: cyclohexane/ethyl acetate, gradient 0% to 40%). The yield was 1.64 g (90% of theory) of the title compound.
LC-MS (method A): Rₜ = 3.05 min; m/z = 441.1 (M+Na)⁺
¹H NMR (300 MHz, CDCl₃): δ [ppm] = 0.93 (s, 3H), 1.42 - 1.76 (m, 6H), 1.98 - 2.21 (m, 6H), 2.30 - 2.41 (m, 1H), 2.44 - 2.53 (m, 4H), 2.94 - 3.03 (m, 2H), 3.12 - 3.19 (m, 2H), 3.66 (s, 3H), 7.47 (d, 1H), 7.61 - 7.68 (m, 2H).

### Intermediate 4-B

### 3-(methylsulfonyl)estra-1(10),2,4-trien-17-one

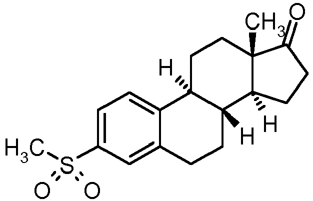

Analogously to the preparation of Intermediate 4-A, 100 mg (0.33 mmol) of 3-(methylsulfanyl)estra-1(10),2,4-trien-17-one (Intermediate 3-B) were reacted with 171 mg (0.76 mmol) of *meta-*chloroperoxybenzoic acid, 77%. The crude residue was purified by chromatography using a prepacked silica gel cartridge (mobile phase: cyclohexane/ethyl acetate, gradient 0% to 40%). The yield was 72 mg (64% of theory) of the title compound.
LC-MS (method C): Rₜ = 3.11 min; m/z = 333.1 (M+H)⁺
¹H NMR (300 MHz, CDCl₃): δ [ppm] = 0.93 (s, 3H), 1.40 - 1.75 (m, 6H), 1.96 - 2.24 (m, 4H), 2.30 - 2.40 (m, 1H), 2.42 - 2.59 (m, 2H), 2.95 - 3.03 (m, 2H), 3.04 (s, 3H), 7.48 (d, 1H), 7.66 - 7.72 (m, 2H).

### Intermediate 5-A

### methyl 4-{[17-{[(trifluoromethyl)sulfonyl]oxy}estra-1(10),2,4,16-tetraen-3-yl]sulfonyl}butanoate

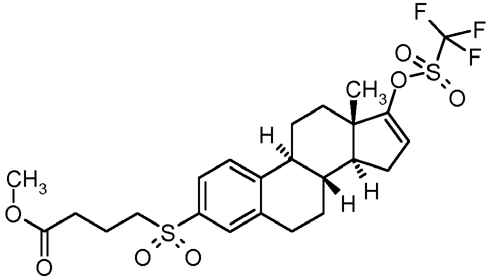

To a solution of 1.53 g (3.65 mmol) of methyl 4-{[17-oxoestra-1(10),2,4-trien-3-yl]sulfonyl}butanoate (Intermediate 4-A) in 30 ml dichloromethane 990 µl (5.84 mmol) of triflic anhydride were added at 0°C and then 764 µl (5.483 mmol) of triethylamine in 5 ml dichlormethane were added dropwise. The mixture was stirred for 16 hours at RT and cooled to 0°C. 492 µl (2.92 mmol) of triflic anhydride and 381 µl (2.74 mmol) of triethylamine were added and the mixture was stirred for another 4 hours at RT. Water was added and the mixture was extracted with dichloromethane. The organic phase was washed with saturated sodium hydrogencarbonate solution and brine, dried over sodium sulphate, filtered and concentrated. The yield was 2.27 g (99% of theory) of the title compound.
LC-MS (method D): Rₜ = 4.60 min; m/z = 551.1 (M+H)⁺
¹H NMR (300 MHz, CDCl₃): δ [ppm] = 1.02 (s, 3H), 1.33 - 1.42 (m, 2H), 1.43 - 2.23 (m, 9H), 2.41 - 2.51 (m, 1H), 2.94 - 3.03 (m, 2H), 3.12 - 3.20 (m, 3H), 3.29 - 3.52 (m, 2H), 3.66 (s, 3H), 5.62 - 5.65 (m, 1H), 7.44 (d, 1H), 7.60 - 7.68 (m, 2H).

### Intermediate 5-B

### 3-(methylsulfonyl)estra-1(10),2,4,16-tetraen-17-yl trifluoromethanesulfonate

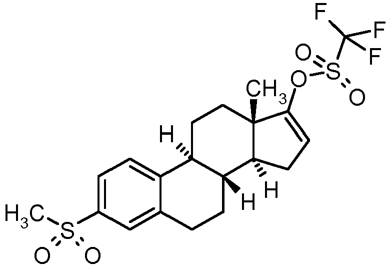

Analogously to the preparation of Intermediate 5-A, 70 mg (0.21 mmol) of 3-(methylsulfonyl)estra-1(10),2,4-trien-17-one (Intermediate 4-B) were treated with 57 µl (0.34 mmol) of triflic anhydride and 26 µl (0.31 mmol) of triethylamine. After 16 hours, 28 µl (0.16 mmol) and 13 µl (0.09 mmol) of triethylamine were added followed by continuous stirring for another 4 hours at RT. The yield was 105 mg (95% of theory) of the title compound.
¹H NMR (300 MHz, CDCl₃): δ [ppm] = 1.02 (s, 3H), 1.31 - 2.05 (m, 8H), 2.24 - 2.49 (m, 1H), 3.03 (s, 6H), 3.30 - 3.52 (m, 1H), 5.64 (dd, 1H), 7.45 (d, 1H), 7.64 - 7.73 (m, 2H).

### Intermediate 6-A

### tert-butyl 4-oxo-4-{[17-oxoestra-1(10),2,4-trien-3-yl]amino}butanoate

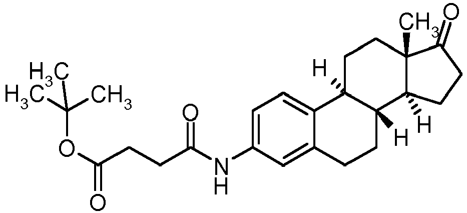

To a solution of 2.5 g (9.29 mmol) of 3-aminoestra-1(10),2,4-trien-17-one (CAS No.: 18119-98-7) in 30 ml of N,N-dimethylformamide 1.63 g (9.38 mmol) of 4-tert-butoxy-4-oxobutanoic acid (CAS No.: 15026-17-2), 3.88 g (10.2 mmol) of 1-[bis-(dimethylamino)methylene]-1H-1,2,3-triazolo[4,5-b]pyridinium 3-oxid hexafluorophosphate and 1.12 ml (10.2 mmol) of 4-methylmorpholine were added. The mixture was stirred at RT for 2 hours. It was then concentrated under reduced pressure and the residue was partitioned between water and ethyl acetate. The organic phase was further washed with a saturated sodium hydrogen carbonate solution, brine, dried over sodium sulfate, filtered and concentrated *in vacuo.* The residue was purified by chromatography using a prepacked silica gel cartridge (dichloromethane/ethyl acetate, gradient 0% to 15%). The yield was 3.58 g (90% of theory) of the title compound.
LC-MS (method A): Rₜ = 3.88 min; m/z = 426.3 (M+H)⁺
¹H-NMR (400 MHz, CDCl₃): δ [ppm] = 0.91 (s, 3H), 1.38 - 1.52 (m, 15H), 1.52 - 1.68 (m, 3H), 1.91 - 2.30 (m, 2H), 2.36 - 2.70 (m, 6H), 2.86 - 2.94 (m, 2H), 7.17 - 7.25 (m, 2H), 7.33 (s, 1H), 7.55 (s, 1H)

### Intermediate 6-B

### methyl 4-oxo-4-{[17-oxoestra-1(10),2,4-trien-3-yl]amino}butanoate

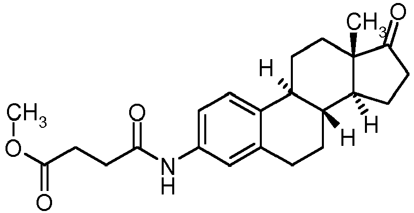

Analogously to the preparation of Intermediate 6-A, 200 mg (0.74 mmol) of 3-aminoestra-1(10),2,4-trien-17-one were reacted with 99 mg (0.75 mmol) of 4-methoxy-4-oxobutanoic acid (CAS No.: 3878-55-5). The residue was purified by chromatography using a prepacked silica gel cartridge (mobile phase: cyclohexane/ethyl acetate, gradient 0% to 50%). The yield was 240 mg (83% of theory) of the title compound.
LC-MS (method D): Rₜ = 3.39 min; m/z = 384.0 (M+H)⁺
¹H NMR (300 MHz, CDCl₃): δ [ppm] = 0.90 (s, 3H), 1.34 - 1.71 (m, 5H), 1.91 - 2.30 (m, 6H), 2.35 - 2.57 (m, 2H), 2.60 - 2.79 (m, 4H), 2.89 (dd, 2H), 3.71 (s, 3H), 7.20 (d, 2H), 7.37 (d, 2H).

### Intermediate 7-A

### tert-butyl 4-{[(17E)-17-hydrazinylideneestra-1(10),2,4-trien-3-yl]amino}-4-oxobutanoate

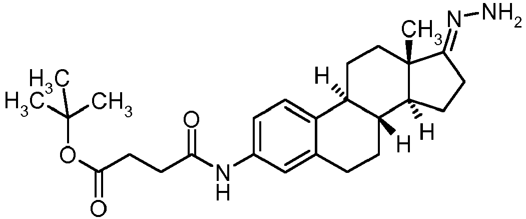

To a solution of 400 mg (0.94 mmol) of *tert*-butyl 4-oxo-4-{[17-oxoestra-1(10),2,4-trien-3-yl]amino}butanoate (Intermediate 6-A) in 3.4 ml of ethanol 0.65 ml (4.7 mmol) of triethylamine and 1.6 ml (32.9 mmol) of hydrazine monohydrate were added. The mixture was stirred under reflux for 3 hours. It was then allowed to cool to room temperature and concentrated under reduced pressure and the residue was partitioned between water and dichloromethane. The organic phase was further washed with water, brine, dried over sodium sulfate, filtered and concentrated *in vacuo.* The yield was 404 mg (97% of theory) of the title compound.
LC-MS (method A): Rₜ = 2.82 min; m/z = 440.5 (M+H)⁺
¹H NMR (300 MHz, CDCl₃): δ [ppm] = 0.88 (s, 3H), 1.22 - 1.68 (m, 14H), 1.91 - 2.08 (m, 3H), 2.18 - 2.42 (m, 4H), 2.53 - 2.70 (m, 4H), 2.83 - 2.91 (m, 2H), 4.78 (s, 2H), 5.30 (s, 1H), 7.13 - 7.25 (m, 2H), 7.32 (s, 1H), 7.56 - 7.63 (m, 1H)

### Intermediate 8-A

### tert-butyl 4-{[17-iodoestra-1(10),2,4,16-tetraen-3-yl]amino}-4-oxobutanoate

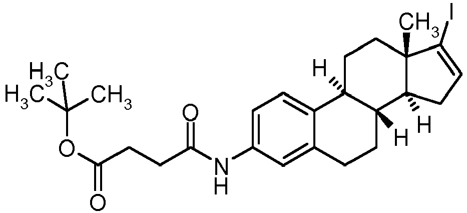

To a solution of 2.44 g (9.61 mmol) of iodine in 38 ml of dry tetrahydrofuran under argon at 0°C 3.01 ml (24.0 mmol) of 1,1,3,3-tetramethylguanidine were added. Then a solution of 2.11 g (4.80 mmol) of *tert*-butyl 4-{[(17E)-17-hydrazinylideneestra-1(10),2,4-trien-3-yl]amino}-4-oxobutanoate (Intermediate 7-A) in 56 ml of tetrahydrofuran and 38 ml of diethyl ether was added dropwise over 2 hours. The reaction mixture was then diluted with ethyl acetate and washed successively with 10% sodium metabisulfite solution, water, brine, dried over sodium sulfate, filtered and concentrated *in vacuo.* The residue was purified by chromatography using a prepacked silica gel cartridge (mobile phase: cyclohexane/ethyl acetate, gradient 0% to 15%). The yield was 1.96 g (70 % of theory) of the title compound.
LC-MS (method D): Rₜ = 5.09 min; m/z = 536.2 (M+H)⁺
¹H-NMR (400 MHz, CDCl₃): δ [ppm] = 0.75 (s, 3H), 1.35 - 2.11 (m, 8H), 2.20 - 2.29 (m, 3H), 2.34 - 2.43 (m, 2H), 2.54 - 2.68 (m, 6H), 2.79 (s, 1H), 2.84 - 2.91 (m, 3H), 3.07 (s, 1H), 3.71 - 3.75 (m, 2H), 6.14 - 6.17 (m, 1H), 7.19 (s, 2H), 7.30 (s, 1H), 7.50 - 7.55 (m, 2H)

### Intermediate 9-A

### tert-butyl [17-(5-fluoropyridin-3-yl)estra-1(10),2,4,16-tetraen-3-yl]carbamate

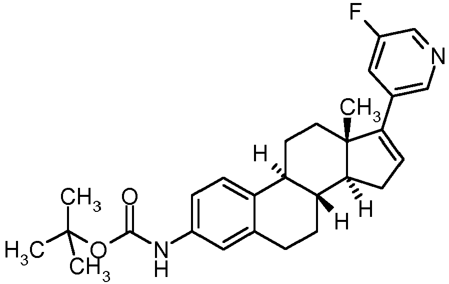

To a solution of 1 g (2.64 mmol) of 17-(5-fluoropyridin-3-yl)estra-1(10),2,4,16-tetraene-3-carboxylic acid (WO 201304540) in 3.0 ml of tert-butanol under argon 0.40 ml (2.91 mmol) of triethylamine and 802 mg (2.91 mmol) of diphenyl phosphoroazidate (CAS No.: 26386-88-9) were added. The reaction mixture was stirred for 18 hours at 100°C under reflux. The reaction mixture was allowed to cool to RT and partitioned between water and ethyl acetate. The organic phase was further washed with brine, dried over sodium sulfate, filtered and concentrated *in vacuo.* The residue was purified by chromatography using a prepacked silica gel cartridge (mobile phase: cyclohexane/ethyl acetate, gradient 0% to 20%). The yield was 1 g (80% of theory) of the title compound.
LC-MS (method A): Rₜ = 4.94 min, m/z = 449.4 (M+H)⁺
¹H-NMR (400 MHz, CDCl₃): δ [ppm] = 1.04 (s, 3H), 1.40 - 1.46 (m, 1H), 1.51 (s, 9H), 1.60 - 1.74 (m, 3H), 1.81 (dt, 1H), 1.91 - 2.02 (m, 1H), 2.10 - 2.22 (m, 2H), 2.28 - 2.43 (m, 3H), 2.88 - 2.96 (m, 2H), 6.09 (dd, 1H), 6.38 (s, 1H), 7.04 (dd, 1H), 7.20 (d, 2H), 7.37 - 7.43 (m, 1H), 8.34 (d, 1H), 8.48 (dd, 1H).

### Intermediate 10-A

### 17-(5-fluoropyridin-3-yl)estra-1(10),2,4,16-tetraen-3-amine

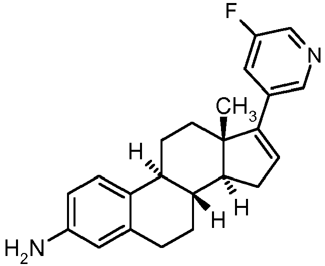

To a solution of 897 mg (2.0 mmol) of tert-butyl [17-(5-fluoropyridin-3-yl)estra-1(10),2,4,16-tetraen-3-yl]carbamate (Intermediate 9-A) in 32 ml of dichloromethane 7.7 ml (100 mmol) of trifluoroacetic acid were added. The mixture was stirred at RT for 1 hour. It was then concentrated under reduced pressure. The crude residue was purified with an Isolute® SCX-2 cartridge (Biotage, mobile phase: dichloromethane/methanol 9:1, followed by 2N ammonia in methanol). The yield was 644 mg (88% of theory) of the title compound.
LC-MS (method A): Rₜ =2.92 min; m/z = 349.3 (M+H)⁺
¹H-NMR (400 MHz, DMSO-d₆): δ [ppm] = 1.02 (s, 3H), 1.32 - 1.59 (m, 4H), 1.60 - 1.91 (m, 2H), 2.06 - 2.22 (m, 3H), 2.31 (ddd, 2H), 2.64 - 2.78 (m, 2H), 4.72 (s, 2H), 6.27 - 6.38 (m, 3H), 6.91 (d, 1H), 7.67 - 7.73 (m, 1H), 8.46 (d, 1H), 8.52 (t, 1H).

### Intermediate 11-A

### 17-(5-fluoropyridin-3-yl)-N-methylestra-1(10),2,4,16-tetraen-3-amine

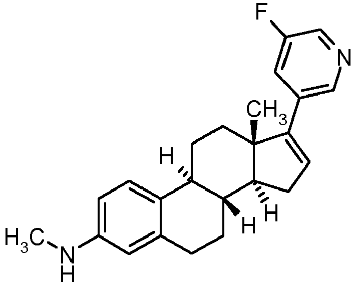

To a solution of 177 mg (0.48 mmol) of 17-(5-fluoropyridin-3-yl)estra-1(10),2,4,16-tetraen-3-amine (Intermediate 10-A) in 10.0 ml of methanol under argon 72.4 mg (2.41 mmol) of paraformaldehyde followed by 130 mg (2.41 mmol) of sodium methoxide were added. The reaction mixture was stirred at 65°C under reflux for 1 hour and then allowed to cool to RT. 91.3 mg (2.41 mmol) of sodium borohydride were added and the reaction mixture was stirred under reflux for 1.5 hours and allowed to cool to RT. Water was carefully added and stirring continued for 2 minutes. The reaction mixture was partitioned between water and ethyl acetate. The organic phase was washed with brine, dried over sodium sulfate, filtered and concentrated *in vacuo.* The residue was purified by chromatography using a prepacked silica gel cartridge (mobile phase: dichloromethane/ethyl acetate, gradient 0% to 15%). The yield was 128 mg (70% of theory) of the title compound.
LC-MS (method A): Rₜ = 3.02 min, m/z = 363.3 (M+H)⁺
¹H NMR (300 MHz, CDCl₃): δ [ppm] = 1.04 (s, 3H), 1.39 - 1.53 (m, 1H), 1.59 - 1.74 (m, 3H), 1.76 - 1.88 (m, 1H), 1.90 - 2.00 (m, 1H), 2.09 - 2.21 (m, 2H), 2.26 - 2.43 (m, 3H), 2.82 (s, 3H), 2.84 - 2.94 (m, 2H), 3.55 (s, 1H), 6.07 - 6.10 (m, 1H), 6.37 - 6.50 (m, 2H), 7.12 (d, 1H), 7.37 - 7.43 (m, 1H), 8.34 (d, 1H), 8.46 - 8.49 (m, 1H).

### Intermediate 12-A

### methyl 4-oxo-4- { [17-{ [(trifluoromethyl)sulfonyl] oxy}estra-1(10),2,4,16-tetraen-3-yl]amino} butanoate

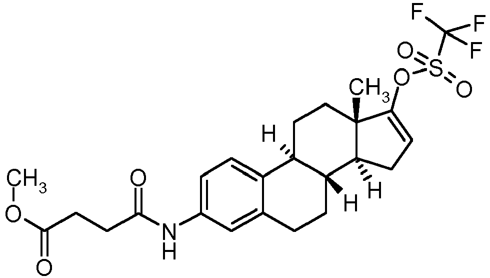

To a solution of 197 mg (0.51 mmol) of methyl 4-oxo-4-{[17-oxoestra-1(10),2,4-trien-3-yl]amino}butanoate (Intermediate 6-B) in 4.5 ml of dichloromethane under argon at 0°C 139 µl (0.82 mmol) of triflic anhydride and then dropwise 107 µl (0.77 mmol) of triethylamine in solution in dichloromethane (0.5 ml) were added. The mixture was allowed to warm to RT for 5 hours. The reaction mixture was quenched by addition of water, then diluted with dichloromethane and separated. The organic phase was further washed with a saturated sodium hydrogen carbonate solution, brine, dried over sodium sulfate, filtered and concentrated *in vacuo.* Trituration with cyclohexane gave 84 mg (27% of theory) of the title compound.
LC-MS (method C): Rₜ = 4.12 min, m/z = 515.9
¹H NMR (300 MHz, CDCl₃): δ [ppm] = 1.00 (s, 3H), 1.33 - 1.99 (m, 7H), 2.02 - 2.17 (m, 1H), 2.26 - 2.44 (m, 3H), 2.60 - 2.80 (m, 4H), 2.80 - 2.94 (m, 2H), 3.62 - 3.70 (m, 1H), 3.71 (s, 2H), 5.59 - 5.64 (m, 1H), 7.19 (s, 2H), 7.33 (s, 1H), 7.41 (s, 1H).

### Intermediate 13-A

### 3-[(S)-methylsulfinyl]estra-1(10),2,4-trien-17-one

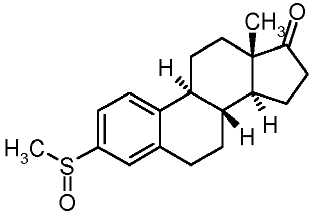

Analogously to the preparation of Intermediate 4-A, 97.0 mg (0.32 mmol) of 3-(methylsulfanyl)estra-1(10),2,4-trien-17-one (Intermediate 3-B) were reacted with 72.3 mg (0.32 mmol) of *meta-*chloroperoxybenzoic acid, 77%. The crude residue was purified by chromatography using a prepacked silica gel cartridge (mobile phase: cyclohexane/ethyl acetate, gradient 0% to 40%). The yield was 85 mg (82% of theory) of the title compound.
LC-MS (method A): Rₜ = 2.94 min; m/z = 317.2 (M+H)⁺
¹H NMR (300 MHz, CDCl₃): δ [ppm] = 0.93 (s, 3H), 1.42 - 1.72 (m, 6H), 1.95 - 2.24 (m, 4H), 2.28 - 2.59 (m, 3H), 2.71 (s, 3H), 2.94 - 3.04 (m, 2H), 7.34 - 7.47 (m, 3H).

### Intermediate 14-A

### 2,2,2-trifluoro-N-{methyl(oxido)[17-oxoestra-1(10),2,4-trien-3-yl]-λ⁶-sulfanylidene}acetamide

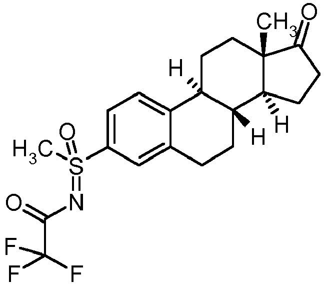

To a solution of 64 mg (0.20 mmol) of 3-(methylsulfinyl)estra-1(10),2,4-trien-17-one (Intermediate 13-A) in 3.5 ml of dichloromethane under argon at RT 54 mg (0.47 mmol) of 2,2,2-trifluoroacetamide (CAS No.: 354-38-1), 114 mg (0.35 mmol) of iodosobenzene diacetate (CAS No.: 3240-34-4), 10.7 mg (0.02 mmol) of rhodium(II)acetate dimer (CAS No.: 15956-28-2) and 38.3 mg (0.95 mmol) of magnesium oxide (CAS No.: 1309-48-4) were added. The reaction mixture was stirred for 5 hours and then filtered, rinsed with dichloromethane and concentrated *in vacuo.* The residue was purified using a prepacked silica gel cartridge (mobile phase: cyclohexane/ethyl acetate, gradient 0% to 40%). The yield was 81 mg (93% of theory) of the title compound.
LC-MS (method C): Rₜ = 3.65 min; m/z = 428.0 (M+H)⁺
¹H-NMR (300 MHz, CDCl₃): δ [ppm] = 0.93 (s, 3H), 1.45 - 1.71 (m, 6H), 2.03 - 2.13 (m, 4H), 2.31 - 2.60 (m, 3H), 2.97 - 3.06 (m, 2H), 3.42 (s, 3H), 7.52 - 7.58 (m, 1H), 7.68 - 7.75 (m, 2H).

### Intermediate 15-A

### 3-[S-methyl-N-(trifluoroacetyl)sulfonimidoyl]estra-1(10),2,4,16-tetraen-17-yl trifluoromethanesulfonate

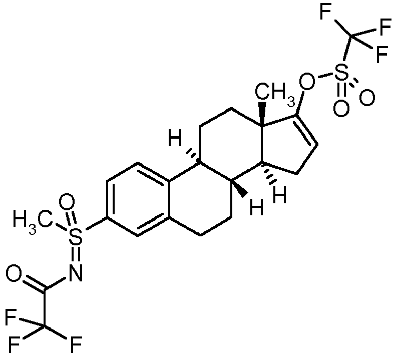

Analogously to the preparation of Intermediate 5-A, 81 mg (0.19 mmol) of 2,2,2-trifluoro-N-{methyl(oxido)[17-oxoestra-1(10),2,4-trien-3-yl]-λ⁶-sulfanylidene}acetamide (Intermediate 14-A) were treated with 51 µl (0.30 mmol) of triflic anhydride and 40 µl (0.28 mmol) and 26 µl (0.31 mmol) of triethylamine. The yield was 106 mg (85% of theory) of the title compound.
LC-MS (method C): Rₜ = 4.48 min; m/z = 559.9 (M+H)⁺
¹H-NMR (300 MHz, CDCl₃): δ [ppm] = 1.02 (s, 3H), 1.33 - 1.89 (m, 6H), 1.90 - 2.22 (m, 3H), 2.31 - 2.49 (m, 2H), 2.97 - 3.07 (m, 2H), 3.35 - 3.51 (m, 4H), 5.62 - 5.66 (m, 1H), 7.49 - 7.58 (m, 1H), 7.67 - 7.75 (m, 2H).

### Examples type IC1

### Example 1

### tert-butyl N-{[17-(5-fluoropyridin-3-yl)estra-1(10),2,4,16-tetraen-3-yl]sulfonyl}-N-methyl-beta-alaninate

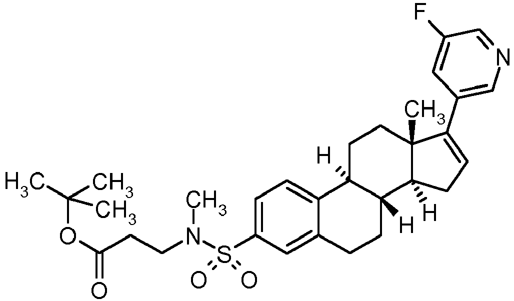

To a solution of 655 mg (1.07 mmol) of tert-butyl N-methyl-N-{[17-{[(trifluoromethyl)sulfonyl]oxy}estra-1(10),2,4,16-tetraen-3-yl]sulfonyl}-beta-alaninate (Intermediate 2-A) in 6.5 ml of dioxane 258 mg (1.83 mmol) of 5-fluoropyridine-3-boronic acid, 60.5 mg (0.08 mmol) of dichlorobis(triphenylphosphine)palladium(II) and 2.15 ml of 2M sodium carbonate solution were added. The reaction mixture was degassed and then stirred in a sealed tube for 2 hours at 90°C. The reaction mixture was allowed to cool to room temperature and partitioned between water and ethyl acetate. The organic phase was further washed with brine, dried over sodium sulfate, filtered and concentrated *in vacuo.* The residue was purified by chromatography using a prepacked silica gel cartridge (mobile phase: cyclohexane/ethyl acetate, gradient 0% to 35%). The yield was 525 mg (83% of theory) of the title compound.
LC-MS (method A): Rₜ = 4.89 min; m/z = 555.4 (M+H)⁺
¹H-NMR (300 MHz, CDCl₃): δ [ppm] = 1.07 (s, 3H), 1.45 (s, 9H), 1.48 - 1.61 (m, 1H), 1.67 - 1.86 (m, 4H), 1.96 - 2.10 (m, 1H), 2.04 - 2.05 (m, 2H), 2.41 (ddd, 3H), 2.54 (dd, 2H), 2.77 (s, 3H), 3.00 (dd, 2H), 3.29 (dd, 2H), 6.11 (dd, 1H), 7.37 - 7.45 (m, 2H), 7.50 - 7.57 (m, 2H), 8.36 (d, 1H), 8.48 (dd, 1H)

### Example 2

### N- {[17-(5-fluoropyridin-3-yl)estra-1(1 0),2,4,16-tetraen-3-yl]sulfonyl} -N-methyl-beta-alanine

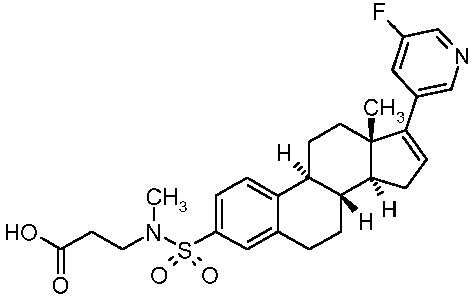

525 mg (0.94 mmol) of *tert*-butyl N-{[17-(5-fluoropyridin-3-yl)estra-1(10),2,4,16-tetraen-3-yl]sulfonyl}-N-methyl-beta-alaninate (Example 1) in 15.7 ml dichloromethane were treated with 3.64 ml (47.3 mmol) of trifluoroacetic acid. The mixture was stirred for 1 h at RT and concentrated under reduced pressure. The crude residue was purified by chromatography using a prepacked silica gel cartridge (mobile phase: dichloromethane/ethyl acetate: methanol: 30% triethylamine in water 7: 3, gradient 0% to 70%). The yield was 326 mg (68% of theory) of the title compound.
LC-MS (method B): Rₜ = 5.28 min, m/z = 499.2 (M+H)⁺
¹H-NMR (400 MHz, DMSO-d₆): δ [ppm] = 1.01 (s, 3H), 1.40 - 1.43 (m, 1H), 1.55 - 1.79 (m, 4H), 1.88 - 1.95 (m, 1H), 2.10 - 2.20 (m, 2H), 2.26 - 2.45 (m, 5H), 2.64 (s, 3H), 2.91 - 2.99 (m, 2H), 3.14 (dd, 2H), 6.26 - 6.28 (m, 1H), 7.43 - 7.53 (m, 3H), 7.66 - 7.71 (m, 1H), 8.43 - 8.51 (m, 2H), 11.2 - 13.4 (br. s, 1H)

### Example 3

### 17-(5-fluoropyridin-3-yl)-N-(3-hydroxypropyl)-N-methylestra-1(10),2,4,16-tetraene-3-sulfonamide

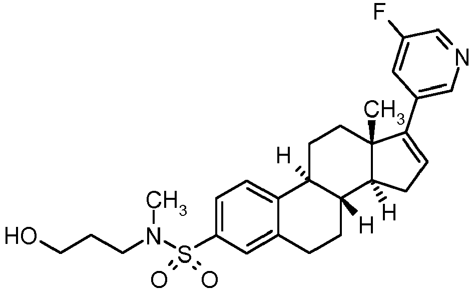

150 mg (0.30 mmol) of N-{[17-(5-fluoropyridin-3-yl)estra-1(10),2,4,16-tetraen-3-yl]sulfonyl}-N-methyl-beta-alanine (Example 2) in 1.2 ml tetrahydrofuran were treated at 0°C with 50 µl (0.36 mmol) of triethylamine, followed by dropwise addition of 47 µl (0.36 mmol) of 2-methylpropyl carbonochloridate (CAS No.: 543-27-1) . The reaction mixture was stirred at 0°C for 45 min and then allowed to warm to room temperature for 15 minutes. The reaction mixture was cooled at 0°C, filtered, rinsed with tetrahydrofuran (200 µl) and the filtrate was cooled at 0°C. 13.6 mg (0.36 mmol) of sodium borohydride was added to the reaction mixture and after 5 minutes 300 µl of methanol. The reaction mixture was stirred at 0°C for 45 minutes and then allowed to slowly warm to room temperature over 2 hours. Water was carefully added and stirring continued for 2 minutes. The reaction mixture was partitioned between water and ethyl acetate. The organic phase was washed with brine, dried over sodium sulfate, filtered and concentrated in vacuo. The residue was purified by chromatography on silica gel (mobile phase: dichloromethane/methanol, gradient 0% to 3%). The yield was 63 mg (43% of theory) of the title compound.
LC-MS (method B): Rₜ = 5.33 min; m/z = 485.1 (M+H)⁺
¹H-NMR (400 MHz, DMSO-d₆): δ [ppm] = 1.01 (s, 3H), 1.56 - 1.77 (m, 6H), 1.88 - 1.97 (m, 1H), 2.09 - 2.20 (m, 2H), 2.26 - 2.45 (m, 4H), 2.62 (s, 3H), 2.95 (dd, 4H), 3.39 (dd, 2H), 4.43 (dd, 1H), 6.26 - 6.28 (m, 1H), 7.42 - 7.52 (m, 3H), 7.66 - 7.71 (m, 1H), 8.43 (d, 1H), 8.51 (t, 1H).

### Example 4

### tert-butyl N-methyl-N- {[17-(pyrimidin-5-yl)estra-1 (1 0),2,4,16-tetraen-3-yl]sulfonyl}-beta-alaninate

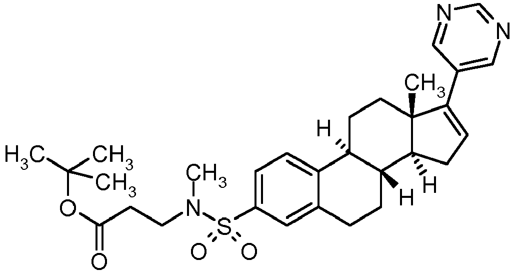

Analogously to the preparation of Example 1, 268 mg (0.44 mmol) of *tert-butyl* N-methyl-N-{[17-{[(trifluoromethyl)sulfonyl]oxy}estra-1 (1 0),2,4,16-tetraen-3-yl] sulfonyl}- beta-alaninate (Intermediate 2-A) were reacted with 93 mg (1.83 mmol) of pyrimidin-5-ylboronic acid and 24.7 mg (0.03 mmol) of dichlorobis(triphenylphosphine)palladium(II). The residue was purified by chromatography using a prepacked silica gel cartridge (mobile phase: cyclohexane/ethyl acetate, gradient 0% to 50%). The yield was 214 mg (86% of theory) of the title compound.
LC-MS (method A): Rₜ = 4.50 min; m/z = 538.4 (M+H)⁺
¹H-NMR (300 MHz, CDCl₃): δ [ppm] = 1.07 (s, 3H), 1.26 (dd, 2H), 1.45 (s, 1H), 1.5 - 1.9 (m, 7H), 2.05 (s, 3H), 2.15 - 2.27 (m, 2H), 2.38 - 2.58 (m, 6H), 2.77 (s, 3H), 3.00 (dd, 2H), 3.29 (dd, 2H), 4.12 (q, 1H), 6.15 (dd, 1H), 7.42 (d, 1H), 7.51 - 7.57 (m, 2H), 8.76 (s, 2H), 9.10 (s, 1H).

### Example 5

### N-methyl-N- {[17-(pyrimidin-5-yl)estra-1(10),2,4,16-tetraen-3-yl]sulfonyl}-beta-alanine

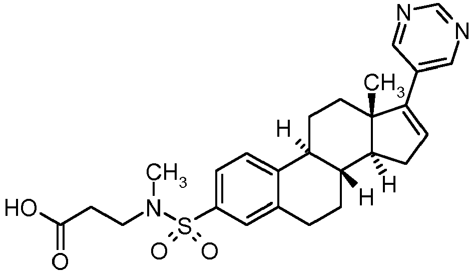

Analogously to the preparation of Example 2, 213 mg (0.39 mmol) of *tert-butyl* N-methyl-N-{[17-(pyrimidin-5-yl)estra-1(10),2,4,16-tetraen-3-yl]sulfonyl}-beta-alaninate were treated with 1.52 ml (19.8 mmol) of trifluoroacetic acid. The crude residue was purified by preparative HPLC (Method E) . The yield was 142 mg (74% of theory) of the title compound.
LC-MS (method B): Rₜ = 4.57 min; m/z = 482.1 (M+H)⁺
¹H-NMR (400 MHz, DMSO-d₆): δ [ppm] = 1.01 (s, 3H), 1.46 - 1.77 (m, 6H), 1.87 - 1.96 (m, 1H), 2.10 - 2.20 (m, 2H), 2.28 - 2.45 (m, 4H), 2.64 (s, 3H), 2.91 - 2.98 (m, 2H), 3.13 (dd, 2H), 6.31 (dd, 1H), 7.43 - 7.53 (m, 3H), 8.83 (s, 2H), 9.05 (s, 1H), 12.3 (br. s, 1H).

### Example 6

### tert-butyl N-methyl-N- {[17-(6-methylpyridazin-4-yl)estra-1(1 0),2,4,16-tetraen-3-yl]sulfonyl}-beta-alaninate

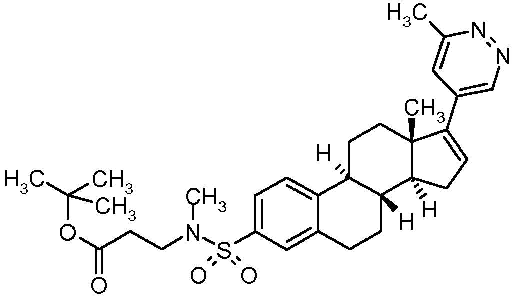

To a solution of 267 mg (0.44 mmol) of tert-butyl N-methyl-N-{[17-{[(trifluoromethyl)sulfonyl]oxy}estra-1(10),2,4,16-tetraen-3-yl]sulfonyl}-beta-alaninate (Intermediate 2-A) in 2.2 ml of toluene and 1.4 ml of ethanol 135 mg (0.61 mmol) of 3-methyl-5-(4,4,5,5-tetramethyl-[1,3,2]dioxaborolan-2-yl)-pyridazine, 37.2 mg (0.88 mmol) of lithium chloride, 24.7 mg (0.03 mmol) of dichlorobis(triphenylphosphine)palladium(II) and 549 µl of 2M sodium carbonate solution were added. The reaction mixture was degassed and then stirred in a sealed tube for 8 hours at 100°C. The reaction mixture was allowed to cool to room temperature and partitioned between water and ethyl acetate. The organic phase was further washed with brine, dried over sodium sulfate, filtered and concentrated *in vacuo.* The residue was purified by chromatography using a prepacked silica gel cartridge (mobile phase: cyclohexane/ethyl acetate, gradient 0% to 70%). The yield was 130 mg (51% of theory) of the title compound.
LC-MS (method A): Rₜ = 4.03 min; m/z = 552.3 (M+H)⁺
¹H-NMR (300 MHz, CDCl₃): δ [ppm] = 1.10 (s, 3H), 1.45 (s, 9H), 1.47 - 1.90 (m, 5H), 1.97 - 2.09 (m, 1H), 2.15 - 2.31 (m, 2H), 2.39 - 2.58 (m, 5H), 2.71 (s, 3H), 2.77 (s, 3H), 3.00 (dd, 2H), 3.29 (dd, 2H), 6.36 (dd, 1H), 7.25 (s, 1H), 7.42 (d, 1H), 7.51 - 7.58 (m, 2H), 9.08 (d, 1H).

### Example 7

### N-methyl-N- {[17-(6-methylpyridazin-4-yl)estra-1 (10),2,4,16-tetraen-3-yl]sulfonyl}-beta-alanine

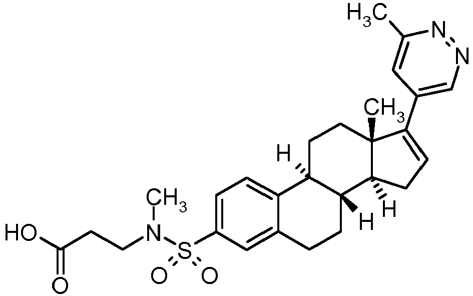

Analogously to the preparation of Example 2, 130 mg (0.23 mmol) of *tert*-butyl N-methyl-N-{[17-(6-methylpyridazin-4-yl)estra-1(10),2,4,16-tetraen-3-yl]sulfonyl}-beta-alaninate were treated with 908 µl (11.7 mmol) of trifluoroacetic acid. The crude residue was purified by preparative HPLC (Method E). The yield was 78 mg (66% of theory) of the title compound.
LC-MS (method B): Rₜ = 3.95 min; m/z = 496.1 (M+H)⁺
¹H-NMR (400 MHz, DMSO-d₆): δ [ppm] = 1.03 (s, 3H), 1.44 - 1.78 (m, 6H), 1.87 - 1.96 (m, 1H), 2.16 (dd, 1H), 2.26 - 2.46 (m, 5H), 2.59 (s, 3H), 2.64 (s, 3H), 2.91 - 2.98 (m, 2H), 3.13 (dd, 2H), 6.56 - 6.57 (m, 1H), 7.43 - 7.51 (m, 4H), 9.11 (d, 1H), 12.5 (br. s, 1H).

### Examples type IC2

### Example 8

### 17-(5-fluoropyridin-3-yl)-3-(methylsulfonyl)estra-1 (10),2,4,16-tetraene

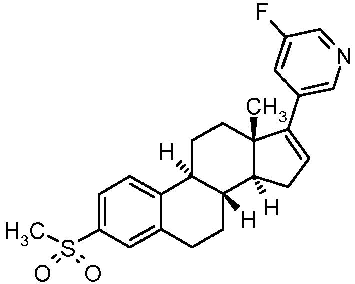

Analogously to the preparation of Example 1, 65 mg (0.14 mmol) of 3-(methylsulfonyl)estra-1(10),2,4,16-tetraen-17-yl trifluoromethanesulfonate (Intermediate 5-B) were treated with 21.7 mg (0.15 mmol) of 5-fluoropyridine-3-boronic acid (CAS No.: 872041-86-6) and 7.85 mg (0.01 mmol) of dichlorobis(triphenylphosphine)palladium(II) (CAS No.: 13965-03-2). The residue was purified using a prepacked silica gel cartridge (mobile phase: cyclohexane/ethyl acetate, gradient 0% to 35%). The yield was 53.4 mg (91% of theory) of the title compound.
LC-MS (method B): Rₜ = 5.43 min; m/z = 412.1 (M+H)⁺
¹H NMR (400 MHz, DMSO-d₆): δ [ppm] = 1.01 (s, 3H), 1.45 - 1.77 (m, 6H), 1.89 - 1.97 (m, 1H), 2.09 - 2.21 (m, 2H), 2.26 - 2.45 (m, 2H), 2.92 - 2.98 (m, 2H), 3.14 (s, 3H), 6.28 (dd, 1H), 7.53 (d, 1H), 7.60 - 7.65 (m, 2H), 7.67 - 7.71 (m, 1H), 8.44 (d, 1H), 8.50 (t, 1H).

### Example 9

### methyl 4- {[17-(5-fluoropyridin-3-yl)estra-1(10),2,4,16-tetraen-3-yl]sulfonyl}butanoate

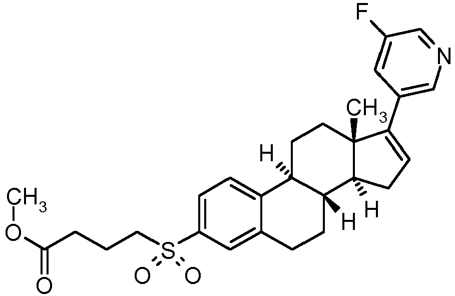

Analogously to the preparation of Example 1, 2.1 g (3.35 mmol) of methyl 4-{[17-{[(trifluoromethyl)sulfonyl]oxy}estra-1(10),2,4,16-tetraen-3-yl]sulfonyl}butanoate (Intermediate 5-A) were treated with 804 mg (5.70 mmol) of 5-fluoropyridine-3-boronic acid (CAS No.: 872041-86-6) and 188 mg (0.26 mmol) of dichlorobis(triphenylphosphine)palladium(II) (CAS No.: 13965-03-2). The residue was purified using a prepacked silica gel cartridge (mobile phase: cyclohexane/ethyl acetate, gradient 0% to 45%). The yield was 1.23 g (73% of theory) of the title compound.
LC-MS (method B): Rₜ = 5.61 min; m/z = 498.2 (M+H)⁺
¹H NMR (400 MHz, DMSO-d₆): δ [ppm] = 1.01 (s, 3H), 1.37 - 1.54 (m, 1H), 1.58 - 1.81 (m, 6H), 1.89 - 1.96 (m, 1H), 2.09 - 2.21 (m, 2H), 2.28 - 2.36 (m, 1H), 2.37 - 2.46 (m, 4H), 2.94 - 2.99 (m, 2H), 3.22 - 3.31 (m, 2H), 3.54 (s, 3H), 6.28 (dd, 1H), 7.53 - 7.61 (m, 3H), 7.67 - 7.72 (m, 1H), 8.44 (d, 1H), 8.50 (t, 1H).

### Example 10

### 4-{[17-(5-fluoropyridin-3-yl)estra-1(10),2,4,16-tetraen-3-yl]sulfonyl}butanoic acid

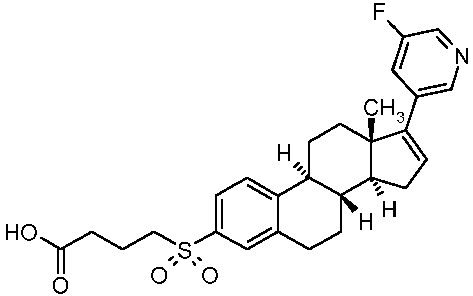

300 mg (0.60 mmol) of methyl 4-{[17-(5-fluoropyridin-3-yl)estra-1(10),2,4,16-tetraen-3-yl]sulfonyl}butanoate (Example 9) in 4.2 ml tetrahydrofuran were treated with 603 µl (1.20 mmol) of 2N lithium hydroxide solution. The mixture was stirred for 16 hours. The reaction mixture was acidified to pH 4 by addition of 1N hydrochloric acid solution and the reaction mixture was concentrated *in vacuo.* A part of the crude residue was purified by preparative HPLC (Method E). The yield was 66.3 mg of the title compound.
LC-MS (method B): Rₜ = 5.03 min; m/z = 484.1 (M+H)⁺
1H NMR (400 MHz, DMSO-d₆): δ [ppm] = 1.01 (s, 3H), 1.41 - 1.43 (m, 1H), 1.57 - 1.78 (m, 5H), 1.87 - 1.97 (m, 1H), 2.08 - 2.20 (m, 2H), 2.27 - 2.45 (m, 6H), 2.94 - 2.99 (m, 2H), 3.18 - 3.28 (m, 2H), 6.28 (dd, 1H), 7.52 - 7.60 (m, 3H), 7.66 - 7.71 (m, 1H), 8.44 (d, 1H), 8.50 (t, 1H, J = 1.7 Hz), 12.2 (br. s, 1H).

### Example 11

### methyl 4- {[17-(pyridin-3-yl)estra-1(10),2,4,16-tetraen-3-yl]sulfonyl}butanoate

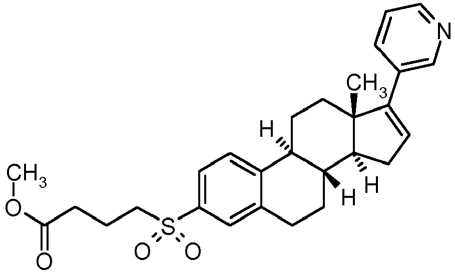

Analogously to the preparation of Example 9, 90 mg (0.16 mmol) of methyl 4-{[17-{[(trifluoromethyl)sulfonyl]oxy}estra-1(10),2,4,16-tetraen-3-yl]sulfonyl}butanoate (Intermediate 5-A) were treated with 34.1 mg (0.27 mmol) of pyridin-3-ylboronic acid (CAS No.: 1692-25-7) and 9.2 mg (0.013 mmol) of dichlorobis(triphenylphosphine)palladium(II) (CAS No.: 13965-03-2). The residue was purified using a prepacked silica gel cartridge (mobile phase: dichloromethane/ethyl acetate, gradient 0% to 40%). The yield was 41 mg (52% of theory) of the title compound.
LC-MS (method C): Rₜ = 3.48 min; m/z = 498.1 (M+H)⁺
1H NMR (300 MHz, CDCl₃): δ [ppm] = 1.07 (s, 3H), 1.46 - 1.63 (m, 1H), 1.64 - 1.91 (m, 5H), 2.00 - 2.24 (m, 3H), 2.38 - 2.51 (m, 6H), 3.01 (dd, 2H), 3.13 - 3.20 (m, 2H), 3.67 (s, 3H), 6.04 (dd, 1H), 7.22 - 7.26 (m, 1H), 7.47 (d, 1H), 7.61 - 7.71 (m, 3H), 8.49 (d, 1H), 8.64 - 8.67 (m, 1H).

### Example 12

### 4-{[17-(pyridin-3-yl)estra-1(10),2,4,16-tetraen-3-yl]sulfonyl}butanoic acid

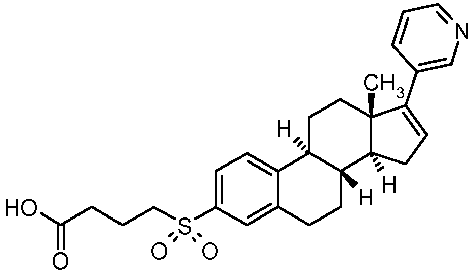

Analogously to the preparation of Example 10, 41 mg (0.085 mmol) of methyl 4-{[17-(pyridin-3-yl)estra-1(10),2,4,16-tetraen-3-yl]sulfonyl}butanoate were treated with 85 µl (0.17 mmol) of 2N lithium hydroxide solution. The crude residue was purified by chromatography using a prepacked C18 cartridge (mobile phase: acetonitrile/water 0.1% formic acid buffer, gradient 10% to 60%). The yield was 34.0 mg (84% of theory) of the title compound.
LC-MS (method B): Rₜ = 3.55 min; m/z = 466.1 (M+H)⁺
¹H NMR (400 MHz, DMSO-d₆): δ [ppm] = 1.00 (s, 3H), 1.41 - 1.53 (m, 1H), 1.57 - 1.80 (m, 6H), 1.89 - 1.96 (m, 1H), 2.07 - 2.17 (m, 2H), 2.27 - 2.33 (m, 3H), 2.33 - 2.45 (m, 2H), 2.92 - 2.99 (m, 2H), 3.10 - 3.19 (m, 2H), 6.12 - 6.14 (m, 1H), 7.33 (dd, 1H), 7.52 - 7.60 (m, 3H), 7.76 - 7.80 (m, 1H), 8.43 (dd, 1H), 8.60 (d, 1H).

### Example 13

### methyl 4-({17-[5-(trifluoromethyl)pyridin-3-yl]estra-1(1 0),2,4,16-tetraen-3-yl} sulfonyl)butanoate

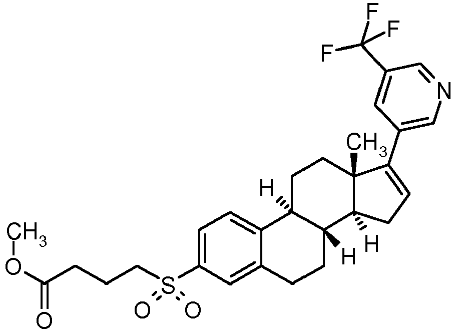

Analogously to the preparation of Example 9, 90 mg (0.16 mmol) of methyl 4-{[17-{[(trifluoromethyl)sulfonyl]oxy}estra-1(10),2,4,16-tetraen-3-yl]sulfonyl}butanoate (Intermediate 5-A) were treated with 53.0 mg (0.27 mmol) of [5-(trifluoromethyl)pyridin-3-yl]boronic acid (CAS No.: 947533-51-9) and 9.2 mg (0.013 mmol) of dichlorobis(triphenylphosphine)palladium(II) (CAS No.: 13965-03-2). The residue was purified using a prepacked silica gel cartridge (mobile phase: cyclohexane/ethyl acetate, gradient 0% to 40%). The yield was 66 mg (73% of theory) of the title compound.
LC-MS (method C): Rₜ = 4.35 min; m/z = 548.1 (M+H)⁺
¹H NMR (300 MHz, CDCl₃): δ [ppm] = 1.09 (s, 3H), 1.53 - 1.59 (m, 2H), 1.69 - 1.78 (m, 2H), 1.79 - 1.93 (m, 1H), 1.98 - 2.11 (m, 3H), 2.15 - 2.28 (m, 2H), 2.40 - 2.52 (m, 5H), 3.02 (dd, 2H), 3.13 - 3.20 (m, 2H), 3.67 (s, 3H), 6.14 - 6.18 (m, 1H), 7.47 (d, 1H), 7.62 - 7.69 (m, 2H), 7.89 (s, 1H), 8.77 (s, 1H), 8.82 - 8.83 (m, 1H).

### Example 14

### 4-({17-[5-(trifluoromethyl)pyridin-3-yl]estra-1(1 0),2,4,16-tetraen-3-yl} sulfonyl)butanoic acid

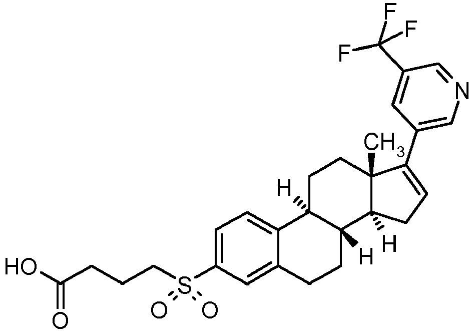

Analogously to the preparation of Example 10, 66 mg (0.12 mmol) of methyl 4-({17-[5-(trifluoromethyl)pyridin-3-yl]estra-1(10),2,4,16-tetraen-3-yl}sulfonyl)butanoate (Example 10) were treated with 121 µl (0.24 mmol) of 2N lithium hydroxide solution. The crude residue was purified by chromatography using a prepacked C18 cartridge (mobile phase: acetonitrile/water 0.1% formic acid buffer, gradient 20% to 80%). The yield was 55 mg of the title compound.
LC-MS (method B): Rₜ = 5.47 min, m/z = 534.1 (M+H)⁺
¹H NMR (400 MHz, DMSO-d₆): δ [ppm] = 1.02 (s, 3H), 1.46 - 1.79 (m, 6H), 1.91 - 1.98 (m, 1H), 2.11 - 2.19 (m, 2H), 2.27 - 2.44 (m, 6H), 2.93 - 3.00 (m, 2H), 3.17 - 3.24 (m, 2H), 6.35 - 6.38 (m, 1H), 7.52 - 7.61 (m, 3H), 8.05 (s, 1H), 8.84 (d, 1H), 8.91 (d, 1H), 12.12 (br. s, 1H).

### Example 15

### 4- {[17-(6-methylpyridin-3-yl)estra-1,3,5(10),16-tetraen-3-yl]sulfonyl}butanoic acid

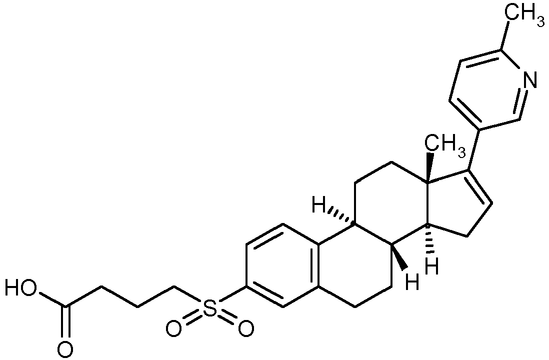

To 95 mg (0.17 mmol) methyl 4-{[17-{[(trifluoromethyl)sulfonyl]oxy}estra-1(10),2,4,16-tetraen-3-yl]sulfonyl}butanoate (Intermediate 5-A) in 2 ml dioxane 0.26 ml 2M aqueous sodium carbonate solution, 35 mg (0.26 mmol, 1.5 eq.) (6-methylpyridin-3-yl)boronic acid and 12 mg dichlorobis(triphenylphosphine)palladium(II) (CAS No.: 13965-03-2) were added and the mixture was heated in a closed vessel at 90°C. Then 0.4 ml 2M aqeous sodium hydroxide solution were added and the mixture was stirred for 17.5 h at 50°C. The mixture was diluted with water, acidified by aqueous citric acid to pH 4, extracted three times with ethyl acetate, evaporated and purified by HPLC (method G) resulting in 36 mg (43% yield) of the title compound.
LC-MS (method F): Rₜ = 0.93 min, m/z = 479.21.
¹H NMR (400 MHz, DMSO-d6): δ [ppm] = 1.02 (s, 3H), 1.43 - 1.83 (m), 1.91 - 2.00 (m, 1H), 2.09 - 2.21 (m, 2H), 2.26 - 2.49 (m), 2.94 - 3.04 (m, 2H), 3.24 - 3.32 (m), 6.06 - 6.11 (m, 1H), 7.22 (d, 1H), 7.55 - 7.65 (m, 3H), 7.70 (dd, 1H), 8.49 (d, 1H), 12.20 (br. s., 1H).

### Example 16

### 4-{[17-(5-methoxypyridin-3-yl)estra-1,3,5(10),16-tetraen-3-yl]sulfonyl}butanoic acid

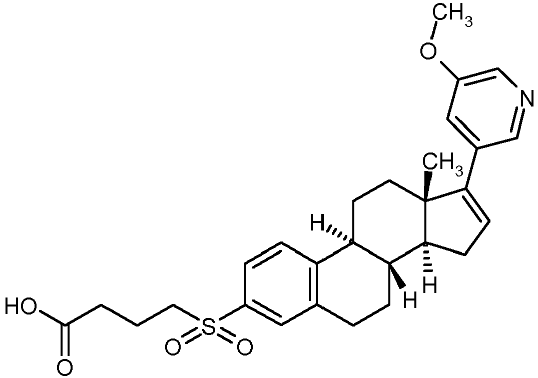

Analogously to Example 15 reaction of 95 mg (0.17 mmol) methyl 4-{[17-{[(trifluoromethyl)sulfonyl]oxy}estra-1(10),2,4,16-tetraen-3-yl]sulfonyl}butanoate (Intermediate 5-A) with 40 mg (5-methoxypyridin-3-yl)boronic acid afforded 33 mg of the title compound.
LC-MS (method F): Rₜ = 1.12 min, m/z = 495.21.
¹H NMR (400 MHz, DMSO-d6): δ [ppm] = 1.04 (s, 3H), 1.44 - 1.84 (m), 1.91 - 2.01 (m, 1H), 2.10 - 2.23 (m, 2H), 2.28 - 2.49 (m), 2.93 - 3.04 (m, 2H), 3.25 - 3.31 (m), 3.86 (s, 3H), 6.18 - 6.23 (m, 1H), 7.28 - 7.31 (m, 1H), 7.54 - 7.65 (m, 3H), 8.20 (d, 1H), 8.25 (d, 1H), 12.21 (br. s., 1H).

### Example 17

### 4- {[17-(5-methylpyridin-3-yl)estra-1,3,5(10),16-tetraen-3-yl]sulfonyl}butanoic acid

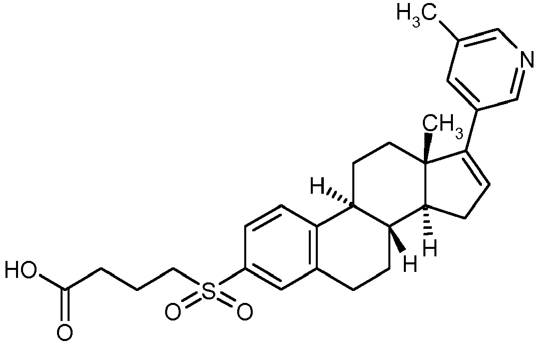

Analogously to Example 15 reaction of 95 mg (0.17 mmol) methyl 4-{[17-{[(trifluoromethyl)sulfonyl]oxy}estra-1(10),2,4,16-tetraen-3-yl]sulfonyl}butanoate (Intermediate 5-A) with 35 mg (5-methylpyridin-3-yl)boronic acid afforded 18 mg of the title compound.
LC-MS (method F): Rₜ = 0.99 min, m/z = 479.21.
¹H NMR (400 MHz, DMSO-d6): δ [ppm] = 1.04 (s, 3H), 1.44 - 1.84 (m), 1.90 - 2.00 (m, 1H), 2.10 - 2.23 (m, 2H), 2.25 - 2.49 (m), 2.92 - 3.04 (m, 2H), 3.22 - 3.32 (m), 6.14 (br. s., 1H), 7.53 - 7.67 (m, 4H), 8.29 - 8.33 (m, 1H), 8.43 (d, 1H), 12.21 (br. s., 1H).

### Example 18

### 4- {[17-(4-methylpyridin-3-yl)estra-1,3,5(10),16-tetraen-3-yl]sulfonyl}butanoic acid

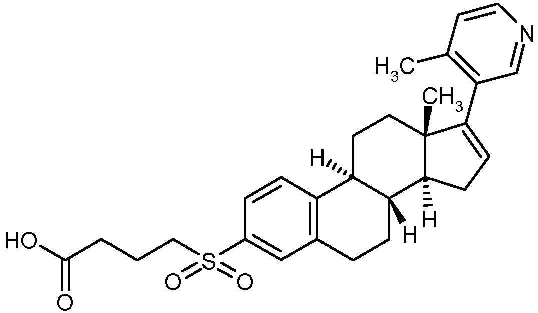

Analogously to Example 15 reaction of 95 mg (0.17 mmol) methyl 4-{[17-{[(trifluoromethyl)sulfonyl]oxy}estra-1(10),2,4,16-tetraen-3-yl]sulfonyl}butanoate (Intermediate 5-A) with 35 mg (4-methylpyridin-3-yl)boronic acid afforded 22 mg of the title compound.
LC-MS (method F): Rₜ = 0.93 min, m/z = 479.21.
¹H NMR (400 MHz, DMSO-d6): δ [ppm] = 0.91 (s, 3H), 1.45 - 1.80 (m), 1.80 - 1.90 (m, 1H), 1.93 - 2.04 (m, 1H), 2.20 (dd, 1H), 2.29 (s, 3H), 2.31 - 2.47 (m), 2.53 - 2.58 (m, 1H), 2.95 - 3.05 (m, 2H), 3.24 - 3.31 (m), 5.77 (d, 1H), 7.28 (d, 1H), 7.52 - 7.65 (m, 3H), 8.26 (s, 1H), 8.33 (d, 1H), 12.21 (br. s., 1H).

### Example 19

### 4-{[17-(5-chloropyridin-3-yl)estra-1,3,5(10),16-tetraen-3-yl]sulfonyl}butanoic acid

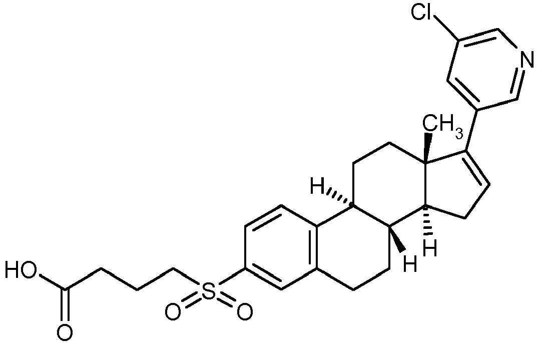

Analogously to Example 15 reaction of 95 mg (0.17 mmol) methyl 4-{[17-{[(trifluoromethyl)sulfonyl]oxy}estra-1(10),2,4,16-tetraen-3-yl]sulfonyl}butanoate (Intermediate 5-A) with 41 mg (5-chloropyridin-3-yl)boronic acid afforded 39 mg of the title compound.
LC-MS (method F): Rₜ = 1.41 min, m/z = 499.16.
¹H NMR (400 MHz, DMSO-d6): δ [ppm] = 1.04 (s, 3H), 1.47 - 1.84 (m), 1.91 - 2.01 (m, 1H), 2.10 - 2.25 (m, 2H), 2.27 - 2.49 (m), 2.92 - 3.04 (m, 2H), 3.21 - 3.32 (m), 6.29 - 6.34 (m, 1H), 7.54 - 7.65 (m, 3H), 7.90 (t, 1H), 8.53 (d, 1H), 8.61 (d, 1H), 12.22 (br. s., 1H).

### Example 20

### 4-{[17-(5-fluoropyridin-3-yl)estra-1(10),2,4,16-tetraen-3-yl]sulfonyl}butanamide

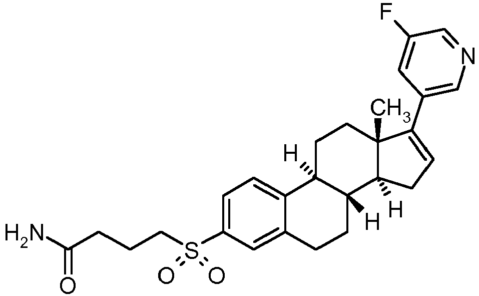

To a solution of 248 mg (0.51 mmol) of 4-{[17-(5-fluoropyridin-3-yl)estra-1(10),2,4,16-tetraen-3-yl]sulfonyl}butanoic acid (Example 10) in N,N-dimethylformamide (5.2 ml) 124 µl (1.12 mmol) of 4-methylmorpholine, 214 mg (0.56 mmol) of HATU and after 10 min, 366 µl (2.564 mmol) of 7N ammonia in methanol were added. The mixture was stirred for 2 hours. It was then partially concentrated under reduced pressure and the residue was partitioned between water and ethyl acetate. The organic phase was further washed with brine, dried over sodium sulfate, filtered and concentrated in vacuo. The residue was purified by HPLC (mobile phase: acetonitrile/water 0.1% formic acid buffer, gradient 10% to 95%). The yield was 137 mg (55% of theory) of the title compound.
LC-MS (method B): Rₜ = 4.71 min; m/z = 483.2 (M+H)⁺
¹H NMR (400 MHz, DMSO-d₆): δ [ppm] = 1.01 (s, 3H), 1.41 - 1.53 (m, 1H), 1.56 - 1.78 (m, 6H), 1.89 - 1.96 (m, 1H), 2.09 - 2.18 (m, 4H), 2.27 - 2.45 (m, 3H), 2.93 - 2.99 (m, 2H), 3.19 - 3.25 (m, 2H), 6.26 - 6.28 (m, 1H), 6.70 - 6.76 (m, 1H), 7.25 - 7.28 (m, 1H), 7.52 - 7.61 (m, 3H), 7.67 - 7.71 (m, 1H), 8.44 (d, 1H), 8.50 (t, 1H).

### Example 21

### 4-{[17-(5-fluoropyridin-3-yl)estra-1(10),2,4,16-tetraen-3-yl]sulfonyl}butan-1-ol

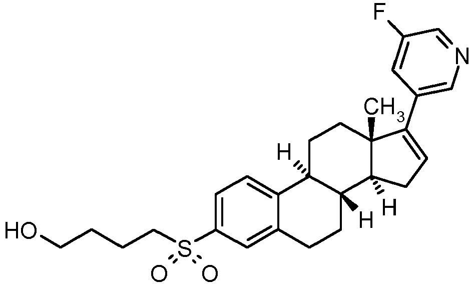

To a solution of 200 mg (0.40 mmol) of methyl 4-{[17-(5-fluoropyridin-3-yl)estra-1(10),2,4,16-tetraen-3-yl]sulfonyl}butanoate (Example 9) in 6.7 ml of dichloromethane under argon at -78°C 1.2 ml (1.20 mmol) of diisobutylaluminum hydride (1M in dichloromethane) was added dropwise over 5 minutes. After 10 minutes at -78°C the reaction mixture was allowed to warm at -20°C and stirred for 20 minutes and then allowed to warm to RT and stirred for 10 minutes. Addition of 500 µl of water dropwise and stirring for 10 minutes, then addition of 520 mg of sodium hydrogen carbonate and stirring for 30 minutes followed by sodium sulfate and stirring for 10 minutes, filtration and concentration *in vacuo.* The residue was purified by chromatography using a prepacked silica gel cartridge (mobile phase: acetonitrile/ethyl acetate, gradient 0% to 30%). The yield was 134 mg (69% of theory) of the title compound.
LC-MS (method B): Rₜ = 5.06 min; m/z = 470.2 (M+H)⁺
¹H-NMR (400MHz, DMSO-d₆): δ [ppm] = 1.01 (s, 3H), 1.40 - 1.77 (m, 9H), 1.87 - 1.97 (m, 1H), 2.09 - 2.20 (m, 2H), 2.27 - 2.45 (m, 3H), 2.92 - 2.99 (m, 2H), 3.18 - 3.25 (m, 4H), 4.40 (dd, 1H), 6.28 (dd, 1H), 7.51 - 7.61 (m, 3H), 7.66 - 7.71 (m, 1H), 8.44 (d, 1H), 8.50 (t, 1H).

### Examples type IC3

### Example 22

### tert-butyl 4-{[17-(5-fluoropyridin-3-yl)estra-(10),2,4,16-tetraen-3-yl]amino}-4-oxobutanoate

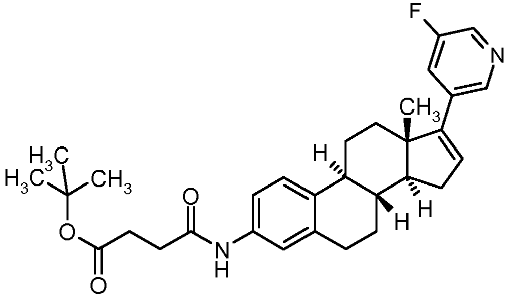

To a solution of 1.41 g (2.63 mmol) of *tert*-butyl 4-{[17-iodoestra-1(10),2,4,16-tetraen-3-yl]amino}-4-oxobutanoate (Intermediate 8-A) in 6.8 ml of toluene and 4.4 ml of ethanol 519 mg (3.68 mmol) of 5-fluoropyridine-3-boronic acid (CAS No.: 872041-86-6), 223 mg (5.26 mmol) of lithium chloride, 243 mg (0.21 mmol) of tetrakis(triphenylphosphine)palladium(0) and 3.3 ml of 2M sodium carbonate solution were added. The reaction mixture was degassed and then stirred in a sealed tube for 3 hours at 100°C. The reaction mixture was allowed to cool to RT and partitioned between water and ethyl acetate. The organic phase was further washed with brine, dried over sodium sulfate, filtered and concentrated *in vacuo.* The residue was purified by chromatography using a prepacked silica gel cartridge (mobile phase: dichloromethane/diethyl ether, gradient 0% to 60%). The yield was 457 mg (34% of theory) of the title compound.
LC-MS (method D): Rₜ = 4.65 min; m/z = 505.3 (M+H)⁺
¹H-NMR (400 MHz, CDCl₃): δ [ppm] = 1.03 (s, 3H), 1.22 - 1.29 (m, 1H), 1.45 (s, 8H), 1.46 - 1.54 (m, 1H), 1.60 - 1.69 (m, 3H), 1.76 - 1.85 (m, 1H), 1.96 (ddd, 1H), 2.10 - 2.19 (m, 2H), 2.27 - 2.41 (m, 3H), 2.54 - 2.68 (m, 4H), 2.87 - 2.94 (m, 2H), 6.08 (dd, 1H), 7.19 (s, 2H), 7.31 (s, 1H), 7.36 - 7.41 (m, 1H), 7.56 (s, 1H), 8.33 (d, 1H), 8.46 (dd, 1H)

### Example 23

### 4-{[17-(5-fluoropyridin-3-yl)estra-1(10),2,4,16-tetraen-3-yl]amino}-4-oxobutanoic acid

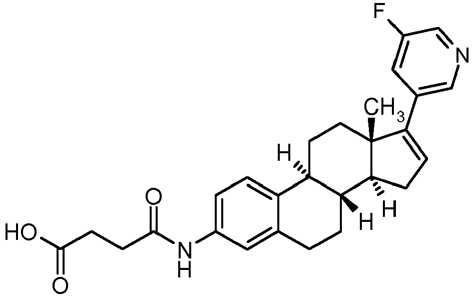

To a solution of 178 mg (0.35 mmol) of *tert*-butyl 4-{[17-(5-fluoropyridin-3-yl)estra-1(10),2,4,16-tetraen-3-yl]amino}-4-oxobutanoate (Example 22) in 7.4 ml of dichloromethane 1.77 ml (22.9 mmol) of trifluoroacetic acid was added. The mixture was stirred at RT for 1 hour and concentrated under reduced pressure. 69 mg of the residue were purified by preparative HPLC (Method E). The yield was 11 mg (20% of theory) of the title compound.
LC-MS (method B): Rₜ = 4.97 min; m/z = 449.2 (M+H)⁺
¹H-NMR (400MHz, DMSO-d₆): δ [ppm] = 1.00 (s, 3H), 1.36 - 1.62 (m, 4H), 1.67 - 1.77 (m, 1H), 1.84 - 1.92 (m, 1H), 2.08 - 2.17 (m, 2H), 2.20 - 2.39 (m, 3H), 2.75 - 2.84 (m, 2H), 3.16 - 3.44 (m, 4H), 6.25 - 6.27 (m, 1H), 7.14 (d, 1H), 7.26 (dd, 1H), 7.30 - 7.32 (m, 1H), 7.66 - 7.71 (m, 1H), 8.43 (d, 1H), 8.49 (dd, 1H), 9.80 - 9.85 (m, 1H), 12.10 (br. s, 1H).

### Example 24

### tert-butyl 4-oxo-4-{[17-(pyrimidin-5-yl)estra-1(10),2,4,16-tetraen-3-yl]amino}butanoate

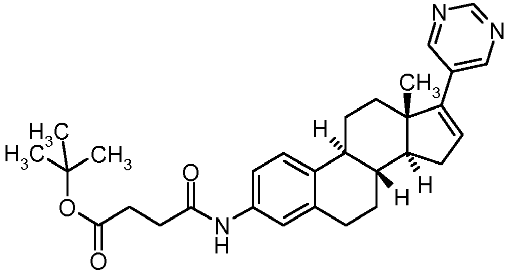

To a solution of 250 mg (0.46 mmol) of *tert*-butyl 4-{[17-iodoestra-1(10),2,4,16-tetraen-3-yl]amino}-4-oxobutanoate (Intermediate 8-A) in 2.8 ml of dioxane 101 mg (0.81 mmol) of pyrimidin-5-ylboronic acid (CAS No.: 109299-78-7), 29.5 mg (0.04 mmol) of dichlorobis(triphenylphosphine)palladium(II) (CAS No.: 13965-03-2) and 934 µl of 2M sodium carbonate solution were added. The reaction mixture was degassed and then stirred in a sealed tube for 18 hours at 90°C. The reaction mixture was allowed to cool to RT and partitioned between water and ethyl acetate. The organic phase was further washed with brine, dried over sodium sulfate, filtered and concentrated *in vacuo.* The residue was purified by chromatography using a prepacked silica gel cartridge (mobile phase: cyclohexane/ethyl acetate, gradient 0% to 50%). The yield was 58 mg (25% of theory) of the title compound.
LC-MS (method A): Rₜ = 4.11 min; m/z = 488.2 (M+H)⁺
¹H NMR (300 MHz, CDCl₃): δ [ppm] = 0.81 - 0.95 (m, 1H), 1.45 (s, 9H), 1.50 - 1.90 (m, 5H), 1.91 - 2.03 (m, 1H), 2.11 - 2.24 (m, 2H), 2.28 - 2.47 (m, 3H), 2.54 - 2.71 (m, 4H), 2.88 - 2.97 (m, 2H), 6.13 (dd, 1H), 7.21 (s, 2H), 7.33 (s, 1H), 7.57 (s, 1H), 8.76 (s, 2H), 9.09 (s, 1H).

### Example 25

### 4-oxo-4-{[17-(pyrimidin-5-yl)estra-1(10),2,4,16-tetraen-3-yl]amino}butanoic acid

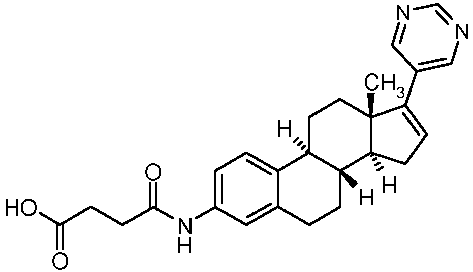

Analogously to the Example 23, 58 mg (0.12 mmol) of *tert-butyl* 4-oxo-4-{[17-(pyrimidin-5-yl)estra-1(10),2,4,16-tetraen-3-yl]amino}butanoate (Example 24) were treated with 0.45 ml (5.94 mmol) of trifluoroacetic acid. The residue was diluted with ethyl acetate and saturated sodium hydrogen carbonate solution was added. The mixture was extracted 4 times with dichloromethane/methanol 9: 1. The organic phase was washed with brine, dried over sodium sulfate, filtered and concentrated *in vacuo.* The yield was 39.8 mg (77% of theory) of the title compound.
LC-MS (method B): Rₜ = 4.21 min; m/z = 432.2 (M+H)⁺
¹H NMR (400 MHz, DMSO-d₆): δ [ppm] = 0.78 - 0.85 (m, 1H), 0.99 (s, 3H), 1.12 - 1.63 (m, 6H), 1.68 - 1.77 (m, 1H), 1.86 - 1.96 (m, 1H), 2.08 - 2.38 (m, 7H), 2.63 - 2.84 (m, 2H), 6.30 (dd, 1H), 7.12 (d, 1H), 7.22 - 7.28 (m, 2H), 8.82 (s, 2H), 9.04 (s, 1H), 11.52 (br. s, 1H).

### Example 26

### N-[17-(5-fluoropyridin-3-yl)estra-1(10),2,4,16-tetraen-3-yl]butanediamide

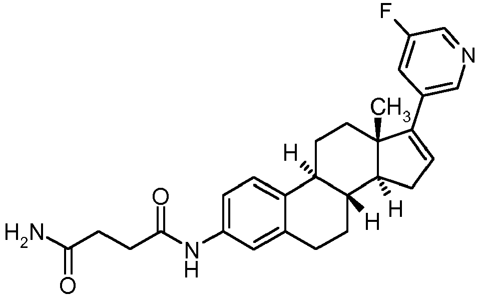

To a solution of 210 mg (0.28 mmol) of 4-{[17-(5-fluoropyridin-3-yl)estra-1(10),2,4,16-tetraen-3-yl]amino}-4-oxobutanoic acid (Example 23) in 4.2 ml of N,N-dimethylformamide 68 µl (0.61 mmol) of 4-methylmorpholine, 117 mg (0.30 mmol) of 1-[bis(dimethylamino)methylene]-1H-1,2,3-triazolo[4,5-b]pyridinium 3-oxid hexafluorophosphate were added and after 5 minutes, 200 µl (1.40 mmol) of 7N ammonia in methanol. The mixture was stirred for 2 hours at RT. It was then partially concentrated under reduced pressure and the residue was partitioned between water and ethyl acetate. The organic phase was further washed with brine, dried over sodium sulfate, filtered and concentrated *in vacuo.* The residue was purified by chromatography using a prepacked reverse phase cartridge (mobile phase: acetonitrile/water 0.1% formic acid buffer, gradient 20% to 95%). The yield was 23 mg (18% of theory) of the title compound.
LC-MS (method B): Rₜ = 4.67 min; m/z = 448.2 (M+H)⁺
¹H-NMR (400 MHz, DMSO-d₆): δ [ppm] = 1.00 (s, 3H), 1.36 - 1.62 (m, 4H), 1.67 - 1.77 (m, 1H), 1.84 - 1.92 (m, 1H), 2.04 - 2.36 (m, 6H), 2.43 - 2.50 (m, 5H), 2.72 - 2.84 (m, 2H), 6.25 - 6.27 (m, 1H), 6.68 - 6.74 (m, 1H), 7.14 (d, 1H), 7.26 (dd, 2H), 7.31 (s, 1H), 7.65 - 7.70 (m, 1H), 8.43 (d, 1H), 8.49 (dd, 1H), 9.72 (s, 1H).

### Example 27

### N-[17-(5-fluoropyridin-3-yl)estra-1(10),2,4,16-tetraen-3-yl]-4-hydroxybutanamide

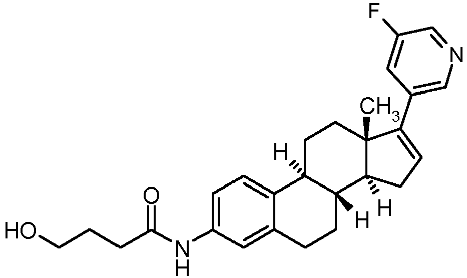

To a solution of 70 mg (0.20 mmol) of 17-(5-fluoropyridin-3-yl)estra-1(10),2,4,16-tetraen-3-amine (Intermediate 10-A) in 1.8 ml of N,N-dimethylformamide under argon 30.4 mg (0.24 mmol) of 4-hydroxybutyric acid, sodium salt (CAS No.: 502-85-2), 27 µl (0.24 mmol) of 4-methylmorpholine and 91.6 mg (0.24 mmol) of 1-[bis-(dimethylamino)methylene]-1H-1,2,3-triazolo[4,5-b]pyridinium 3-oxid hexafluorophosphate were added. The mixture was stirred at RT for 2 hours. Then further 30.4 mg (0.24 mmol) of 4-hydroxybutyric acid, sodium salt, 27 µl (0.24 mmol) of 4-methylmorpholine and 91.6 mg (0.24 mmol) of 1-[bis-(dimethylamino)methylene]-1H-1,2,3-triazolo[4,5-b]pyridinium 3-oxid hexafluorophosphate were added and stirring continued for another 16 hours at RT. The reaction mixture was partitioned between water and ethyl acetate. The organic phase was further washed with brine, dried over sodium sulfate, filtered and concentrated *in vacuo.* The residue was purified by chromatography using a prepacked silica gel cartridge (mobile phase: dichloromethane/methanol, gradient 0% to 5%) and then by chromatography using a prepacked reverse phase cartridge (mobile phase: acetonitrile/water 0.1% formic acid buffer, gradient 40% to 98%). The yield was 25 mg (28% of theory) of the title compound.
LC-MS (method B): Rₜ = 4.89 min; m/z = 435.2 (M+H)⁺
¹H-NMR (400 MHz, CD₃OD): δ [ppm] = 1.08 (s, 3H), 1.42 - 1.54 (m, 1H), 1.61 - 1.72 (m, 3H), 1.79 - 2.02 (m, 4H), 2.15 - 2.23 (m, 2H), 2.39 - 2.47 (m, 6H), 2.86 - 2.93 (m, 2H), 3.60 (dd, 2H), 6.20 (dd, 1H), 7.18 - 7.27 (m, 3H), 7.61 - 7.66 (m, 1H), 8.32 (d, 1H), 8.44 (dd, 1H).

### Example 28

### N-[17-(5-fluoropyridin-3-yl)estra-1(10),2,4,16-tetraen-3-yl]-3-sulfamoylpropanamide

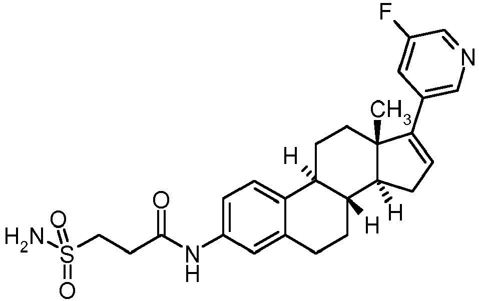

Analogously to the preparation of Intermediate 6-A, 155 mg (0.445 mmol) of 17-(5-fluoropyridin-3-yl)estra-1(10),2,4,16-tetraen-3-amine (Intermediate 10-A) were treated with 372 mg (0.98 mmol) of 1-[bis-(dimethylamino)methylene]-1H-1,2,3-triazolo[4,5-b]pyridinium 3-oxid hexafluorophosphate, 150 mg (0.98 mmol) of 3-sulfamoylpropanoic acid (CAS No.: 15441-10-8) and 108 µl (0.98 mmol) of 4-methylmorpholine. The residue was purified by chromatography using a prepacked silica gel cartridge (mobile phase: dichloromethane/methanol, gradient 0% to 6%). The yield was 187 mg (86% of theory) of the title compound.
LC-MS (method A): Rₜ = 3.63 min; m/z = 484.2 (M+H)⁺
¹H NMR (300 MHz, CDCl₃): δ [ppm] = 1.00 (s, 3H), 1.38 - 1.46 (m, 1H), 1.52 - 1.68 (m, 3H), 1.72 - 1.78 (m, 1H), 1.87 - 1.94 (m, 1H), 2.05 - 2.18 (m, 2H), 2.19 - 2.49 (m, 3H), 2.77 - 2.95 (m, 4H), 3.50 - 3.61 (m, 2H), 5.08 - 5.33 (m, 2H), 6.08 (s, 1H), 7.14 - 7.21 (m, 2H), 7.22 - 7.27 (m, 1H), 7.38 (d, 1H), 7.77 - 7.93 (m, 1H), 8.33 (d, 1H), 8.45 (s, 1H).

### Example 29

### 3-(acetylsulfamoyl)-N-[17-(5-fluoropyridin-3-yl)estra-1(10),2,4,16-tetraen-3-yl]propanamide

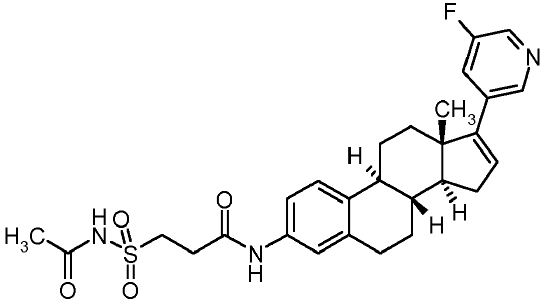

To a solution of 167 mg (0.34 mmol) of N-[17-(5-fluoropyridin-3-yl)estra-1(10),2,4,16-tetraen-3-yl]-3-sulfamoylpropanamide (Example 28) in dichloromethane (8.5 ml) were added 35 µl (0.60 mmol) of acetic acid, 116 mg (0.60 mmol) of 1-(3-dimethylaminopropyl)-3-ethylcarbodiimide hydrochloride and 73.8 mg (0.60 mmol) of 4-dimethylaminopyridine. The reaction was repeated using 20 mg N-[17-(5-fluoropyridin-3-yl)estra-1 (1 0),2,4,16-tetraen-3 -yl] -3 -sulfamoylpropanamide. The combined reaction mixtures were diluted with dichloromethane and washed with brine, the organic phase was dried over sodium sulfate, filtered and concentrated in vacuo. The residue was purified by reverse phase C18 chromatography (mobile phase: acetonitrile/water 0.1% formic acid buffer, gradient 10% to 95%). The yield was 126 mg of the title compound.
LC-MS (method B): Rₜ = 4.99 min; m/z = 526.1 (M+H)⁺
¹H NMR (400 MHz, DMSO-d₆): δ [ppm] = 1.00 (s, 3H), 1.37 - 1.62 (m, 4H), 1.68 - 1.77 (m, 1H), 1.84 - 1.92 (m, 1H), 1.96 (s, 3H), 2.08 - 2.40 (m, 5H), 2.70 - 2.85 (m, 4H), 3.61 (t, 2H), 6.25 - 6.27 (m, 1H), 7.17 (d, 1H), 7.24 - 7.30 (m, 2H), 7.66 - 7.70 (m, 1H), 8.44 (d, 1H), 8.48 - 8.51 (m, 1H), 9.92 (s, 1H), 11.63 (br. s., 1H).

### Example 30

### N-[17-(5-fluoropyridin-3-yl)estra-1(10),2,4,16-tetraen-3-yl]-N-methy1-3-sulfamoylpropanamide

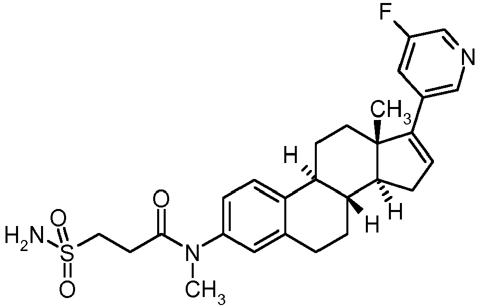

Analogously to the preparation of Intermediate 6-A, 230 mg (0.634 mmol) of 17-(5-fluoropyridin-3-yl)-N-methylestra-1(10),2,4,16-tetraen-3-amine (Intermediate 11-A) were treated with 531 mg (1.39 mmol) of 1-[bis-(dimethylamino)methylene]-1H-1,2,3-triazolo[4,5-b]pyridinium 3-oxid hexafluorophosphate, 214 mg (1.39 mmol) of 3-sulfamoylpropanoic acid (CAS No.: 15441-10-8) and 153 µl (1.39 mmol) of 4-methylmorpholine. The residue was purified by chromatography using a prepacked silica gel cartridge (mobile phase: dichloromethane/methanol, gradient 0% to 5%). The yield was 246 mg (77% of theory) of the title compound.
LC-MS (method C): Rₜ = 3.46 min; m/z = 498.0 (M+H)⁺
¹H NMR (400 MHz, CDCl₃): δ [ppm] = 1.09 (s, 3H), 1.46 - 1.60 (m, 1H), 1.63 - 1.89 (m, 4H), 1.98 - 2.05 (m, 1H), 2.14 - 2.24 (m, 2H), 2.36 - 2.47 (m, 3H), 2.67 (t, 2H), 2.91 - 2.98 (m, 2H), 3.26 (s, 3H), 3.45 (t, 2H), 4.90 (s, 2H), 6.11 (dd, 1H), 6.92 - 6.99 (m, 2H), 7.33 (d, 1H), 7.38 - 7.43 (m, 1H), 8.35 (d, 1H), 8.48 (dd, 1H).

### Example 31

### 3-(acetylsulfamoyl)-N-[17-(5-fluoropyridin-3-yl)estra-1(10),2,4,16-tetraen-3-yl]-N-methylpropanamide

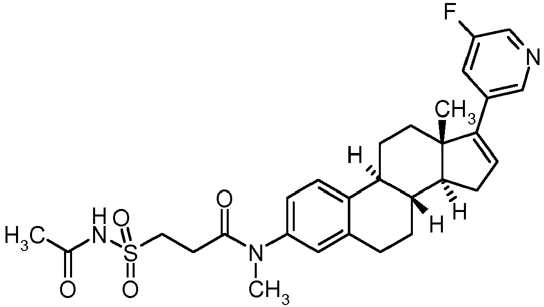

Analogously to the preparation of Example 29, 185 mg (0.37 mmol) of N-[17-(5-fluoropyridin-3-yl)estra-1(10),2,4,16-tetraen-3-yl]-N-methyl-3-sulfamoylpropanamide in dichloromethane (9.25 ml) were treated with 37 µl (0.65 mmol) of acetic acid, 125 mg (0.65 mmol) of 1-(3-dimethylaminopropyl)-3-ethylcarbodiimide hydrochloride (CAS No.: 25952-53-8) and 79.5 mg (0.65 mmol) of 4-dimethylaminopyridine. The mixture was stirred for 18 hours. The reaction was repeated analogously using 20 mg N-[17-(5-fluoropyridin-3-yl)estra-1(10),2,4,16-tetraen-3-yl]-N-methyl-3-sulfamoylpropanamide. The combined reaction mixtures were diluted with dichloromethane and washed with brine, organic phase was dried over sodium sulfate, filtered and concentrated in vacuo. The residue was purified by preparative HPLC (Method E). The yield was 140 mg of the title compound.
LC-MS (method B): Rt = 5.12 min; m/z = 540.2 (M+H)+
1H NMR (400 MHz, DMSO-d6): δ [ppm] = 1.02 (s, 3H), 1.38 - 1.56 (m, 1H), 1.56 - 1.76 (m, 4H), 1.91 (s, 4H), 2.08 - 2.20 (m, 2H), 2.27 - 2.37 (m, 2H), 2.38 - 2.45 (m, 3H), 2.83 - 2.91 (m, 2H), 3.10 (s, 3H), 3.50 (dd, 2H), 6.26 - 6.28 (m, 1H), 7.04 - 7.12 (m, 2H), 7.34 (d, 1H), 7.66 - 7.71 (m, 1H), 8.44 (d, 1H), 8.49 - 8.51 (m, 1H), 11.51 (br. s, 1H).

### Example 32

### N-[17-(5-fluoropyridin-3-yl)estra-1(10),2,4,16-tetraen-3-yl]-3-(2H-tetrazol-5-yl)propanamide

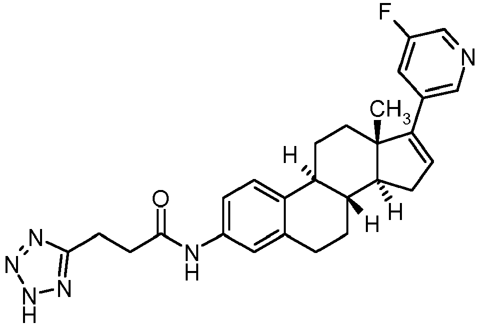

Analogously to the preparation of Intermediate 6-A, 85 mg (0.24 mmol) of 17-(5-fluoropyridin-3-yl)estra-1(10),2,4,16-tetraen-3-amine (Intermediate 10-A) were treated with 185 mg (0.48 mmol) of 1-[bis-(dimethylamino)methylene]-1H-1,2,3-triazolo[4,5-b]pyridinium 3-oxid hexafluorophosphate, 69.3 mg (0.48 mmol) of 3-(2H-tetrazol-5-yl)propanoic acid (CAS No.: 100508-42-7, Afferchem Inc.) and 107 µl (0.97 mmol) of 4-methylmorpholine. The residue was purified by chromatography using a prepacked C18 cartridge (mobile phase: acetonitrile: water 0.1% formic acid buffer, gradient 10% to 90%). The yield was 54 mg (46% of theory) of the title compound.
LC-MS (method B): Rₜ = 4.81 min; m/z = 473.2 (M+H)⁺
¹H NMR (400 MHz, DMSO-d₆): δ [ppm] = 0.99 (s, 3H), 1.36 - 1.62 (m, 4H), 1.67 - 1.77 (m, 1H), 1.84 - 1.92 (m, 1H), 2.08 - 2.39 (m, 6H), 2.72 - 2.84 (m, 4H), 3.04 - 3.15 (m, 2H), 6.25 - 6.27 (m, 1H), 7.15 (d, 1H), 7.24 - 7.31 (m, 2H), 7.65 - 7.70 (m, 1H), 8.43 (d, 1H), 8.48 - 8.50 (m, 1H), 9.87 (s, 1H).

### Example 33

### N-[17-(5-fluoropyridin-3-yl)estra-1(10),2,4,16-tetraen-3-yl]-N-methyl]-3-(2H-tetrazol-5-yl)propanamide

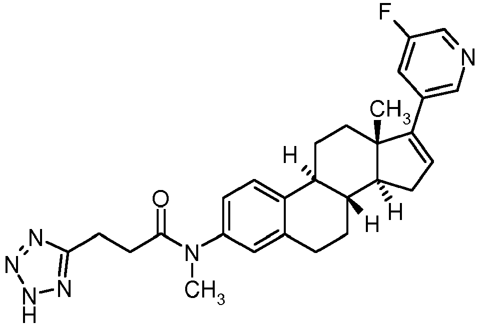

Analogously to the preparation of Intermediate 6-A, 90 mg (0.24 mmol) of 17-(5-fluoropyridin-3-yl)-N-methylestra-1(10),2,4,16-tetraen-3-amine (Intermediate 11-A) were treated with 123 mg (0.32 mmol) of 1-[bis-(dimethylamino)methylene]-1H-1,2,3-triazolo[4,5-b]pyridinium 3-oxid hexafluorophosphate, 46.0 mg (0.32 mmol) of 3-(2H-tetrazol-5-yl)propanoic acid (CAS No.: 100508-42-7, Afferchem Inc.) and 71 µl (0.64 mmol) of 4-methylmorpholine. The residue was purified by preparative HPLC (Method E) . The yield was 35 mg (24% of theory) of the title compound.
LC-MS (method B): Rₜ = 5.05 min, m/z = 487.1 (M+H)⁺
¹H NMR (400 MHz, DMSO-d₆): δ [ppm] = 1.01 (s, 3H), 1.38 - 1.52 (m, 1H), 1.56 - 1.76 (m, 4H), 1.86 - 1.94 (m, 1H), 2.08 - 2.19 (m, 2H), 2.26 - 2.36 (m, 2H), 2.39 - 2.44 (m, 1H), 2.49 - 2.56 (m, 2H), 2.82 - 2.87 (m, 2H), 3.00 (t, 2H), 3.08 (s, 3H), 6.26 - 6.28 (m, 1H), 6.98 - 7.08 (m, 2H), 7.33 (d, 1H), 7.66 - 7.71 (m, 1H), 8.44 (d, 1H), 8.50 (t, 1H).

### Examples type IC4

### Example 34

### tert-butyl 4-{[17-(5-fluoropyridin-3-yl)estra-1(10),2,4,16-tetraen-3-yl](methyl)amino}-4-oxobutanoate

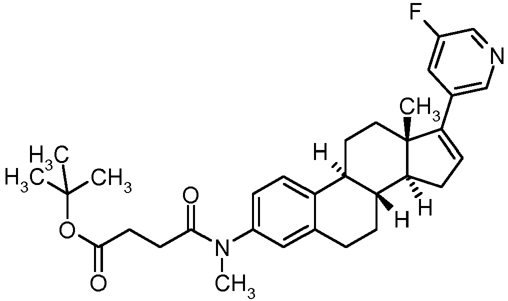

To a solution of 131 mg (0.26 mmol) of *tert*-butyl 4-{[17-(5-fluoropyridin-3-yl)estra-1(10),2,4,16-tetraen-3-yl]amino}-4-oxobutanoate (Example 22) in 800 µl of dry N,N-dimethylformamide at 0°C under argon 81 µl (1.29 mmol) of iodomethane followed by 11.5 mg (0.28 mmol) of sodium hydride (60% in oil) were added. The mixture was stirred for 1 hour at 0°C and then allowed to warm to RT for 30 minutes. Water was carefully added to the reaction mixture and then extraction into ethyl acetate. The organic phase was further washed with brine, dried over sodium sulfate, filtered and concentrated *in vacuo.* The residue was purified by chromatography using a prepacked silica gel cartridge (mobile phase: dichloromethane/ethyl acetate, gradient 0% to 15%). The yield was 32 mg (24% of theory) of the title compound.
LC-MS (method D): Rₜ = 4.91 min, m/z = 519.5 (M+H)⁺
¹H-NMR (400 MHz, CDCl₃): δ [ppm] = 1.08 (s, 3H), 1.43 (s, 9H), 1.46 - 1.58 (m, 1H), 1.66 - 1.76 (m, 3H), 1.76 - 1.88 (m, 1H), 1.96 - 2.04 (m, 1H), 2.13 - 2.24 (m, 2H), 2.31 - 2.45 (m, 5H), 2.51 (dd, 2H), 2.90 - 2.97 (m, 2H), 3.24 (s, 3H), 6.11 (dd, 1H), 6.94 - 6.95 (m, 1H), 6.98 (dd, 1H), 7.31 (d, 1H), 7.38 - 7.43 (m, 1H), 8.35 (d, 1H), 8.48 (dd, 1H).

### Example 35

### 4-{[17-(5-fluoropyridin-3-yl)estra-1(10),2,4,16-tetraen-3-yl](methyl)amino}-4-oxobutanoic acid

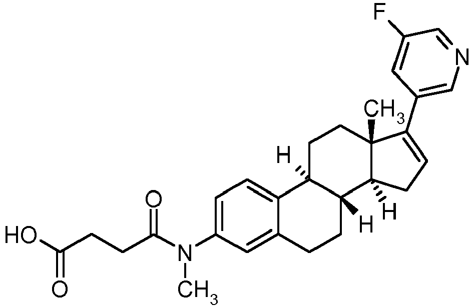

Analogously to the preparation of Example 23, 32 mg (0.06 mmol) of *tert*-butyl 4-{[17-(5-fluoropyridin-3-yl)estra-1(10),2,4,16-tetraen-3-yl](methyl)amino}-4-oxobutanoate were reacted with 238 µl (3.08 mmol) of trifluoroacetic acid. The crude residue was purified by chromatography using a prepacked silica gel cartridge (mobile phase: ethyl acetate/ methanol: 30% triethylamine in water 7: 3, gradient 0% to 20%). The yield was 31 mg (83% of theory) of the title compound as a 1.0 equivalent trifluoroacetic acid salt.
LC-MS (method B): Rₜ = 5.19 min; m/z = 463.2 (M+H)⁺
¹H-NMR (400 MHz, CD₃OD): δ [ppm] = 1.09 (s, 3H), 1.27 (s, 1H), 1.46 - 1.59 (m, 1H), 1.66 - 1.75 (m, 3H), 1.82 - 1.91 (m, 1H), 1.99 - 2.07 (m, 1H), 2.16 - 2.25 (m, 2H), 2.31 - 2.52 (m, 6H), 2.91 - 2.99 (m, 2H), 3.20 (s, 3H), 6.21 (dd, 1H), 7.01 - 7.07 (m, 2H), 7.38 (d, 1H), 7.62 - 7.67 (m, 1H), 8.33 (d, 1H), 8.45 (s, 1H).

### Examples type IC5

### Example 36

### methyl 3- {[17 -(5-fluoropyridin-3-yl)estra-1 (1 0),2,4,16-tetraen-3 -yl] (methyl)sulfamoyl} propanoate

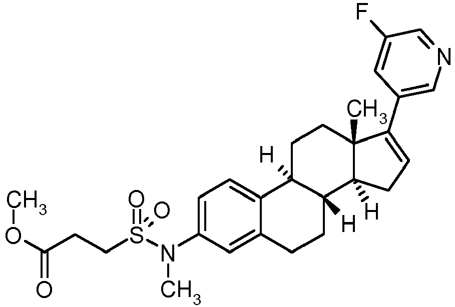

To a solution of 32 mg (0.08 mmol) of 17-(5-fluoropyridin-3-yl)-N-methylestra-1(10),2,4,16-tetraen-3-amine (Intermediate 11-A) in 700 µl of N,N-dimethylformamide under argon at 0°C 37 µl (0.26 mmol) of triethylamine and 33 mg (0.17 mmol) of 3-chlorosulfonyl-propionic acid methyl ester (CAS No.: 15441-07-3) were added. The mixture was then allowed to warm to RT and stirred for 1 hour. Water was then added and the contents extracted into ethyl acetate. The organic phase was further washed with brine, dried over sodium sulfate, filtered and concentrated *in vacuo.* The residue was purified by chromatography using a prepacked silica gel cartridge (mobile phase: cyclohexane/ethyl acetate, gradient 0% to 40%). The yield was 30 mg (60% of theory) of the title compound.
LC-MS (method A): Rₜ = 4.31 min, m/z = 513.4 (M+H)⁺
¹H-NMR (300 MHz, CDCl₃): δ [ppm] = 1.06 (s, 3H), 1.42 - 1.57 (m, 3H), 1.61 - 1.89 (m, 1H), 1.93 - 2.04 (m, 1H), 2.11 - 2.24 (m, 2H), 2.29 - 2.44 (m, 3H), 2.81 - 2.98 (m, 4H), 3.30 - 3.33 (m, 6H), 3.72 (d, 3H), 6.10 (dd, 1H), 7.09 - 7.17 (m, 2H), 7.27 - 7.32 (m, 1H), 7.36 - 7.43 (m, 1H), 8.30 - 8.36 (m, 1H), 8.48 (dd, 1H).

### Example 37

### methyl 3-{[17-(5-fluoropyridin-3-yl)estra-1(10),2,4,16-tetraen-3-yl]sulfamoyl}propanoate

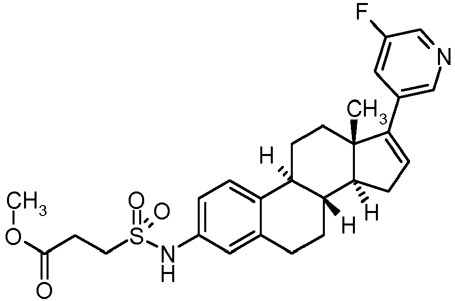

Analogously to the preparation of Example 36, 233 mg (0.67 mmol) of 17-(5-fluoropyridin-3-yl)estra-1(10),2,4,16-tetraen-3-amine (Intermediate 10-A) were converted into 189 mg (54% of theory) of the title compound.
LC-MS (method A): Rₜ = 4.16 min; m/z = 499.4 (M+H)⁺
¹H-NMR (300 MHz, CDCl₃): δ [ppm] = 1.06 (s, 3H), 1.40 - 1.91 (m, 5H), 1.93 - 2.04 (m, 1H), 2.10 - 2.24 (m, 2H), 2.27 - 2.45 (m, 3H), 2.83 - 2.95 (m, 4H), 3.42 (dd, 2H), 3.71 (d, 3H), 6.10 (dd, 1H), 6.38 - 6.73 (m, 1H), 7.01 (d, 2H), 7.21 - 7.26 (m, 1H), 7.37 - 7.43 (m, 1H), 8.35 (d, 1H), 8.48 (dd, 1H).

### Example 38

### 3-{[17-(5-fluoropyridin-3-yl)estra-1(10),2,4,16-tetraen-3-yl](methyl)sulfamoyl}propanoic acid

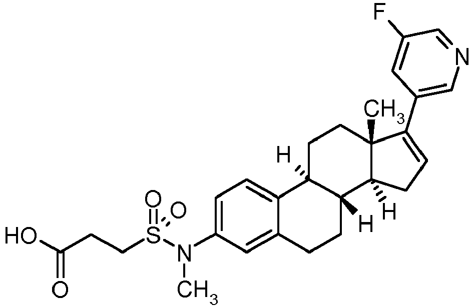

30 mg (0.06 mmol) of methyl 3-{[17-(5-fluoropyridin-3-yl)estra-1(10),2,4,16-tetraen-3-yl](methyl)sulfamoyl}propanoate (Example 36) in 600 µl THF were reacted with 117 µl (0.23 mmol) of 2N lithium hydroxide solution. The mixture was stirred for 16 hours. The reaction mixture was acidified to ∼ pH 4 by addition of 1N hydrochloric acid solution and the reaction mixture was concentrated *in vacuo.* The crude residue was purified by chromatography using a prepacked C18 cartridge (mobile phase: acetonitrile/water 0.1% formic acid buffer, gradient 40% to 98%). The yield was 5.5 mg (18% of theory) of the title compound.
LC-MS (method B): Rₜ = 5.33 min; m/z = 499.2 (M+H)⁺
¹H NMR (400 MHz, CD₃OD): δ [ppm] = 1.08 (s, 3H), 1.44 (s, 3H), 1.46 - 1.57 (m, 1H), 1.66 - 1.74 (m, 3H), 1.80 - 1.89 (m, 1H), 1.97 - 2.05 (m, 1H), 2.15 - 2.24 (m, 2H), 2.32 - 2.50 (m, 3H), 2.64 - 2.72 (m, 2H), 2.90 - 2.97 (m, 2H), 3.35 (t, 2H), 6.21 (dd, 1H), 7.12 - 7.19 (m, 2H), 7.31 (d, 1H), 7.63 (d, 1H), 8.33 - 8.34 (m, 1H), 8.43 - 8.46 (m, 1H)

### Example 39

### 3-{[17-(5-fluoropyridin-3-yl)estra-1(10),2,4,16-tetraen-3-yl]sulfamoyl}propanoic acid

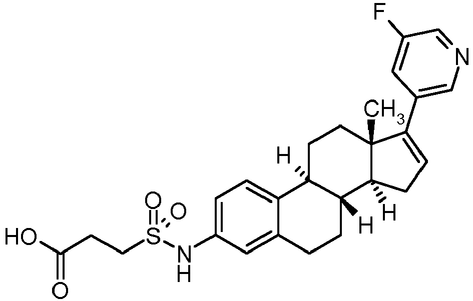

Analogously to the preparation of Example 38, 189 mg (0.38 mmol) of methyl 3-{[17-(5-fluoropyridin-3-yl)estra-1(10),2,4,16-tetraen-3-yl]sulfamoyl}propanoate (Example 37) were reacted with 379 µl (0.75 mmol) of 2N lithium hydroxide solution. The crude residue was purified by preparative HPLC (Method E) to obtain 126 mg (68% of theory) of the title compound.
LC-MS (method B): Rₜ = 4.95 min; m/z = 485.1 (M+H)⁺
¹H-NMR (400 MHz, DMSO-d6): δ [ppm] = 1.00 (s, 3H), 1.37 - 1.77 (m, 6H), 1.84 - 1.92 (m, 1H), 2.07 - 2.17 (m, 1H), 2.20 - 2.39 (m, 3H), 2.61 (dd, 2H,), 2.77 - 2.85 (m, 2H), 3.23 (dd, 2H), 6.27 (dd, 1H), 6.89 - 6.97 (m, 2H), 7.20 (d, 1H), 7.66 - 7.70 (m, 1H), 8.43 - 8.50 (m, 2H), 9.65 (s, 1H), 12.52 (br. s, 1H).

### Examples type IC6

### Example 40

### N-[17-(5-fluoropyridin-3-yl)estra-1(10),2,4,16-tetraen-3-yl]acetamide

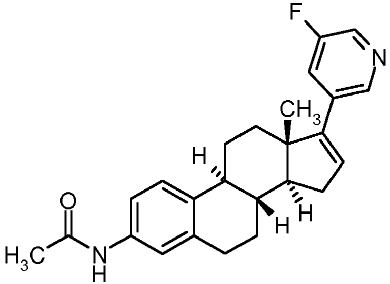

To a solution of 100 mg (0.28 mmol) of 17-(5-fluoropyridin-3-yl)estra-1(10),2,4,16-tetraen-3-amine (Intermediate 10-A) in 1.5 ml of N,N-dimethylformamide under argon at 0°C 80 µl (0.57 mmol) of triethylamine and 21 µl (0.30 mmol) of acetyl chloride were added. The mixture was then allowed to warm to RT and stirred for 1 hour. Water and ethyl acetate were added to the reaction mixture and the organic phase was separated and further washed with brine, dried over sodium sulfate, filtered and concentrated *in vacuo.* The residue was purified by preparative HPLC (Method E). The yield was 52 mg (46% of theory) of the title compound.
LC-MS (method B): Rₜ = 5.18 min; m/z = 391.2 (M+H)⁺
¹H-NMR (400 MHz, DMSO-d₆): δ [ppm] = 1.00 (s, 3H), 1.36 - 1.63 (m, 3H), 1.67 - 1.77 (m, 1H), 1.84 - 1.92 (m, 1H), 1.98 (s, 3H), 2.07 - 2.39 (m, 6H), 2.75 - 2.84 (m, 2H), 6.27 (dd, 1H), 7.15 (d, 1H), 7.24 - 7.30 (m, 2H), 7.66 - 7.70 (m, 1H), 8.43 - 8.50 (m, 2H), 9.72 (s, 1H).

### Examples type IC7

### Example 41

### ethyl N- {[17-(5-fluoropyridin-3-yl)estra-1(10),2,4,16-tetraen-3-yl]carbamoyl}-beta-alaninate

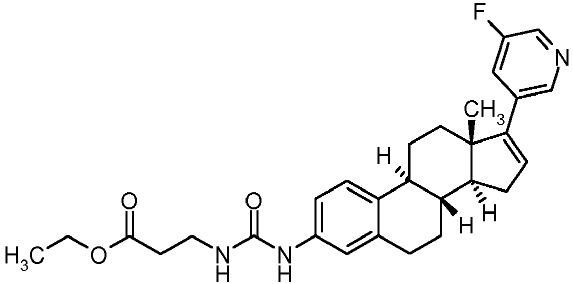

To a solution of 50 mg (0.14 mmol) of 17-(5-fluoropyridin-3-yl)estra-1(10),2,4,16-tetraen-3-amine (Intermediate 10-A) in 2.5 ml of dichloromethane at 0°C under argon 37 mg (0.25 mmol) of ethyl N-(oxomethylidene)-beta-alaninate (CAS No.: 5100-34-5) was added. The reaction mixture was allowed to warm to RT and stirred for 4 hours. It was then concentrated under reduced pressure and the residue was purified using a prepacked silica gel cartridge (mobile phase: cyclohexane/ethyl acetate, gradient 0% to 60%). The yield was 71 mg (96% of theory) of the title compound.
LC-MS (method A): Rₜ = 4.10 min; m/z = 492.4 (M+H)⁺
¹H-NMR (300 MHz, CDCl₃): δ [ppm] = 1.05 (s, 3H), 1.22 - 1.28 (m, 3H), 1.42 - 1.51 (m, 1H), 1.60 - 1.89 (m, 5H), 1.91 - 2.02 (m, 1H), 2.09 - 2.23 (m, 2H), 2.26 - 2.45 (m, 3H), 2.55 - 2.60 (m, 2H), 2.86 - 2.95 (m, 2H), 3.53 (q, 2H), 4.10 - 4.18 (m, 1H), 5.41 (t, 1H), 6.09 (dd, 1H), 6.45 (s, 1H), 6.97 - 7.07 (m, 2H), 7.22 (d, 1H), 7.40 (ddt, 1H), 8.34 (d, 1H), 8.48 (t, 1H).

### Example 42

### N- {[17-(5-fluoropyridin-3-yl)estra-1(10),2,4,16-tetraen-3-yl]carbamoyl}-beta-alanine

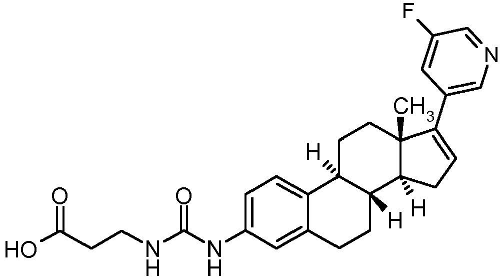

To a solution of 71 mg (0.13 mmol) of ethyl N-{[17-(5-fluoropyridin-3-yl)estra-1(10),2,4,16-tetraen-3-yl]carbamoyl}-beta-alaninate (Example 41) in 780 µl of tetrahydrofuran under argon 137 µl (0.27 mmol) of 2N lithium hydroxide solution was added. The mixture was stirred at RT for 16 hours. The reaction mixture was acidified to pH ∼ 4 by addition of 1N hydrochloric acid solution and the reaction mixture was concentrated *in vacuo.* The crude residue was purified by preparative HPLC (Method E) . The yield was 45 mg (70% of theory) of the title compound.
LC-MS (method B): Rₜ = 4.79 min; m/z = 464.2 (M+H)⁺
¹H-NMR (400MHz, DMSO-d₆): δ [ppm] = 1.00 (s, 3H), 1.35 - 1.61 (m, 4H), 1.67 - 1.76 (m, 1H), 1.83 - 1.91 (m, 1H), 2.10 - 2.39 (m, 9H), 2.73 - 2.82 (m, 2H), 6.12 (dd, 1H), 6.26 (dd, 1H), 7.08 (d, 3H), 7.65 - 7.70 (m, 1H), 8.35 (s, 1H), 8.42 - 8.50 (m, 2H), 12.2 (br. s, 1H).

### Examples type IC8

### Example 43

### methyl 4- {[17-(5-fluoropyridin-3 -yl)estra-1 (1 0),2,4,16-tetraen-3 -yl] (methyl)amino}butanoate

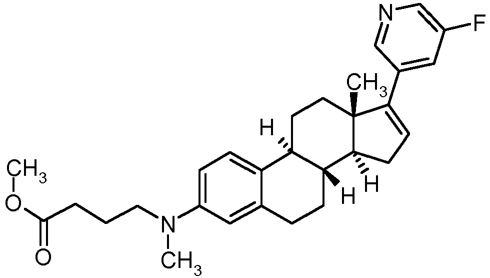

To a solution of 150 mg (0.41 mmol) of 17-(5-fluoropyridin-3-yl)-N-methylestra-1(10),2,4,16-tetraen-3-amine (Intermediate 11-A) in 600 µl of acetonitrile and 300 µl of N,N-dimethylformamide under argon 86 mg (0.62 mmol) of potassium carbonate followed by 82 mg (0.45 mmol) of methyl 4-bromobutanoate (CAS No.: 4897-84-1) were added. The reaction mixture was stirred at 65°C for 16 hours, allowed to cool to RT and partitioned between water and ethyl acetate. The phases were separated, the organic phase was further washed with water, brine, dried over sodium sulfate, filtered and concentrated *in vacuo.* The residue was purified by chromatography using a prepacked silica gel cartridge (mobile phase: dichloromethane/ethyl acetate, gradient 0% to 70%). The yield was 163 mg (81% of theory) of the title compound.
LC-MS (method C): Rₜ = 3.76 min; m/z = 463.1 (M+H)⁺
¹H NMR (300 MHz, CDCl₃): δ [ppm] = 1.04 (s, 3H), 1.45 - 1.98 (m, 7H), 2.09 - 2.20 (m, 3H), 2.32 - 2.43 (m, 6H), 2.81 - 2.96 (m, 4H), 3.32 (t, 2H), 3.68 (s, 3H), 6.07 - 6.11 (m, 1H), 6.45 - 6.48 (m, 1H), 6.57 (dd, 1H), 7.15 (d, 1H), 7.37 - 7.43 (m, 1H), 8.34 (d, 1H), 8.48 (s, 1H).

### Example 44

### 4- {[17-(5-fluoropyridin-3-yl)estra-1(10),2,4,16-tetraen-3-yl](methyl)amino} butanoic acid

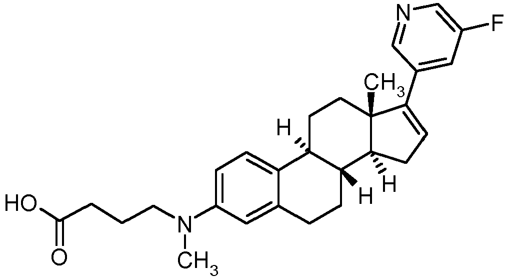

Analogously to the preparation of Example 42, 160 mg (0.34 mmol) of methyl 4-{[17-(5-fluoropyridin-3-yl)estra-1(10),2,4,16-tetraen-3-yl](methyl)amino}butanoate (Example 43) were reacted with 346 µl (0.69 mmol) of 2N lithium hydroxide solution. The crude residue was purified by preparative HPLC (Method E) to obtain 76 mg (48.5% of theory) of the title compound.
LC-MS (method B): Rₜ = 4.34 min; m/z = 449.2 (M+H)⁺
¹H NMR (400 MHz, DMSO-d₆): δ [ppm] = 0.99 (s, 3H), 1.33 - 1.76 (m, 6H), 1.82 - 1.90 (m, 1H), 2.07 - 2.22 (m, 4H), 2.26 - 2.36 (m, 2H), 2.77 - 2.79 (m, 4H), 3.15 - 3.28 (m, 5H), 6.25 - 6.27 (m, 1H), 6.38 (d, 1H), 6.49 (dd, 1H), 7.02 (d, 1H), 7.65 - 7.70 (m, 1H), 8.43 (d, 1H), 8.49 - 8.50 (m, 1H).

### Examples type IC9

### Example 45

### 4-{[17-(6-methylpyridazin-4-yl)estra-1(10),2,4,16-tetraen-3-yl]amino}-4-oxobutanoic acid

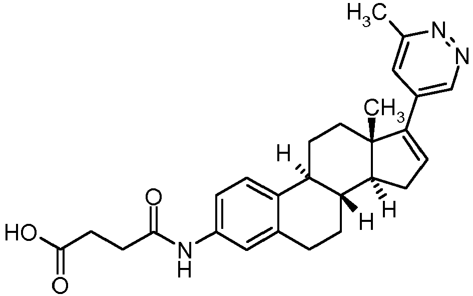

Analogously to the preparation of Example 24, 73 mg (0.14 mmol) of methyl 4-oxo-4-{[17-{[(trifluoromethyl)sulfonyl]oxy}estra-1 (1 0),2,4,16-tetraen-3-yl]amino}butanoate (Intermediate 12-A) were reacted with 3-methyl-5-(4,4,5,5-tetramethyl-1,3,2-dioxaborolan-2-yl)pyridazine (CAS No.: 1350543-95-1) and 7.95 mg (0.01 mmol) of dichlorobis(triphenylphosphine)palladium(II). After 4 hours at 95°C, the reaction mixture was allowed to cool to 50°C and 71 µl of 2N lithium hydroxide solution were added. After 2 hours of stirring the reaction mixture was allowed to cool to room temperature and concentrated *in vacuo.* The residue was partially dissolved in a solution of 5% of methanol in dichloromethane, filtered and purified by chromatography using a prepacked silica gel cartridge (mobile phase: dichloromethane/dichloromethane: methanol: acetic acid: water 240: 20: 3: 2, gradient 0% to 50%). The yield was 21 mg (33% of theory) of the title compound.
LC-MS (method B): Rₜ = 3.57 min, m/z = 446.2 (M+H)⁺
¹H NMR (400 MHz, DMSO-d₆): δ [ppm] = 1.02 (s, 3H), 1.36 - 1.72 (m, 6H), 1.83 - 1.92 (m, 1H), 2.09 - 2.40 (m, 6H), 2.43 - 2.51 (m, 1H), 2.59 (s, 3H), 2.63 - 2.84 (m, 3H), 6.56 (dd, 1H), 7.15 (d, 1H), 7.24 - 7.31 (m, 2H), 7.49 (d, 1H), 9.11 (d, 1H), 9.79 (s, 1H), 12.03 (br. s, 1H).

### Examples type IC10

### Example 46

### 17-(5-fluoropyridin-3-yl)-3-(S-methylsulfonimidoyl)estra-1(10),2,4,16-tetraene

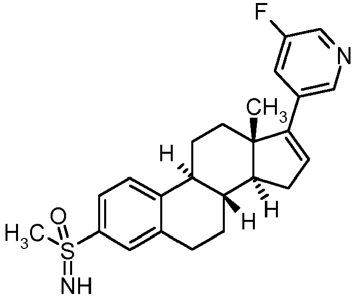

Analogously to the preparation of Example 24, 106 mg (0.161 mmol) of 3-[S-methyl-N-(trifluoroacetyl)sulfonimidoyl]estra-1(10),2,4,16-tetraen-17-yl trifluoromethanesulfonate (Intermediate 15-A) were treated with 31.7 mg (0.22 mmol) of 5-fluoropyridine-3-boronic acid (CAS No.: 872041-86-6) and 9.0 mg (0.013 mmol) of dichlorobis(triphenylphosphine)palladium(II) (CAS No.: 13965-03-2). The residue was purified using a prepacked silica gel cartridge (mobile phase: dichloromethane/ethyl acetate, gradient 0% to 80%) and then by chromatography using a prepacked C18 cartridge (mobile phase: acetonitrile/water 0.1% formic, gradient 10% to 90%). The yield was 22.2 mg (33% of theory) of the title compound.
LC-MS (method B): Rₜ = 4.59 min; m/z = 411.1 (M+H)⁺
¹H NMR (400 MHz, DMSO-d₆): δ [ppm] = 1.01 (s, 3H), 1.45 - 1.77 (m, 6H), 1.89 - 1.96 (m, 1H), 2.09 - 2.20 (m, 2H), 2.29 - 2.43 (m, 3H), 2.94 (dd, 2H), 3.00 (s, 3H), 4.02 (s, 1H), 6.28 (dd, 1H), 7.47 (d, 1H), 7.60 - 7.66 (m, 2H), 7.67 - 7.72 (m, 1H), 8.49 - 8.51 (m, 1H).

### Biological assays:

Example compounds were tested in selected biological assays one or more times. When tested more than once, data are reported as either average values or as median values, wherein
- the average value, also referred to as the arithmetic mean value, represents the sum of the values obtained divided by the number of times tested, and
- the median value represents the middle number of the group of values when ranked in ascending or descending order. If the number of values in the data set is odd, the median is the middle value. If the number of values in the data set is even, the median is the arithmetic mean of the two middle values.

Example compounds were synthesized one or more times. When synthesized more than once, data from biological assays represent average values or median values calculated utilizing data sets obtained from testing of one or more synthetic batch.

### Example 47 (AKR1C3-inhibitory action)

The AKR1C3-inhibitory action of the substances of this invention was measured in the AKR1C3 assay described in the paragraphs which follow.

Essentially, the enzyme activity is measured by quantifying the coumberol formed from coumberone (Halim, M., Yee, D.,J., and Sames, D., J. A., M.. (2008). Chem Soc 130, 14123 - 14128 and Yee, D.,J., Balsanek, V., Bauman, D., R., Penning, T., M., and Sames, D. (2006). Proc Natl Acad Sci USA, 103, 13304 - 13309). In this assay, the increase in the highly fluorescent coumberol can be determined by NADPH (nicotinamide adenine dinucleotide phosphate)-dependent reduction of the nonfluorescent coumberone by AKR1C3.

The enzyme used was recombinant human AKR1C3 (aldo-keto reductase family 1 member C3) (GenBank Accession No. NM_003739). This was expressed as the GST (glutathione S-transferase) fusion protein in *E. coli* and purified by means of glutathione-Sepharose affinity chromatography. The GST was removed by thrombin digestion with subsequent size exclusion chromatography (Dufort, I., Rheault, P., Huang, X., F., Soucy, P., and Luu-The, V. (1999). Endocrinology ,140, 568-574).

For the assay, 40-50 nl of a 100-fold concentrated solution of the test substance in DMSO were pipetted into a black low-volume 384-well microtitre plate (Greiner Bio-One, Frickenhausen, Germany), 2 - 2.5 µl of a solution of AKR1C3 in assay buffer [50 mM potassium phosphate buffer pH 7, 1 mM DTT, 0.0022% (w/v) Pluronic F-127, 0.01% BSA (w/v) and protease inhibitor cocktail (complete, EDTA-free Protease Inhibitor Cocktail from Roche)] were added and the mixture was incubated for 20 min, in order to enable preliminary binding of the substances to the enzyme prior to the enzyme reaction. Then the enzyme reaction was started by adding 2 - 2.5 µl of a solution of NADPH and coumberone in assay buffer (final assay concentration was 10 µM NADPH and 0.3µM coumberone) and the resulting mixture was incubated at room temperature for 3h. Typical concentrations were depending on the linear range of the enzyme reaction during the reaction time about 1 nM. The reaction was stopped by adding 2 ul of a stop solution consisting of the AKR1C3 inhibitor EM-1404 to a final concentration of 1 µM. Subsequently, the fluorescence of coumberol was measured at 520 nm (excitation at 380 nm) with a suitable measuring instrument (Pherastar, BMG). The intensity of the fluorescence was used as a parameter for the amount of coumberol formed and hence for the enzyme activity of AKR1C3. The data were normalized (enzyme + coumberon substrate in assay buffer = 0% inhibition; coumberone substrate only in assay buffer = 100% inhibition). Substances were usually tested at 11 different concentrations on the same microtiter plate in a range between 96.8 pM to 20 µM (20 µM, 5,9 µM, 1,7 µM, 0,5 µM, 0,15 µM, 44 nM, 12,9 nM, 3,8 nM, 1,1 nM, 0,3 nM und 96,8 pM). The dilutions were carried out on a 100fold concentrated solution by serial dilution in duplicates. From the obtained data IC₅₀ values were calculated by a 4-parameter fit.

As described, the pharmacological substances claimed were tested for their inhibitory effect on the AKR1C3 enzyme (see Table 1). The compounds tested show strong inhibition of AKR1C3 *in vitro* (IC₅₀ values < 500 nM) and predominantly even IC₅₀ values < 20 nM.

**Table 1: Inhibition of AKR1C3 by the compounds of the present invention (mean values)**

| Example compound | AKR1C3 enzyme inhibition IC₅₀ [nmol/1] |
|---|---|
| 01 | |
| 02 | 3.5 |
| 03 | 2.4 |
| 04 | |
| 05 | 91.7 |
| 06 | |
| 07 | 0.5 |
| 08 | 4.1 |
| 09 | 7.7 |
| 10 | 2.9 |
| 11 | |
| 12 | 12.0 |
| 13 | |
| 14 | 14.3 |
| 15 | 73.0 |
| 16 | 9.7 |
| 17 | 1.6 |
| 18 | 33.5 |
| 19 | 1.7 |
| 20 | 3.2 |
| 21 | 5.3 |
| 22 | |
| 23 | 3.8 |
| 24 | |
| 25 | 313 |
| 26 | 8.6 |
| 27 | 5.0 |
| 28 | |
| 29 | 3.2 |
| 30 | |
| 31 | 4.1 |
| 32 | 11.8 |
| 33 | 2.5 |
| 34 | |
| 35 | 3.2 |
| 36 | |
| 37 | |
| 38 | 4.9 |
| 39 | 3.0 |
| 40 | 5.3 |
| 41 | |
| 42 | 8.3 |
| 43 | |
| 44 | 18.5 |
| 45 | 1.8 |
| 46 | 2.2 |

### Example 48 (Test of AKR1C3 inhibition in a cell-based system)

The inhibition of AKR1C3 by the substances described in this invention was measured in a cell-based assay using coumberon as the substrate for AKR1C3 (Halim, M., Yee, D.,J., and Sames, D., J. (2008). Am Chem Soc, 130, 14123 - 14128 and Yee, D., J., Balsanek, V., Bauman, D., R., Penning, T., M., and Sames, D. (2006), Proc Natl Acad Sci USA 103, 13304 - 13309) (cf. Example 48).

The cell system used was HEK293 cells (ATCC, USA) (DMEM/F12, 10% FCS, 2mM L-Glutamine, PSG). The cells were transfected with an AKR1C3 expression plasmid (pCMV6-AC-AKR1C3, GenBank Accession No. NM_003739.4), harvested after 24 hours and subsequently frozen. For assays, the frozen cells were thawed in assay medium (DMEM/F12 w/o phenolred, 0.005% Tween, 10% FCS, 2 mM L-Glutamine, PSG). Cells were seeded at 2500 cells/well into 384-well low volume or 1536-well plates (Greiner Bio-One, Frickenhausen, Germany) that contained 40-50nl of a 100fold concentrated compound solution in DMSO. After incubation at room temperature for 15 minutes, coumberon in PBS was added to a final concentration of 6x10⁻⁶M. Following a 3h incubation at room temperature, the reaction was stopped by lysing the cells with 0.4% Triton X-100. Coumberol fluorescence intensity was measured at 520nm (excitation 380nm) with a suitable measuring instrument (Pherastar, BMG). The intensity of the fluorescence was used as a parameter for the amount of coumberol formed and hence for the enzyme activity of AKR1C3. The data were normalized (transfected cells + coumberon = 0% inhibition; no cells + coumberone = 100% inhibition). Substances were usually tested at 11 different concentrations on the same microtiter plate in a range between 96.8pM to 20µM (20 µM, 5,9 µM, 1,7 µM, 0,5 µM, 0,15 µM, 44 nM, 12,9 nM, 3,8 nM, 1,1 nM, 0,3 nM und 96,8 pM). The dilutions were carried out on a 100fold concentrated solution by serial dilution in duplicates. From the obtained data IC₅₀ values were calculated by a 4-parameter fit.

The pharmacological substances claimed were tested for their inhibitory action on the AKR1C3 enzyme by means of the cell-based assay described above (see Table 2). The compounds tested exhibited a significant inhibition of cellular AKR1C3 *in vitro,* most of them show IC50 < 1 µM.

**Table 2: Inhibition of AKR1C3 by the compounds of the present invention in a cellular assay (mean values)**

| Example compound | Cellular AKR1C3 inhibition IC₅₀ [µmol/l] |
|---|---|
| 01 | |
| 02 | 0.38 |
| 03 | 0.05 |
| 04 | |
| 05 | 2.71 |
| 06 | |
| 07 | 0.80 |
| 08 | 0.08 |
| 09 | 0.05 |
| 10 | 0.49 |
| 11 | |
| 12 | 0.70 |
| 13 | |
| 14 | 0.49 |
| 15 | 3.18 |
| 16 | 0.54 |
| 17 | 0.17 |
| 18 | 1.79 |
| 19 | 0.15 |
| 20 | 0.03 |
| 21 | 0.04 |
| 22 | |
| 23 | 0.78 |
| 24 | |
| 25 | 8.34 |
| 26 | 0.15 |
| 27 | 0.11 |
| 28 | |
| 29 | 5.00 |
| 30 | |
| 31 | 1.54 |
| 32 | 2.17 |
| 33 | 0.26 |
| 34 | |
| 35 | 0.21 |
| 36 | |
| 37 | |
| 38 | 0.86 |
| 39 | 5.10 |
| 40 | 0.10 |
| 41 | |
| 42 | 8.09 |
| 43 | |
| 44 | 1.12 |
| 45 | 0.98 |
| 46 | 0.03 |

### Example 49: Determination of antiandrogenic action

The antiandrogenic action of the substance was measured in adult monkeys (Macaca fascicularis), as a surrogate for the antiproliferative effects in prostate cancer and metastases thereof. The monkeys (4 per group) were treated by the oral route by means of a gavage with 1, 3 or 10 mg/kg substance or with vehicle over 4 weeks. The size of the prostate and of the seminal vesicle was determined by ultrasound at the start of the experiment and after one, two, three and four weeks. The decrease in the weight of these organs was taken as evidence for the antiandrogenicity of the substances. In addition, the blood concentrations (in the serum or in the plasma) of various steroids (DHEA, testosterone, androstenedione, hydroxyprogesterone) and prostaglandins (PGD2, PGJ2, PGF2alpha) were determined at the start of the experiment and after one, two, three or four weeks. Since AKR1C3 is involved both in the steroid synthesis route and in the prostaglandin synthesis route, changes in the blood concentrations of these steroids and prostaglandins are taken as an indication of the *in vivo* effect of the substance.

## Claims

1. Compounds of the general formula (I) 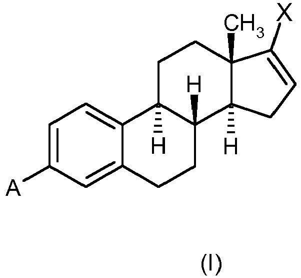 where:
A represents a group selected from: 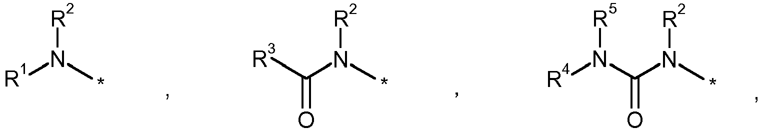 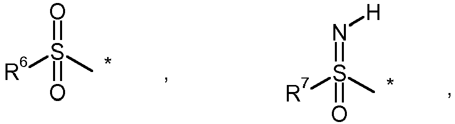 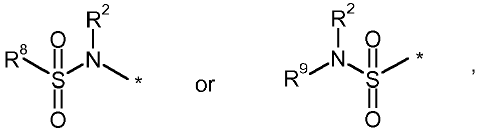 wherein * indicates the point of attachment of said group with the rest of the molecule;
X is a group selected from: 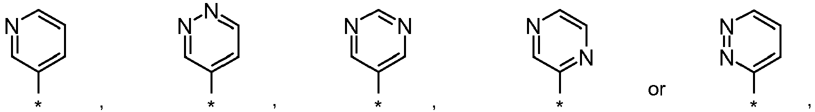 wherein * indicates the point of attachment of the group with the rest of the molecule and where the group is optionally substituted, one or more times, independently from each other, with a substituent selected from halogen, CN, OH, RR²N-, C₁-C₆-alkyl, C₁-C₆-haloalkyl, C₁-C₆-alkoxy, C₁-C₆-haloalkoxy, wherein C₁-C₆-alkyl and C₁-C₆-haloalkyl groups are optionally substituted with OH;
R¹ is C₁-C₆-alkyl or C₁-C₆-haloalkyl, and where R¹ is optionally substituted with one or two substituents, independently from each other, selected from OH, CN, RO(CO)-, RR²N(CO)-, RR²NSO₂-, C₁-C₆-alkyl-(CO)(NH)SO₂- or 5-tetrazolyl;
R², R⁵ are, independently from each other, hydrogen or C₁-C₆-alkyl, where C₁-C₆-alkyl groups are optionally substituted, one or more times, independently from each other, with halogen,
R³, R⁴ are, independently from each other, C₁-C₆-alkyl or C₁-C₆-haloalkyl, and whereby
R³ and R⁴ are optionally substituted with one or two substituents, independently from each other, selected from OH, CN, RO(CO)-, RR²N(CO)-, RR²NSO₂-, C₁-C₆-alkyl-(CO)(NH)SO₂- or 5-tetrazolyl;
R⁶, R⁷, R⁸, R⁹ are, independently from each other, C₁-C₆-alkyl or C₁-C₆-haloalkyl, and whereby
R⁶, R⁷, R⁸ and R⁹ are optionally substituted with one or two substituents, independently from each other, selected from OH, CN, RO(CO)-, RR²N(CO)-, RR²NSO₂-, C₁-C₆-alkyl-(CO)(NH)SO₂- or 5-tetrazolyl;
R is hydrogen or a C₁-C₆-alkyl group;
or the tautomers, N-oxides, hydrates, solvates or salts thereof, or a mixture consisting of the above.

2. Compounds of the general formula (I) according to Claim 1,
where
A represents a group selected from: 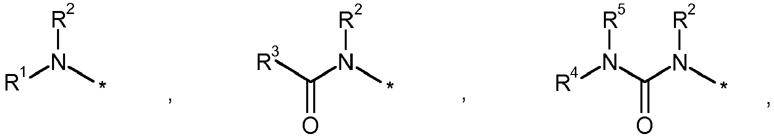 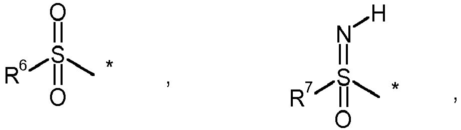 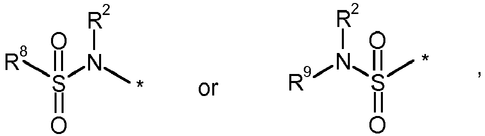 wherein * indicates the point of attachment of said group with the rest of the molecule;
X is a group selected from: 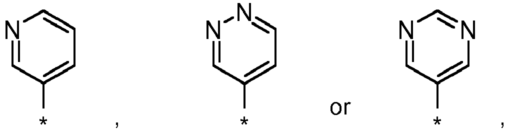 wherein * indicates the point of attachment of the group with the rest of the molecule and where the group is optionally substituted, one or more times, independently from each other, with a substituent selected from halogen, C₁-C₄-alkyl, C₁-C₄-haloalkyl, C₁-C₄-alkoxy, wherein C₁-C₄-alkyl and C₁-C₄-haloalkyl groups are optionally substituted with OH;
R¹ is C₁-C₄-alkyl, which is optionally substituted with one or two substituents, independently from each other, selected from OH, RO(CO)-, RR²N(CO)-, RR²NSO₂- or 5-tetrazolyl;
R², R⁵ are, independently from each other, hydrogen or C₁-C₄-alkyl, where C₁-C₄-alkyl groups are optionally substituted, one or more times, independently from each other, with halogen;
R³, R⁴ are, independently from each other, C₁-C₄-alkyl, and whereby R³ and R⁴ are optionally substituted with one or two substituents, independently from each other, selected from OH, RO(CO)-, RR²N(CO)-, RR²NSO₂-, C₁-C₄-alkyl-(CO)(NH)SO₂- or 5-tetrazolyl;
R⁶, R⁷, R⁸, R⁹ are, independently from each other, C₁-C₄-alkyl, and whereby R⁶, R⁷, R⁸ and R⁹ are optionally substituted with one or two substituents, independently from each other, selected from OH, RO(CO)-, RR²N(CO)-, RR²NSO₂- or 5-tetrazolyl;
R is hydrogen or a C₁-C₄-alkyl group;
or a tautomer, an N-oxide, a hydrate, a solvate, or a salt thereof, or a mixture consisting of the above.

3. Compounds of the general formula (I) according to Claims 1-2,
where
A represents a group selected from: 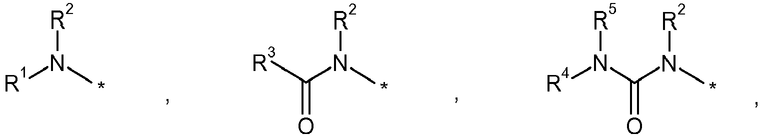 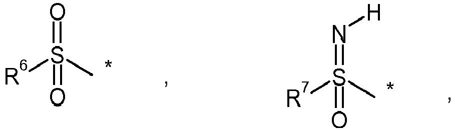 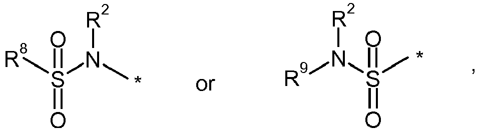 wherein * indicates the point of attachment of said group with the rest of the molecule;
X is a group selected from: 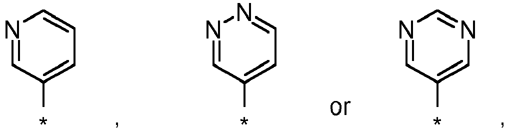 wherein * indicates the point of attachment of the group with the rest of the molecule and where the group is optionally substituted, one or two times, independently from each other, with a substituent selected from fluoro, chloro, methyl, trifluoromethyl or methoxy;
R¹ is propyl, which is optionally substituted with RO(CO)-;
R², R⁵ are, independently from each other, hydrogen or methyl;
R³, R⁴ are, independently from each other, C₁-C₃-alkyl, and whereby
R³ and R⁴ are optionally substituted with one or two substituents, independently from each other, selected from OH, RO(CO)-, RR²N(CO)-, RR²NSO₂-, C₁-C₂-alkyl-(CO)(NH)SO₂- or
5-tetrazolyl;
R⁶, R⁷, R⁸, R⁹ are, independently from each other, C₁-C₄-alkyl, and whereby
R⁶, R⁷, R⁸ and R⁹ are optionally substituted with one or two substituents, independently from each other, selected from OH, RO(CO)- or RR²N(CO)-;
R is hydrogen or a C₁-C₄-alkyl group;
or a tautomer, an N-oxide, a hydrate, a solvate, or a salt thereof, or a mixture consisting of the above.

4. Compounds of the general formula (I) according to Claims 1, 2 or 3, where
A represents a group selected from: 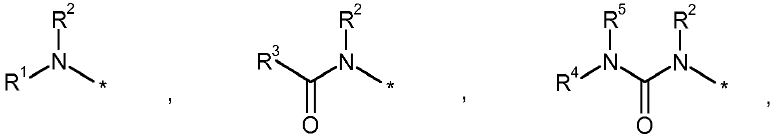 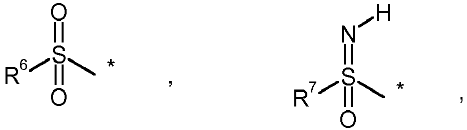 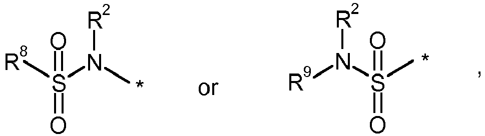 wherein * indicates the point of attachment of said group with the rest of the molecule;
X is a group selected from: 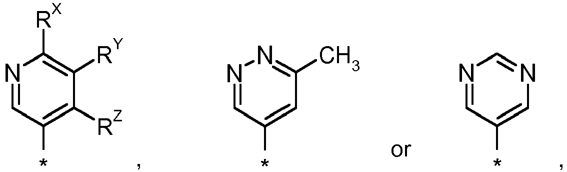 wherein * indicates the point of attachment of the group with the rest of the molecule and,
R^{X} is hydrogen or methyl,
R^{Y} is hydrogen, fluoro, chloro, methyl, trifluoromethyl or methoxy,
R^{Z} is hydrogen or methyl,
wherein only one of R^{X}, R^{Y} and R^{Z} is different from hydrogen and;
R¹ is -CH₂-CH₂-CH₂-COOH;
R² is hydrogen or methyl;
R³ is C₁-C₃-alkyl, which is optionally substituted with one substituent, selected from OH, HO(CO)-, H₂N(CO)-, CH₃-(CO)(NH)SO₂- or 5-tetrazolyl;
R⁴ is -CH₂-CH₂-COOH;
R⁵ is hydrogen;
R⁶ is C₁-C₄-alkyl, which is optionally substituted with one substituent, selected from OH, RO(CO)- or H₂N(CO)-;
R⁷ is methyl;
R⁸ is -CH₂-CH₂-COOH;
R⁹ is C₂-C₃-alkyl, which is substituted with one substituent, selected from OH or HO(CO)-;
R is hydrogen or methyl;
or a tautomer, an N-oxide, a hydrate, a solvate, or a salt thereof, or a mixture consisting of the above.

5. Compounds according to any of Claims 1, 2 or 3, selected from a group comprising the following compounds:
• tert-butyl N-{[17-(5-fluoropyridin-3-yl)estra-1(10),2,4,16-tetraen-3-yl]sulfonyl}-N-methyl-beta-alaninate
• N-{[17-(5-fluoropyridin-3-yl)estra-1(10),2,4,16-tetraen-3-yl]sulfonyl}-N-methyl-beta-alanine
• 17-(5-fluoropyridin-3-yl)-N-(3-hydroxypropyl)-N-methylestra-1(10),2,4,16-tetraene-3-sulfonamide
• tert-butyl N-methyl-N-{[17-(pyrimidin-5-yl)estra-1(10),2,4,16-tetraen-3-yl]sulfonyl}-beta-alaninate
• N-methyl-N-{[17-(pyrimidin-5-yl)estra-1(10),2,4,16-tetraen-3-yl]sulfonyl}-beta-alanine
• tert-butyl N-methyl-N-{[17-(6-methylpyridazin-4-yl)estra-1(10),2,4,16-tetraen-3-yl]sufonyl}-beta-alaninate
• N-methyl-N-{[17-(6-methylpyridazin-4-yl)estra-1(10),2,4,16-tetraen-3-yl]sulfonyl}-beta-alanine
• 17-(5-fluoropyridin-3-yl)-3-(methylsulfonyl)estra-1(10),2,4,16-tetraene
• methyl 4-{[17-(5-fluoropyridin-3-yl)estra-1(10),2,4,16-tetraen-3-yl]sulfonyl}butanoate
• 4-{[17-(5-fluoropyridin-3-yl)estra-1(10),2,4,16-tetraen-3-yl]sulfonyl}butanoic acid
• methyl 4-{[17-(pyridin-3-yl)estra-1(10),2,4,16-tetraen-3-yl]sulfonyl}butanoate
• 4-{[17-(pyridin-3-yl)estra-1(10),2,4,16-tetraen-3-yl]sulfonyl}butanoic acid
• methyl 4-({17-[5-(trifluoromethyl)pyridin-3-yl]estra-1(10),2,4,16-tetraen-3-yl} sulfonyl) butanoate
• 4-({17-[5-(trifluoromethyl)pyridin-3-yl]estra-1(10),2,4,16-tetraen-3-yl}sulfonyl)butanoic acid
• 4-{[17-(6-methylpyridin-3-yl)estra-1,3,5(10),16-tetraen-3-yl]sulfonyl}butanoic acid
• 4-{[17-(5-methoxypyridin-3-yl)estra-1,3,5(10),16-tetraen-3-yl]sulfonyl}butanoic acid
• 4-{[17-(5-methylpyridin-3-yl)estra-1,3,5(10),16-tetraen-3-yl]sulfonyl}butanoic acid
• 4-{[17-(4-methylpyridin-3-yl)estra-1,3,5(10),16-tetraen-3-yl]sulfonyl}butanoic acid
• 4-{[17-(5-chloropyridin-3-yl)estra-1,3,5(10),16-tetraen-3-yl]sulfonyl}butanoic acid
• 4-{[17-(5-fluoropyridin-3-yl)estra-1(10),2,4,16-tetraen-3-yl]sulfonyl}butanamide
• 4-{[17-(5-fluoropyridin-3-yl)estra-1(10),2,4,16-tetraen-3-yl]sulfonyl}butan-1-ol
• tert-butyl 4-{[17-(5-fluoropyridin-3-yl)estra-1(10),2,4,16-tetraen-3-yl]amino}-4-oxobutanoate
• 4-{[17-(5-fluoropyridin-3-yl)estra-1(10),2,4,16-tetraen-3-yl]amino}-4-oxobutanoic acid
• tert-butyl 4-oxo-4-{[17-(pyrimidin-5-yl)estra-1(10),2,4,16-tetraen-3-yl]amino}butanoate
• 4-oxo-4-{[17-(pyrimidin-5-yl)estra-1(10),2,4,16-tetraen-3-yl]amino}butanoic acid
• N-[17-(5-fluoropyridin-3-yl)estra-1(10),2,4,16-tetraen-3-yl]butanediamide
• N-[17-(5-fluoropyridin-3-yl)estra-1(10),2,4,16-tetraen-3-yl]-4-hydroxybutanamide
• N-[17-(5-fluoropyridin-3-yl)estra-1(10),2,4,16-tetraen-3-yl]-3-sulfamoylpropanamide
• 3-(acetylsulfamoyl)-N-[17-(5-fluoropyridin-3-yl)estra-1(10),2,4,16-tetraen-3-yl]propanamide
• N-[17-(5-fluoropyridin-3-yl)estra-1(10),2,4,16-tetraen-3-yl]-N-methyl-3-sulfamoylpropanamide
• 3-(acetylsulfamoyl)-N-[17-(5-fluoropyridin-3-yl)estra-1(10),2,4,16-tetraen-3-yl]-N-methyl propanamide
• N-[17-(5-fluoropyridin-3-yl)estra-1(10),2,4,16-tetraen-3-yl]-3-(2H-tetrazol-5-yl)propanamide
• N-[17-(5-fluoropyridin-3-yl)estra-1(10),2,4,16-tetraen-3-yl]-N-methyl-3-(2H-tetrazol-5-yl) propanamide
• tert-butyl 4-{[17-(5-fluoropyridin-3-yl)estra-1(10),2,4,16-tetraen-3-yl](methyl)amino}-4-oxobutanoate
• 4-{[17-(5-fluoropyridin-3-yl)estra-1(10),2,4,16-tetraen-3-yl](methyl)amino}-4-oxobutanoic acid
• methyl 3-{[17-(5-fluoropyridin-3-yl)estra-1(10),2,4,16-tetraen-3-yl](methyl)sulfamoyl} propanoate
• methyl 3-{[17-(5-fluoropyridin-3-yl)estra-1(10),2,4,16-tetraen-3-yl]sulfamoyl}propanoate
• 3-{[17-(5-fluoropyridin-3-yl)estra-1(10),2,4,16-tetraen-3-yl](methyl)sulfamoyl}propanoic acid
• 3-{[17-(5-fluoropyridin-3-yl)estra-1(10),2,4,16-tetraen-3-yl]sulfamoyl}propanoic acid
• N-[17-(5-fluoropyridin-3-yl)estra-1(10),2,4,16-tetraen-3-yl]acetamide
• ethyl N-{[17-(5-fluoropyridin-3-yl)estra-1(10),2,4,16-tetraen-3-yl]carbamoyl}-beta-alaninate
• N-{[17-(5-fluoropyridin-3-yl)estra-1(10),2,4,16-tetraen-3-yl]carbamoyl}-beta-alanine
• methyl 4-{[17-(5-fluoropyridin-3-yl)estra-1(10),2,4,16-tetraen-3-yl](methyl)amino}butanoate
• 4-{[17-(5-fluoropyridin-3-yl)estra-1(10),2,4,16-tetraen-3-yl](methyl)amino}butanoic acid
• 4-{[17-(6-methylpyridazin-4-yl)estra-1(10),2,4,16-tetraen-3-yl]amino}-4-oxobutanoic acid
• 17-(5-fluoropyridin-3-yl)-3-(S-methylsulfonimidoyl)estra-1(10),2,4,16-tetraene and the tautomers, N-oxides, hydrates, solvates or salts thereof, or a mixture consisting of the above.

6. Compound of the formula (I) as defined in any of Claims 1, 2, 3, 4 or 5 for treatment and/or prophylaxis of diseases.

7. Compound of the formula (I) as defined in any of Claims 1, 2, 3, 4 or 5 for use in a method for treatment and/or prophylaxis of endometriosis, of leiomyoma, of uterine bleeding disorders, of dysmenorrhoea, of prostate carcinoma, of prostate hyperplasia, of acne, of seborrhoea, of hair loss, of premature sexual maturity, of polycystic ovary syndrome, of breast cancer, of lung cancer, of endometrial carcinoma, of renal cell carcinoma, of bladder carcinoma, of non-Hodgkins lymphoma, of chronic obstructive pulmonary disease (COPD), of obesity, or of inflammation-related pain.

8. Use of a compound according to any of Claims 1, 2, 3, 4 or 5 for production of a medicament for treatment and/or prophylaxis of diseases.

9. Use of a compound as defined in any of Claims 1, 2, 3, 4 or 5 for production of a medicament for prophylaxis of endometriosis, of leiomyoma, of uterine bleeding disorders, of dysmenorrhoea, of prostate carcinoma, of prostate hyperplasia, of acne, of seborrhoea, of hair loss, of premature sexual maturity, of polycystic ovary syndrome, of breast cancer, of lung cancer, of endometrial carcinoma, of renal cell carcinoma, of bladder carcinoma, of non-Hodgkins lymphoma, of chronic obstructive pulmonary disease (COPD), of obesity or of inflammation-related pain.

10. Medicament comprising a compound as defined in any of Claims 1, 2, 3, 4 or 5 in combination with one or more further active ingredients, especially with selective oestrogen receptor modulators (SERMs), oestrogen receptor (ER) antagonists, aromatase inhibitors, 17-HSD1 inhibitors, steroid sulphatase (STS) inhibitors, GnRH agonists and antagonists, kisspeptin receptor (KISSR) antagonists, selective androgen receptor modulators (SARMs), androgens, 5-reductase inhibitors, C(17,20)-lyase inhibitors, selective progesterone receptor modulators (SPRMs), gestagens, antigestagens, oral contraceptives, inhibitors of mitogen-activated protein (MAP) kinases and inhibitors of the MAP kinases (Mkk3/6, Mekl/2, Erkl/2), inhibitors of the protein kinases B (PKBα/β/γ; Aktl/2/3), inhibitors of the phosphoinositide 3-kinases (PI3K), inhibitors of cyclin-dependent kinase (CDK1/2), inhibitors of the hypoxia-induced signalling pathway (HIF1 alpha inhibitors, activators of prolylhydroxylases), histone deacetylase (HDAC) inhibitors, prostaglandin F receptor (FP) (PTGFR) antagonists and non-steroidal inflammation inhibitors (NSAIDs).

11. Medicament comprising a compound of the formula (I) as defined in any of Claims 1, 2, 3, 4 or 5 in combination with an inert, nontoxic, pharmaceutically suitable excipient.

12. Medicament according to Claim 10 or 11 for treatment and prophylaxis of endometriosis, of uterine leiomyoma, of uterine bleeding disorders, of dysmenorrhoea, of prostate carcinoma, of prostate hyperplasia, of acne, of seborrhoea, of hair loss, of premature sexual maturity, of polycystic ovary syndrome, of breast cancer, of lung cancer, of endometrial carcinoma, of renal cell carcinoma, of bladder carcinoma, of non-Hodgkins lymphoma, of chronic obstructive pulmonary disease (COPD), of obesity or of inflammation-related pain.

## Patentansprüche

1. Verbindungen der allgemeinen Formel (I) 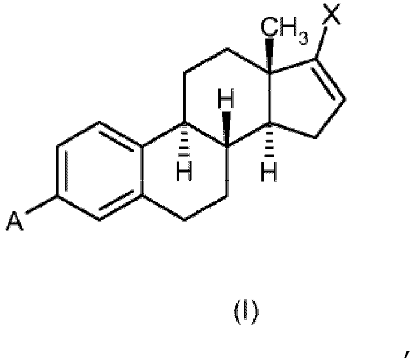 wobei:
A für eine Gruppe ausgewählt aus: 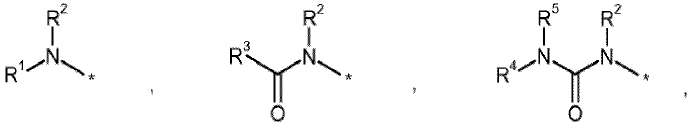 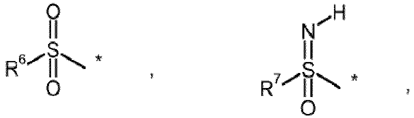 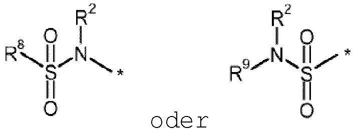 steht,
wobei * den Anbindungspunkt der Gruppe an den Rest des Moleküls kennzeichnet,
X für eine Gruppe ausgewählt aus: 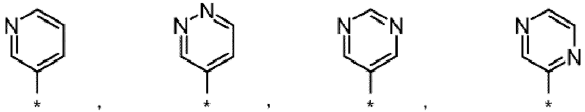 oder 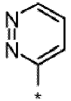 steht,
wobei * den Anbindungspunkt der Gruppe an den Rest des Moleküls kennzeichnet und wobei die Gruppe gegebenenfalls ein- oder mehrfach unabhängig voneinander durch einen Substituenten ausgewählt aus Halogen, CN, OH, RR²N-, C₁-C₆-Alkyl, C₁- C₆-Halogenalkyl, C₁-C₆-Alkoxy, C₁-C₆- Halogenalkoxy substituiert ist, wobei C₁- C₆-Alkyl- und C₁-C₆-Halogenalkylgruppen gegebenenfalls durch OH substituiert sind,
R¹ für C₁-C₆-Alkyl oder C₁-C₆-Halogenalkyl steht und wobei R¹ gegebenenfalls durch einen oder zwei unabhängig voneinander aus OH, CN, RO(CO)-, RR²N(CO)-, RR²NSO₂-, C₁-C₆-Alkyl-(CO)(NH)SO₂- und 5-Tetrazolyl ausgewählte Substituenten substituiert ist,
R², R⁵ unabhängig voneinander für Wasserstoff oder C₁-C₆-Alkyl stehen, wobei C₁-C₆- Alkylgruppen gegebenenfalls ein- oder mehrfach unabhängig voneinander durch Halogen substituiert sind,
R³, R⁴ unabhängig voneinander für C₁-C₆-Alkyl oder C₁-C₆-Halogenalkyl stehen und wobei R³ und R⁴ gegebenenfalls durch einen oder zwei unabhängig voneinander aus OH, CN, RO(CO)-, RR²N(CO)-, RR²NSO₂-, C₁-C₆-Alkyl- (CO)(NH)SO₂- und 5-Tetrazolyl ausgewählte Substituenten substituiert sind,
R⁶, R⁷, R⁸, R⁹ unabhängig voneinander für C₁-C₆-Alkyl oder C₁-C₆-Halogenalkyl stehen und wobei R⁶, R⁷, R⁸ und R⁹ gegebenenfalls durch einen oder zwei unabhängig voneinander aus OH, CN, RO(CO)-, RR²N(CO)-, RR²NSO₂-, C₁-C₆-Alkyl-(CO)(NH)SO₂ und 5-Tetrazolyl ausgewählte Substituenten substituiert sind,
R für Wasserstoff oder eine C₁-C₆- Alkylgruppe steht,
oder die Tautomere, N-Oxide, Hydrate, Solvate oder Salze davon oder eine aus den obigen bestehende Mischung.

2. Verbindungen der allgemeinen Formel (I) nach Anspruch 1,
wobei
A für eine Gruppe ausgewählt aus: 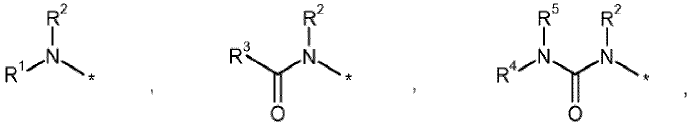 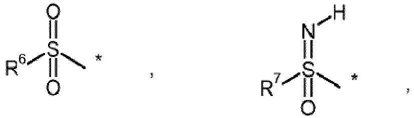 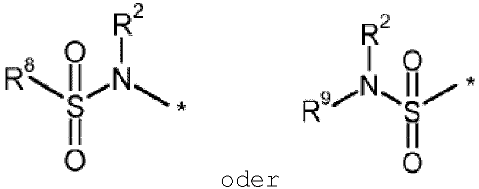 steht,
wobei * den Anbindungspunkt der Gruppe an den Rest des Moleküls kennzeichnet,
X für eine Gruppe ausgewählt aus: 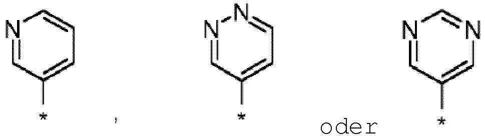 steht,
wobei * den Anbindungspunkt der Gruppe an den Rest des Moleküls kennzeichnet und wobei die Gruppe gegebenenfalls ein- oder mehrfach unabhängig voneinander durch einen Substituenten ausgewählt aus Halogen, C₁-C₄-Alkyl, C₁-C₄-Halogenalkyl, C₁-C₄-Alkoxy substituiert ist, wobei C₁- C₄-Alkyl- und C₁-C₄-Halogenalkylgruppen gegebenenfalls durch OH substituiert sind,
R¹ für C₁-C₄-Alkyl steht, welches gegebenenfalls durch einen oder zwei unabhängig voneinander aus OH, RO(CO)-, RR²N(CO)-, RR²NSO₂- und 5- Tetrazolyl ausgewählte Substituenten substituiert ist,
R², R⁵ unabhängig voneinander für Wasserstoff oder C₁-C₄-Alkyl stehen, wobei C₁-C₄- Alkylgruppen gegebenenfalls ein- oder mehrfach unabhängig voneinander durch Halogen substituiert sind,
R³, R⁴ unabhängig voneinander für C₁-C₄-Alkyl stehen und wobei R³ und R⁴ gegebenenfalls durch einen oder zwei unabhängig voneinander aus OH, RO(CO)-, RR²N(CO)-, RR²NSO₂-, C₁-C₄-Alkyl- (CO) (NH)SO₂- und 5-Tetrazolyl ausgewählte Substituenten substituiert sind,
R⁶, R⁷, R⁸, R⁹ unabhängig voneinander für C₁-C₄-Alkyl stehen und wobei R⁶, R⁷, R⁸ und R⁹ gegebenenfalls durch einen oder zwei unabhängig voneinander aus OH, RO(CO)-, RR²N(CO)-, RR²NSO₂- und 5-Tetrazolyl ausgewählte Substituenten substituiert sind,
R für Wasserstoff oder eine C₁-C₄- Alkylgruppe steht,
oder ein Tautomer, N-Oxid, Hydrat, Solvat oder Salz davon oder eine aus den obigen bestehende Mischung.

3. Verbindungen der allgemeinen Formel (I) nach den Ansprüchen 1-2,
wobei
A für eine Gruppe ausgewählt aus: 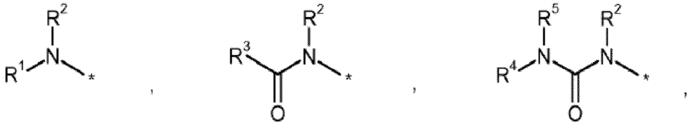 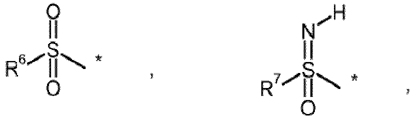 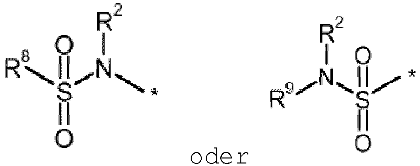 steht,
wobei * den Anbindungspunkt der Gruppe an den Rest des Moleküls kennzeichnet,
X für eine Gruppe ausgewählt aus: 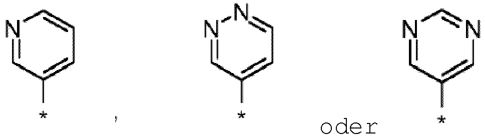 steht,
wobei * den Anbindungspunkt der Gruppe an den Rest des Moleküls kennzeichnet und wobei die Gruppe gegebenenfalls ein- oder mehrfach unabhängig voneinander durch einen Substituenten ausgewählt aus Fluor, Chlor, Methyl, Trifluormethyl und Methoxy substituiert ist,
R¹ für Propyl steht, welches gegebenenfalls durch RO(CO)- substituiert ist,
R², R⁵ unabhängig voneinander für Wasserstoff oder Methyl stehen,
R³, R⁴ unabhängig voneinander für C₁-C₃-Alkyl stehen und wobei R³ und R⁴ gegebenenfalls durch einen oder zwei unabhängig voneinander aus OH, RO(CO)-, RR²N(CO)-, RR²NSO₂-, C₁-C₂-Alkyl- (CO)(NH)SO₂- und 5-Tetrazolyl ausgewählte Substituenten substituiert sind,
R⁶, R⁷, R⁸, R⁹ unabhängig voneinander für C₁-C₄-Alkyl stehen und wobei R⁶, R⁷, R⁸ und R⁹ gegebenenfalls durch einen oder zwei unabhängig voneinander aus OH, RO(CO)- und RR²N(CO)- ausgewählte Substituenten substituiert sind,
R für Wasserstoff oder eine C₁-C₄- Alkylgruppe steht,
oder ein Tautomer, ein N-Oxid, ein Hydrat, ein Solvat oder ein Salz davon, oder eine aus den obigen bestehende Mischung.

4. Verbindungen der allgemeinen Formel (I) nach Anspruch 1, 2 oder 3,
wobei
A für eine Gruppe ausgewählt aus: 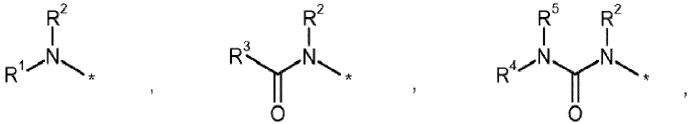 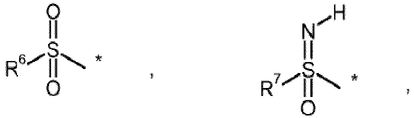 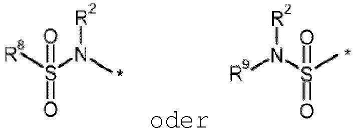 steht,
wobei * den Anbindungspunkt der Gruppe an den Rest des Moleküls kennzeichnet,
X für eine Gruppe ausgewählt aus: 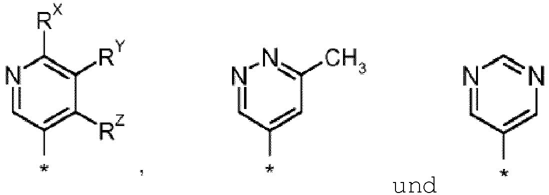 steht,
wobei * den Anbindungspunkt der Gruppe an den Rest des Moleküls kennzeichnet, und
R^{X} für Wasserstoff oder Methyl steht,
R^{Y} für Wasserstoff, Fluor, Chlor, Methyl, Trifluormethyl oder Methoxy steht,
R^{Z} für Wasserstoff oder Methyl steht, wobei nur einer der Reste R^{X}, R^{Y} und R^{Z} von Wasserstoff verschieden ist, und
R¹ für -CH₂-CH₂-CH₂-COOH steht,
R² für Wasserstoff oder Methyl steht,
R³ für C₁-C₃-Alkyl steht, welches gegebenenfalls durch einen aus OH, HO(CO)-, H₂N(CO)-, CH₃-(CO)(NH)SO₂- und 5-Tetrazolyl ausgewählten Substituenten substituiert ist,
R⁴ für -CH₂-CH₂-COOH steht,
R⁵ für Wasserstoff steht,
R⁶ für C₁-C₄-Alkyl steht, welches gegebenenfalls durch einen aus OH, RO(CO)- und H₂N(CO)- ausgewählten Substituenten substituiert ist,
R⁷ für Methyl steht,
R⁸ für -CH₂-CH₂-COOH steht,
R⁹ für C₂-C₃-Alkyl steht, welches durch einen aus OH und HO(CO)- ausgewählten Substituenten substituiert ist,
R für Wasserstoff oder Methyl steht,
oder ein Tautomer, ein N-Oxid, ein Hydrat, ein Solvat oder ein Salz davon, oder eine aus den obigen bestehende Mischung.

5. Verbindungen nach einem der Ansprüche 1, 2 oder 3, ausgewählt aus einer die folgenden Verbindungen umfassenden Gruppe:
• N-{[17-(5-Fluorpyridin-3-yl)estra-1(10),2,4,16-tetraen-3-yl]sulfonyl}-N-methylbetaalanin-tert.-butylester
• N-{[17-(5-Fluorpyridin-3-yl)estra-1(10),2,4,16-tetraen-3-yl]sulfonyl}-N-methylbetaalanin
• 17-(5-Fluorpyridin-3-yl)-N-(3-hydroxypropyl)-N-methylestra-1(10),2,4,16-tetraen-3-sulfonamid
• N-Methyl-N-{[17-(pyrimidin-5-yl)estra-1(10),2,4,16-tetraen-3-yl]sulfonyl}betaalanin-tert.-butylester
• N-Methyl-N-{[17-(pyrimidin-5-yl)estra-1(10),2,4,16-tetraen-3-yl]sulfonyl}betaalanin
• N-Methyl-N-{[17-(6-methylpyridazin-4-yl)estra-1(10),2,4,16-tetraen-3-yl]sulfonyl}betaalanin-tert.-butylester
• N-Methyl-N-{[17-(6-methylpyridazin-4-yl)estra-1(10),2,4,16-tetraen-3-yl]sulfonyl}betaalanin
• 17-(5-Fluorpyridin-3-yl)-3-(methylsulfonyl)estra-1(10),2,4,16-tetraen
• 4-{[17-(5-Fluorpyridin-3-yl)estra-1(10),2,4,16-tetraen-3-yl]sulfonyl}butansäuremethylester
• 4-{[17-(5-Fluorpyridin-3-yl)estra-1(10),2,4,16-tetraen-3-yl]sulfonyl}butansäure
• 4-{[17-(Pyridin-3-yl)estra-1(10),2,4,16-tetraen-3-yl]sulfonyl}butansäuremethylester
• 4-{[17-(Pyridin-3-yl)estra-1(10),2,4,16-tetraen-3-yl]sulfonyl}butansäure
• 4-({17-[5-Trifluormethyl)pyridin-3-yl]estra-1(10),2,4,16-tetraen-3-yl}sulfonyl)butansäuremethylester
• 4-({17-[5-(Trifluormethyl)pyridin-3-yl]estra-1(10),2,4,16-tetraen-3-yl}sulfonyl)butansäure
• 4-{[17-(6-Methylpyridin-3-yl)estra-1,3,5(10),16-tetraen-3-yl]sulfonyl}butansäure
• 4-{[17-(5-Methoxypyridin-3-yl)estra-1,3,5(10),16-tetraen-3-yl]sulfonyl}butansäure
• 4-{[17-(5-Methylpyridin-3-yl)estra-1,3,5(10),16-tetraen-3-yl]sulfonyl}butansäure
• 4-{[17-(4-Methylpyridin-3-yl)estra-1,3,5(10),16-tetraen-3-yl]sulfonyl}butansäure
• 4-{[17-(5-Chlorpyridin-3-yl)estra-1,3,5(10),16-tetraen-3-yl]sulfonyl}butansäure
• 4-{[17-(5-Fluorpyridin-3-yl)estra-1(10),2,4,16-tetraen-3-yl]sulfonyl}butanamid
• 4-{[17-(5-Fluorpyridin-3-yl)estra-1(10),2,4,16-tetraen-3-yl]sulfonyl}butan-1-ol
• 4-{[17-(5-Fluorpyridin-3-yl)estra-1(10),2,4,16-tetraen-3-yl]amino}-4-oxobutansäure-tert.-butylester
• 4-{[17-(5-Fluorpyridin-3-yl)estra-1(10),2,4,16-tetraen-3-yl]amino}-4-oxobutansäure
• 4-Oxo-4-{[17-(pyrimidin-5-yl)estra-1(10),2,4,16-tetraen-3-yl]amino}butansäure-tert.-butylester
• 4-Oxo-4-{[17-(pyrimidin-5-yl)estra-1(10),2,4,16-tetraen-3-yl]amino}butansäure
• N-[17-(5-Fluorpyridin-3-yl)estra-1(10),2,4,16-tetraen-3-yl]butandiamid
• N-[17-(5-Fluorpyridin-3-yl)estra-1(10),2,4,16-tetraen-3-yl]-4-hydroxybutanamid
• N-[17-(5-Fluorpyridin-3-yl)estra-1(10),2,4,16-tetraen-3-yl]-3-sulfamoylpropanamid
• 3-(Acetylsulfamoyl)-N-[17-(5-fluorpyridin-3-yl)estra-1(10),2,4,16-tetraen-3-yl]propanamid
• N-[17-(5-Fluorpyridin-3-yl)estra-1(10),2,4,16-tetraen-3-yl]-N-methyl-3-sulfamoylpropanamid
• 3-(Acetylsulfamoyl)-N-[17-(5-fluorpyridin-3-yl)estra-1(10),2,4,16-tetraen-3-yl]-N-methylpropanamid
• N-[17-(5-Fluorpyridin-3-yl)estra-1(10),2,4,16-tetraen-3-yl]-3-(2H-tetrazol-5-yl)propanamid
• N-[17-(5-Fluorpyridin-3-yl)estra-1(10),2,4,16-tetraen-3-yl]-N-methyl-3-(2H-tetrazol-5-yl)propanamid
• 4-{[17-(5-Fluorpyridin-3-yl)estra-1(10),2,4,16-tetraen-3-yl] (methyl)amino}-4-oxobutansäure-tert.-butylester
• 4-{[17-(5-Fluorpyridin-3-yl)estra-1(10),2,4,16-tetraen-3-yl] (methyl)amino}-4-oxobutansäure
• 3-{[17-(5-Fluorpyridin-3-yl)estra-1(10),2,4,16-tetraen-3-yl](methyl)sulfamoyl}propansäuremethylester
• 3-{[17-(5-Fluorpyridin-3-yl)estra-1(10),2,4,16-tetraen-3-yl]sulfamoyl}propansäuremethylester
• 3-{[17-(5-Fluorpyridin-3-yl)estra-1(10),2,4,16-tetraen-3-yl] (methyl)sulfamoyl}propansäure
• 3-{[17-(5-Fluorpyridin-3-yl)estra-1(10),2,4,16-tetraen-3-yl]sulfamoyl}propansäure
• N-[17-(5-Fluorpyridin-3-yl)estra-1(10),2,4,16-tetraen-3-yl]}acetamid
• N-{[17-(5-Fluorpyridin-3-yl)estra-1(10),2,4,16-tetraen-3-yl]carbamoyl}betaalaninethylester
• N-{[17-(5-Fluorpyridin-3-yl)estra-1(10),2,4,16-tetraen-3-yl]carbamoyl}betaalanin
• 4-{[17-(5-Fluorpyridin-3-yl)estra-1(10),2,4,16-tetraen-3-yl] (methyl)amino}butansäuremethylester
• 4-{[17-(5-Fluorpyridin-3-yl)estra-1(10),2,4,16-tetraen-3-yl] (methyl)amino}butansäure
• 4-{[17-(6-Methylpyridazin-4-yl)estra-1(10),2,4,16-tetraen-3-yl]amino}4-oxobutansäure
• 17-(5-Fluorpyridin-3-yl)-3-(S-methylsulfonimidoyl)estra-1(10),2,4,16-tetraen und die Tautomere, N-Oxide, Hydrate, Solvate oder Salze davon, oder eine aus den obigen bestehende Mischung.

6. Verbindung der Formel (I), definiert wie in einem der Ansprüche 1, 2, 3, 4 oder 5, zur Behandlung und/oder Prophylaxe von Krankheiten.

7. Verbindung der Formel (I), definiert wie in einem der Ansprüche 1, 2, 3, 4 oder 5, zur Verwendung in einem Verfahren zur Behandlung und/oder Prophylaxe von Endometriose, von Leiomyom, von Gebärmutterblutungsstörungen, von Dysmenorrhö, von Prostatakarzinom, von Prostatahyperplasie, von Akne, von Seborrhö, von Haarverlust, von vorzeitiger sexueller Reifung, von dem Syndrom der polyzystischen Ovarien, von Brustkrebs, von Lungenkrebs, von Endometriumkarzinom, von Nierenzellkarzinom, von Blasenkarzinom, von Nicht-Hodgkin-Lymphom, von chronischer obstruktiver Lungenkrankheit (Chronic Obstructive Pulmonary Disease, COPD), von Obesitas oder von mit Entzündungen in Zusammenhang stehenden Schmerzen.

8. Verwendung einer Verbindung nach einem der Ansprüche 1, 2, 3, 4 oder 5 zur Herstellung eines Medikaments zur Behandlung und/oder Prophylaxe von Krankheiten.

9. Verwendung einer wie in einem der Ansprüche 1, 2, 3, 4 oder 5 definierten Verbindung zur Herstellung eines Medikaments zur Prophylaxe von Endometriose, von Leiomyom, von Gebärmutterblutungsstörungen, von Dysmenorrhö, von Prostatakarzinom, von Prostatahyperplasie, von Akne, von Seborrhö, von Haarverlust, von vorzeitiger sexueller Reifung, von dem Syndrom der polyzystischen Ovarien, von Brustkrebs, von Lungenkrebs, von Endometriumkarzinom, von Nierenzellkarzinom, von Blasenkarzinom, von Nicht-Hodgkin-Lymphom, von chronischer obstruktiver Lungenkrankheit (Chronic Obstructive Pulmonary Disease, COPD), von Obesitas oder von mit Entzündungen in Zusammenhang stehenden Schmerzen.

10. Medikament, umfassend eine wie in einem der Ansprüche 1, 2, 3, 4 oder 5 definierte Verbindung in Kombination mit einem oder mehreren weiteren Wirkstoffen, insbesondere mit selektiven Estrogenrezeptormodulatoren (SERMs), Estrogen-rezeptor(ER)-Antagonisten, Aromataseinhibitoren, 17-HSD1-Inhibitoren, Steroidsulphatase(STS)-Inhibitoren, GnRH-Agonisten und -Antagonisten, Kisspeptinrezeptor(KISSR)-Antagonisten, selektiven Androgenrezeptormodulatoren (SARMs), Androgenen, 5-Reduktase-Inhibitoren, C(17,20)-Lyaseinhibitoren, selektiven Progesteronrezeptormodulatoren (SPRMs), Gestagenen, Antigestagenen, oralen Kontrazeptiva, Inhibitoren von Mitogen-Activated Protein(MAP)-Kinasen und Inhibitoren der MAP-Kinasen (Mkk3/6, Mek1/2, Erk1/2), Inhibitoren der Proteinkinasen B (PKBα/β/γ; Akt1/2/3), Inhibitoren der Phosphoinositid-3-Kinasen (PI3K), Inhibitoren von cyclinabhängiger Kinase (CDK1/2), Inhibitoren des hypoxieinduzierten Signalpfades (HIF1alpha-Inhibitoren, Prolylhydroxylase-Aktivatoren), Histondeacetylase(HDAC)-Inhibitoren, Prostaglandin-F-Rezeptor(FP) (PTGFR)-Antagonisten und nichtsteroidalen entzündungshemmenden Mitteln (Non-Steroidal Anti-Inflammatory Drugs, NSAIDs).

11. Medikament, umfassend eine wie in einem der Ansprüche 1, 2, 3, 4 oder 5 definierte Verbindung der Formel (I) in Kombination mit einem inerten, nicht toxischen, pharmazeutisch geeigneten Exzipienten.

12. Medikament nach Anspruch 10 oder 11 zur Behandlung und Prophylaxe von Endometriose, von Gebärmutter-Leiomyom, von Gebärmutterblutungsstörungen, von Dysmenorrhö, von Prostatakarzinom, von Prostatahyperplasie, von Akne, von Seborrhö, von Haarverlust, von vorzeitiger sexueller Reifung, von dem Syndrom der polyzystischen Ovarien, von Brustkrebs, von Lungenkrebs, von Endometriumkarzinom, von Nierenzellkarzinom, von Blasenkarzinom, von Nicht-Hodgkin-Lymphom, von chronischer obstruktiver Lungenkrankheit (Chronic Obstructive Pulmonary Disease, COPD), von Obesitas oder von mit Entzündungen in Zusammenhang stehenden Schmerzen.

## Revendications

1. Composés de formule générale (I) 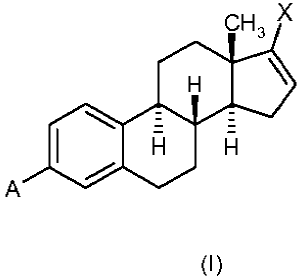 où :
A représente un groupe choisi parmi : 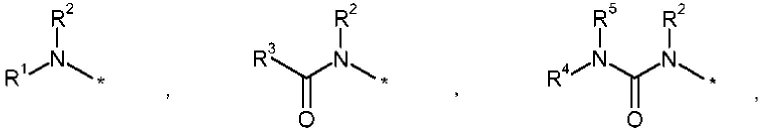 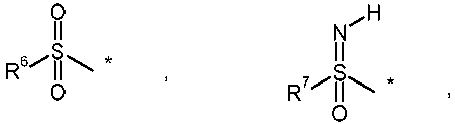 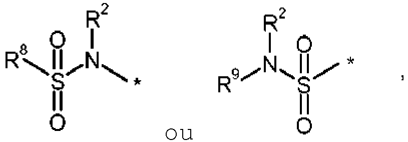
dans lequel * indique le point de liaison dudit groupe au reste de la molécule ;
X est un groupe choisi parmi : 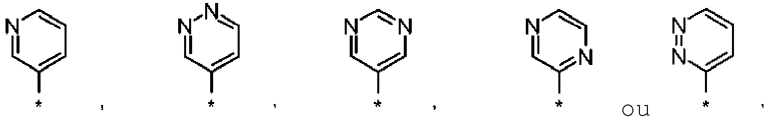
dans lequel * indique le point de liaison du groupe au reste de la molécule et où le groupe est facultativement substitué, une ou plusieurs fois, indépendamment les unes des autres, par un substituant choisi parmi halogène, CN, OH, RR²N-, alkyle en C₁-C₆, halogénoalkyle en C₁-C₆, alcoxy en C₁-C₆, halogénoalcoxy en C₁-C₆, dans lequel les groupes alkyle en C₁-C₆ et halogénoalkyle en C₁-C₆ sont facultativement substitués par OH ;
R¹ est alkyle en C₁-C₆ ou halogénoalkyle en C₁-C₆, et où R¹ est facultativement substitué par un ou deux substituants, indépendamment l'un de l'autre, choisis parmi OH, CN, RO(CO)-, RR²N(CO)-, RR²NSO₂-, (alkyle en C₁-C₆)-(CO)(NH)SO₂- ou 5-tétrazolyle ;
R², R⁵ sont, indépendamment l'un de l'autre, hydrogène ou alkyle en C₁-C₆, où les groupes alkyle en C₁-C₆ sont facultativement substitués, une ou plusieurs fois, indépendamment les unes des autres, par halogène,
R³, R⁴ sont, indépendamment l'un de l'autre, alkyle en C₁-C₆ ou halogénoalkyle en C₁-C₆, et
où
R³ et R⁴ sont facultativement substitués par un ou deux substituants, indépendamment l'un de l'autre, choisis parmi OH, CN, RO(CO)-, RR²N(CO)-, RR²NSO₂-, (alkyle en C₁-C₆)-(CO)(NH)SO₂- ou 5-tétrazolyle ;
R⁶, R⁷, R⁸, R⁹ sont, indépendamment les uns des autres, alkyle en C₁-C₆ ou halogénoalkyle en C₁-C₆, et où
R⁶, R ⁷, R⁸ et R⁹ sont facultativement substitués par un ou deux substituants, indépendamment les uns des autres, choisis parmi OH, CN, RO(CO)-, RR²N(CO)-, RR²NSO₂-, (alkyle en C₁-C₆)-(CO)(NH)SO₂- ou 5-tétrazolyle ;
R est hydrogène ou un groupe alkyle en C₁-C₆ ;
ou les tautomères, N-oxydes, hydrates, solvates ou sels de ceux-ci, ou un mélange constitué de ceux-ci.

2. Composés de formule générale (I) selon la revendication 1,
où
A représente un groupe choisi parmi : 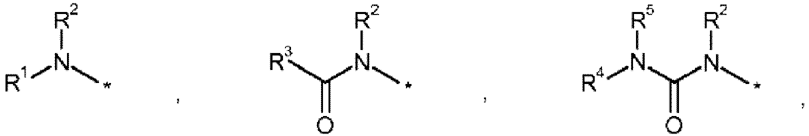 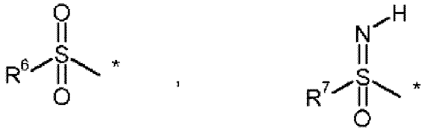 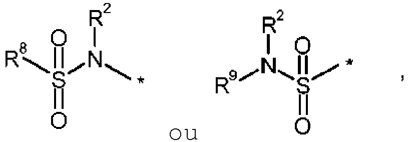
dans lequel * indique le point de liaison dudit groupe au reste de la molécule ;
X est un groupe choisi parmi : 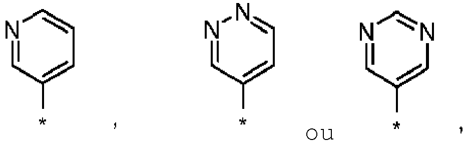
dans lequel * indique le point de liaison du groupe au reste de la molécule et où le groupe est facultativement substitué, une ou plusieurs fois, indépendamment les unes des autres, par un substituant choisi parmi halogène, alkyle en C₁-C₄, halogénoalkyle en C₁-C₄, alcoxy en C₁-C₄, dans lequel les groupes alkyle en C₁-C₄ et halogénoalkyle en C₁-C₄ sont facultativement substitués par OH ;
R¹ est alkyle en C₁-C₄, qui est facultativement substitué par un ou deux substituants, indépendamment l'un de l'autre, choisis parmi OH, RO(CO)-, RR²N(CO)-, RR²NSO₂- ou 5-tétrazolyle ;
R², R⁵ sont, indépendamment l'un de l'autre, hydrogène ou alkyle en C₁-C₄, où les groupes alkyle en C₁-C₄ sont facultativement substitués, une ou plusieurs fois, indépendamment les unes des autres, par halogène ;
R³, R⁴ sont, indépendamment l'un de l'autre, alkyle en C₁-C₄, et où R³ et R⁴ sont facultativement substitués par un ou deux substituants, indépendamment l'un de l'autre, choisis parmi OH, RO(CO)-, RR²N(CO)-, RR²NSO₂-, (alkyle en C₁-C₄)-(CO)(NH)SO₂- ou 5-tétrazolyle ;
R⁶, R⁷, R⁸, R⁹ sont, indépendamment les uns des autres, alkyle en C₁-C₄, et où R⁶, R⁷, R⁸ et R⁹ sont facultativement substitués par un ou deux substituants, indépendamment l'un de l'autre, choisi parmi OH, RO(CO)-, RR²N(CO)-, RR²NSO₂- ou 5-tétrazolyle ;
R est hydrogène ou un groupe alkyle en C₁-C₄ ;
ou un tautomère, un N-oxyde, un hydrate, un solvate, ou un sel de ceux-ci, ou un mélange constitué de ceux-ci.

3. Composés de formule générale (I) selon les revendications 1 à 2,
où
A représente un groupe choisi parmi : 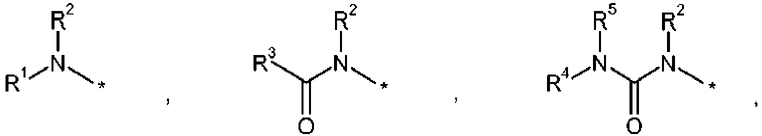 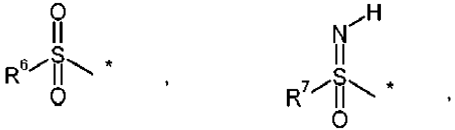 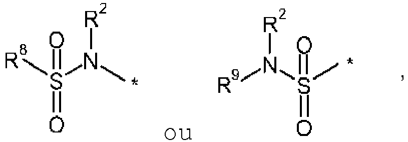
dans lequel * indique le point de liaison dudit groupe avec le reste de la molécule ;
X est un groupe choisi parmi : 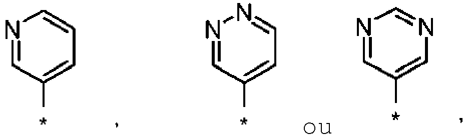
dans lequel * indique le point de liaison du groupe au reste de la molécule et où le groupe est facultativement substitué, une ou deux fois, indépendamment l'une de l'autre, par un substituant choisi parmi fluoro, chloro, méthyle, trifluorométhyle ou méthoxy ;
R¹ est propyle, qui est facultativement substitué par RO(CO)- ;
R², R⁵ sont, indépendamment l'un de l'autre, hydrogène ou méthyle ;
R³, R⁴ sont, indépendamment l'un de l'autre, alkyle en C₁-C₃, et où
R³ et R⁴ sont facultativement substitués par un ou deux substituants, indépendamment l'un de l'autre, choisis parmi OH, RO(CO)-, RR²N(CO)-, RR²NSO₂-, (alkyle en C₁-C₂)-(CO)(NH)SO₂- ou 5-tétrazolyle ;
R⁶, R⁷, R⁸, R⁹ sont, indépendamment les uns des autres, alkyle en C₁-C₄, et où
R⁶, R⁷, R⁸ et R⁹ sont facultativement substitués par un ou deux substituants, indépendamment les uns des autres, choisi parmi OH, RO(CO)- ou RR²N(CO)- ;
R est hydrogène ou un groupe alkyle en C₁-C₄ ;
ou un tautomère, un N-oxyde, un hydrate, un solvate, ou un sel de ceux-ci, ou un mélange constitué de ceux-ci.

4. Composés de formule générale (I) selon les revendications 1, 2 ou 3, où
A représente un groupe choisi parmi : 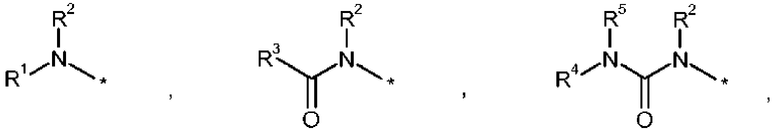 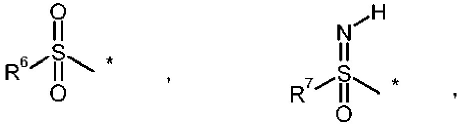 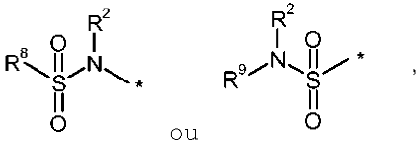
dans lequel * indique le point de liaison dudit groupe au reste de la molécule ;
X est un groupe choisi parmi : 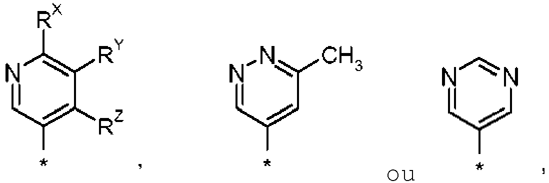
dans lequel * indique le point de liaison du groupe au reste de la molécule et,
R^{X} est hydrogène ou méthyle,
R^{Y} est hydrogène, fluoro, chloro, méthyle, trifluorométhyle ou méthoxy,
R^{Z} est hydrogène ou méthyle,
dans lequel un seul parmi R^{X}, R^{Y} et R^{Z} est différent d'hydrogène et ;
R¹ est -CH₂-CH₂-CH₂-COOH ;
R² est hydrogène ou méthyle ;
R³ est alkyle en C₁-C₃, qui est facultativement substitué par un substituant, choisi parmi OH, HO(CO)-, H₂N(CO)-, CH₃-(CO)(NH)SO₂- ou 5-tétrazolyle ;
R⁴ est -CH₂-CH₂-COOH ;
R⁵ est hydrogène ;
R⁶ est alkyle en C₁-C₄, qui est facultativement substitué par un substituant, choisi parmi OH, RO(CO)- ou H₂N(CO)- ;
R⁷ est méthyle ;
R⁸ est -CH₂-CH₂-COOH ;
R⁹ est alkyle en C₂-C₃, qui est substitué par un substituant, choisi parmi OH ou HO(CO)- ;
R est hydrogène ou méthyle ;
ou
un tautomère, un N-oxyde, un hydrate, un solvate, ou un sel de ceux-ci, ou un mélange constitué de ceux-ci.

5. Composés selon l'une quelconque des revendications 1, 2 ou 3, choisi parmi un groupe comprenant les composés suivants :
• N-{[17-(5-fluoropyridin-3-yl)estra-1(10),2,4,16-tétraén-3-yl]sulfonyl}-N-méthyl-bâta-alaninate de tert-butyle
• N-{[17-(5-fluoropyridin-3-yl)estra-1(10),2,4,16-tétraén-3-yl]suifonyl}-N-méthyl-bêta-alanine
• 17-(5-fluoropyridin-3-yl)-N-(3-hydroxypropyl)-N-méthylestra-1(10),2,4,16-tétraene-3-sulfonamide
• N-méthyl-N-{[17-(pyrimidin-5-yl)estra-1(10),2,4,16-tétraén-3-yl]sulfonyl}-bêta-alaninate de tert-butyle
• N-méthyl-N-{[17-(pyrimidin-5-yl)estra-1(10),2,4,16-tétraén-3-yl]sulfonyl}-bêta-alanine
• N-méthyl-N-{[17-(6-méthylpyridazin-4-yl)estra-1(10),2,4,16-tétraén-3-yl]sulfonyl}-bêta-alaninate de tert-butyle
• N-méthyl-N-{[17-(6-méthylpyridazin-4-yl)estra-1(10),2,4,16-tétraén-3-yl]sulfonyl}-bêta-alanine
• 17-(5-fluoropyridin-3-yl)-3-(méthylsulfonyl)estra-1(10),2,4,16-tétraène
• 4-{[17-(5-fluoropyridin-3-yl)estra-1(10),2,4,16-tétraén-3-yl]sulfonyl}butanoate de méthyle
• acide 4-{[17-(5-fluoropyridin-3-yl)estra-1(10),2,4,16-tétraén-3-yl]sulfonyl}butanoïque
• 4-1[17-(pyridin-3-yl)estra-1(10),2,4,16-tétraén-3-yl]sulfonyl}butanoate de méthyle
• acide 4-{[17-(pyridin-3-yl)estra-1(10),2,4,16-tétraén-3-yl]sulfonyl}butanoïque
• 4-({17-[5-(trifluorométhyl)pyridin-3-yl]estra-1(10),2,4,16-tétraén-3-yl}sulfonyl)butanoate de méthyle
• acide 4-({17-[5-(trifluorométhyl)pyridin-3-yl]estra-1(10),2,4,16-tétraén-3-yl}sulfonyl)butanoïque
• acide 4-{[17-(6-méthylpyridin-3-yl)estra-1,3,5(10),16-tétraén-3-yl]sulfonyl}butanoïque
• acide 4-{[17-(5-méthoxypyridin-3-yl)estra-1,3,5(10),16-tétraén-3-yl]sulfonyl}butanoïque
• acide 4-{[17-(5-méthylpyridin-3-yl)estra-1,3,5(10),16-tétraén-3-yl]sulfonyl}butanoïque
• acide 4-{[17-(4-méthylpyridin-3-yl)estra-1,3,5(10),16-tétraén-3-yl]sulfonyl}butanoïque
• acide 4-{[17-(5-chloropyridin-3-yl)estra-1,3,5(10),16-tétraén-3-yl]sulfonyl}butanoïque
• 4-{[17-(5-fluoropyridin-3-yl)estra-1(10),2,4,16-tétraén-3-yl]sulfonyl}butanamide
• 4-{[17-(5-fluoropyridin-3-yl)estra-1(10),2,4,16-tétraén-3-yl]sulfonyl}butan-1-ol
• 4-{[17-(5-fluoropyridin-3-yl)estra-1(10),2,4,16-tétraén-3-yl]amino}-4-oxobutanoate de tert-butyle
• acide 4-{[17-(5-fluoropyridin-3-yl)estra-1(10),2,4,16-tétraén-3-yl]amino}-4-oxobutanoïque
• 4-oxo-4-{[17-(pyrimidin-5-yl)estra-1(10),2,4,16-tétraén-3-yl]amino}butanoate de tert-butyle
• acide 4-oxo-4-{[17-(pyrimidin-5-yl)estra-1(10),2,4,16-tétraén-3-yl]amino}butanoïque
• N-[17-(5-fluoropyridin-3-yl)estra-1(10),2,4,16-tétraén-3-yl]butanediamide
• N-[17-(5-fluoropyridin-3-yl)estra-1(10),2,4,16-tétraén-3-yl]-4-hydroxybutanamide
• N-[17-(5-fluoropyridin-3-yl)estra-1(10),2,4,16-tétraén-3-yl]-3-sulfamoylpropanamide
• 3-(acétylsulfamoyl)-N-[17-(5-fluoropyridin-3-yl)estra-1(10),2,4,16-tétraén-3-yl]propanamide
• N-[17-(5-fluoropyridin-3-yl)estra-1(10),2,4,16-tétraén-3-yl]-N-méthyl-3-sulfamoylpropanamide
• 3-(acétylsulfamoyl)-N-[17-(5-fluoropyridin-3-yl)estra-1(10),2,4,16-tétraén-3-yl]-N-méthylpropanamide
• N-[17-(5-fluoropyridin-3-yl)estra-1(10),2,4,16-tétraén-3-yl]-3-(2H-tétrazol-5-yl)propanamide
• N-[17-(5-fluoropyridin-3-yl)estra-1(10),2,4,16-tétraén-3-yl]-N-méthyl-3-(2H-tétrazol-5-yl)propanamide
• 4-{[17-(5-fluoropyridin-3-yl)estra-1(10),2,4,16-tétraén-3-yl](méthyl)amino}-4-oxobutanoate de tert-butyle
• acide 4-{[17-(5-fluoropyridin-3-yl)estra-1(10),2,4,16-tétraén-3-yl](méthyl)amino}-4-oxobutanoïque
• 3-{[17-(5-fluoropyridin-3-yl)estra-1(10),2,4,16-tétraén-3-yl](méthyl)sulfamoyl}propanoate de méthyle
• 3-{[17-(5-fluoropyridin-3-yl)estra-1(10),2,4,16-tétraén-3-yl]sulfamoyl}propanoate de méthyle
• acide 3-{[17-(5-fluoropyridin-3-yl)estra-1(10),2,4,16-tétraén-3-yl](méthyl)sulfamoyl}propanoique
• acide 3-{[17-(5-fluoropyridin-3-yl)estra-1(10),2,4,16-tétraén-3-yl]sulfamoyl}propanoïque
• N-[17-(5-fluoropyridin-3-yl)estra-1(10),2,4,16-tétraén-3-yl]acétamide
• N-{[17-(5-fluoropyridin-3-yl)estra-1(10),2,4,16-tétraén-3-yl]carbamoyl}-bêta-alaninate d'éthyle
• N-{[17-(5-fluoropyridin-3-yl)estra-1(10),2,4,16-tétraén-3-yl]carbamoyl}-bêta-alanine
• 4-{[17-(5-fluoropyridin-3-yl)estra-1(10),2,4,16-tétraén-3-yl](méthyl)amino}butanoate de méthyle
• acide 4-{[17-(5-fluoropyridin-3-yl)estra-1(10),2,4,16-tétraén-3-yl](méthyl)amino}butanoïque
• acide 4-{[17-(6-méthylpyridazin-4-yl)estra-1(10),2,4,16-tétraén-3-yl]amino}-4-oxobutanoïque
• 17-(5-fluoropyridin-3-yl)-3-(S-méthylsulfonimidoyl)estra-1(10),2,4,16-tétraène et les tautomères, N-oxydes, hydrates, solvates ou sels de ceux-ci, ou un mélange constitué de ceux-ci.

6. Composé de formule (I) tel que défini dans l'une quelconque des revendications 1, 2, 3, 4 ou 5 pour le traitement et/ou la prophylaxie de maladies.

7. Composé de formule (I) tel que défini dans l'une quelconque des revendications 1, 2, 3, 4 ou 5 pour utilisation dans un procédé pour le traitement et/ou la prophylaxie de l'endométriose, du léiomyome, des troubles hémorragiques utérins, de la dysménorrhée, du carcinome de la prostate, de l'hyperplasie de la prostate, de l'acné, de la séborrhée, de la perte des cheveux, de la maturité sexuelle prématurée, du syndrome des ovaires polykystiques, du cancer du sein, du cancer du poumon, du carcinome de l'endomètre, du carcinome à cellules rénales, du carcinome de la vessie, du lymphome non hodgkinien, de la bronchopneumopathie obstructive chronique (BPCO), de l'obésité ou de la douleur liée à l'inflammation.

8. Utilisation d'un composé selon l'une quelconque des revendications 1, 2, 3, 4 ou 5 pour la production d'un médicament pour le traitement et/ou la prophylaxie de maladies.

9. Utilisation d'un composé tel que défini dans l'une quelconque des revendications 1, 2, 3, 4 ou 5 pour la production d'un médicament pour la prophylaxie de l'endométriose, du léiomyome, des troubles hémorragiques utérins, de la dysménorrhée, du carcinome de la prostate, de l'hyperplasie de la prostate, de l'acné, de la séborrhée, de la perte des cheveux, de la maturité sexuelle prématurée, du syndrome des ovaires polykystiques, du cancer du sein, du cancer du poumon, du carcinome de l'endomètre, du carcinome à cellules rénales, du carcinome de la vessie, du lymphome non hodgkinien, de la bronchopneumopathie obstructive chronique (BPCO), de l'obésité ou de la douleur liée à l' inflammation.

10. Médicament comprenant un composé tel que défini dans l'une quelconque des revendications 1, 2, 3, 4 ou 5 en combinaison avec une ou plusieurs autres substances actives, en particulier avec des modulateurs sélectifs des récepteurs d'oestrogène (SERM), des antagonistes des récepteurs d'oestrogène (ER), des inhibiteurs d'aromatase, des inhibiteurs de 17-HSD1, des inhibiteurs de stéroïde sulfatase (STS), des agonistes et antagonistes de GnRH, des antagonistes du récepteur de kisspeptine (KISSR), des modulateurs sélectifs des récepteurs d'androgène (SARM), des inhibiteurs d'androgène 5-réductase, des inhibiteurs de C(17,20)-lyase, des modulateurs sélectifs des récepteurs de progestérone (SPRM), des gestagènes, des antigestagènes, des contraceptifs oraux, des inhibiteurs de protéine kinases activées par les mitogènes (MAP) et des inhibiteurs de MAP kinases (Mkk3/6, Mek1/2, Erk1/2), des inhibiteurs des protéine kinases B (PKBα/β/γ ; Akt1/2/3), des inhibiteurs des phosphoinositide 3-kinases (PI3K), des inhibiteurs de kinase cycline-dépendante (CDK1/2), des inhibiteurs de la voie de signalisation induite par l'hypoxie (inhibiteurs de HIF1 alpha, activateurs de prolylhydroxylases), des inhibiteurs d'histone désacétylase (HDAC), des antagonistes de récepteur de prostaglandine F (FP) (PTGFR) et des inhibiteurs d'inflammation non stéroïdiens (AINS).

11. Médicament comprenant un composé de formule (I) tel que défini dans l'une quelconque des revendications 1, 2, 3, 4 ou 5 en combinaison avec un excipient inerte, non toxique, pharmaceutiquement adapté.

12. Médicament selon la revendication 10 ou 11 pour le traitement et la prophylaxie de l'endométriose, du léiomyome, des troubles hémorragiques utérins, de la dysménorrhée, du carcinome de la prostate, de l'hyperplasie de la prostate, de l'acné, de la séborrhée, de la perte des cheveux, de la maturité sexuelle prématurée, du syndrome des ovaires polykystiques, du cancer du sein, du cancer du poumon, du carcinome de l'endomètre, du carcinome à cellules rénales, du carcinome de la vessie, du lymphome non hodgkinien, de la bronchopneumopathie obstructive chronique (BPCO), de l'obésité ou de la douleur liée à l'inflammation.
